# EUROPEAN PATENT SPECIFICATION

(11) **EP 3 572 425 B1**
(45) Date of publication and mention of the grant of the patent: **18.11.2026**
(21) Application number: 19166848.2
(22) Date of filing: 17.12.2014
(51) Int. Cl.: C07K 16/06, C07K 16/30, A61P 35/00, A61P 35/02, A61K 47/68

(54) **MEANS AND METHODS FOR COUNTERACTING MYELOPROLIFERATIVE OR LYMPHOPROLIFERATIVE DISORDERS**
VORRICHTUNG UND VERFAHREN GEGEN MYELOPROLIFERATIVE ODER LYMPHOPROLIFERATIVE ERKRANKUNGEN
MOYENS ET PROCÉDÉ DE COMPENSATION DES TROUBLES MYÉLOPROLIFÉRATIFS OU LYMPHOPROLIFÉRATIFS

(30) Priority: 17.12.2013 EP 13197882
(43) Date of publication of application: 27.11.2019
(62) Document number(s) of the earlier application(s) in accordance with Art. 76 EPC: 14825462.6 / 3 083 678
(73) Proprietor: Kling Biotherapeutics B.V., 1105 BA Amsterdam (NL)
(72) Inventor: Spits, Hergen, 1105 BA Amsterdam Zuidoost (NL); Beaumont, Tim, 1105 BA Amsterdam Zuidoost (NL); Gillissen, Marijn Aletta, 1105 BA Amsterdam Zuidoost (NL); Bakker, Adrianus Quirinus, 1105 BA Amsterdam Zuidoost (NL); Hazenberg, Mette Deborah, 1105 BA Amsterdam Zuidoost (NL); Kedde, Martijn, 1105 BA Amsterdam Zuidoost (NL)
(74) Representative: V.O.

(56) References cited:
- MATSUOKA S ET AL: "A Novel Type of Cell Death of Lymphocytes Induced by a Monoclonal Antibody without Participation of Complement", THE JOURNAL OF EXPERIMENTAL MEDICINE, ROCKEFELLER UNIVERSITY PRESS, US, vol. 181, 1 June 1995 (1995-06-01), pages 2007 - 2015, XP002980487, ISSN: 0022-1007, DOI: 10.1084/JEM.181.6.2007
- ZHANG C ET AL: "A cell surface receptor defined by a mAb mediates a unique type of cell death similar to oncosis", PROCEEDINGS OF THE NATIONAL ACADEMY OF SCIENCES (PNAS), US, vol. 95, 1 May 1998 (1998-05-01), pages 6290 - 6295, XP002978699, ISSN: 0027-8424, DOI: 10.1073/PNAS.95.11.6290

## Description

The invention is as defined in the appended set of claims and relates to the fields of biology, immunology, medicine and cancer therapy, in particular therapy against myeloproliferative or lymphoproliferative disorders. More in particular, the invention relates to antibodies against acute myeloid leukemia cells.

Acute myeloid leukemia (AML) is a high risk malignancy with five year survival rates of 40 - 50% in patients younger than 60 years of age. For patients over 65 years of age outcomes are even worse, with only less than 20% of patients obtaining durable remissions. Allogeneic stem cell transplantation is frequently applied in the treatment of acute leukemia. It was initially designed to rescue patients from otherwise lethal myeloablative chemotherapy but was subsequently found to be complicated by alloreactive immune response related complications (graft versus host disease; GvHD). T cell depletion of grafts before reinfusion averted GvHD but the observation that T cell depleted graft recipients, similar to monozygotic twin donor transplant recipients, experienced much higher rates of relapse made it increasingly clear that the success of allogeneic SCT is dependent on the induction of an anti-leukemic immune response (graft versus leukemia (GvL)). This has led to the development of strategies to apply allogeneic stem cell transplantation without myeloablative conditioning (reduced intensity stem cell transplantation, RIST), to reduce cytotoxicity and to allow allogeneic SCT in a larger group of patients including older patients and heavily pretreated patients. Preparative regimens in RIST are aimed at decimating the recipients adaptive immune system to prevent graft rejection, without complete ablation of the recipients bone marrow thereby reducing early SCT toxicity. Following transplantation, donor stem cells gradually replace stem cells of the recipient and full donor chimerism is usually achieved within three months after SCT. Although allogeneic SCT is curative in significant numbers of patients, and much progress has been made in the supportive care of SCT recipients, still 15-30% of patients die as a result of transplantation related complications such as GvHD and infectious complications (arising as a result of slow immune recovery following SCT or as a complication of immunosuppressive therapy of GvHD).

Hence, although SCT is potentially curative when potent graft versus leukemia (GvL) responses are induced, its therapeutic success is limited by anti-host immune responses leading to GvHD which causes high morbidity and mortality.

About 70% of SCT patients develop GvHD at one point following SCT, with target organs including the skin, liver, intestine and lung. GvHD is treated with local or systemic immunosuppressive therapy including corticosteroids. A significant number of GvHD patients is not responsive to steroid therapy, and about half of these patients also respond poorly to alternative (and partly still experimental) measures such as mesenchymal stromal cell (MSC) transplantation or T cell modulating therapy such as anti-tumor necrosis factor α (anti-TNFα; infliximab). Extensive and long-term immunosuppressive therapy is undesirable, since it may hamper the development of therapeutic antileukemia responses. Indeed, when an AML relapse occurs while the patient is still on immunosuppressive therapy, the first step is to rapidly taper immunosuppressants. This may induce a curative GvL response, often at the expense of GvHD. In relapsed patients on immunosuppressive therapy who do not respond to tapering of immunosuppressants or relapsed patients in whom immunosuppressants were already phased out before relapse occurred but who had not developed GvHD, tumor load reduction by chemotherapy, followed by increasing doses of donor- lymphocyte infusions (DLI) may lead to durable remission of disease. While the absence of diagnostic tests to demonstrate the presence of a robust GvL makes it difficult to estimate exactly how often allogeneic SCT induce GvL responses in primary or relapsed AML, some studies provided compelling evidence of the induction of such a response in a considerable number of patients. For example, Schmid and colleagues demonstrated potent GvL responses by DLI in 50% of a small group of AML patients with disease relapse who were in second remission following chemotherapy (Schmid et al., 2007). Schlenk and colleagues convincingly showed the additive value of allogeneic SCT in primary AML, with a doubling of the 5-year disease free survival in patients with high risk AML who received an allogeneic SCT from a sibling, compared with patients who did not have a suitable sibling donor (Schlenk et al., 2008). Thus, GvL responses often occur at the cost of GvHD, and the observation that T cell depletion from the graft reduced GvHD incidence but increased disease relapse rates suggested that both are primarily mediated by T cell dependent immune responses against recipient-antigens.

In view of the high GvHD incidence after allogeneic stem cell transplantation, resulting in death of 15-30% of the patients, as well as the fact that a suitable donor is not always available for a given patient, alternative treatment approaches are needed. It is an object of the present invention to provide alternative means and methods for counteracting and/or preventing AML.

The present invention provides patient-derived, AML-specific, human antibodies that are able to bind intact AML cells. Importantly, the antibodies are derived from human AML patients that received an allogeneic SCT and are in complete remission indicating that the antibodies are effective against AML. Indeed, in the Examples it has been demonstrated that antibodies according to the present invention are able to bind intact AML cells. The AML-specific human antibodies according to the present invention are, therefore, particularly suitable for use in anti AML therapy. For instance, an antibody according to the invention is provided with a toxic moiety. After administration, AML cells will be bound and/or internalised and the toxic moiety will exert its toxic effect on the AML cell. Alternatively, in some embodiments antibody-dependent cell-mediated cytotoxicity (ADCC) or complement-dependent cytotoxicity (CDC) is induced using an antibody according to the present invention, optionally bound to an immunomodulatory compound. The fact that human antibodies are provided, or functional parts of human antibodies, diminishes the chance of side effects in human beings, which is an important advantage. Hence, with the antibodies provided by the present invention, novel AML treatment options have become available which can be used in addition to existing AML therapy, or as an alternative.

An important embodiment of the present invention provides human AML-specific antibodies that are capable of diminishing proliferation of AML cells. Administration of such antibodies to an AML patient will counteract AML cells without the need to add additional (toxic) moieties to the antibody. A particularly preferred embodiment provides human AML-specific antibodies according to the claims that are capable of diminishing proliferation of AML cells independently from antibody-dependent cell-mediated cytotoxicity (ADCC), complement-dependent cytotoxicity (CDC), apoptosis or phagocytosis by tumor-associated myeloid cells such as macrophages or dendritic cells. Preferably, such antibodies are capable of diminishing proliferation of AML cells (essentially) independently from any other immune cells, or complement or apoptosis. As shown in the Examples, such antibodies are capable of directly diminishing or inhibiting AML cell growth, or are even capable of killing AML cells, in the absence of immune cells like NK cells or macrophages, and in the absence of complement. This embodiment is in contrast to prior art approaches such as for instance described in Majeti et al., 2009 and Willingham et al., 2012, wherein a CD47-specific antibody is used in order to enable macrophage phagocytosis of tumor cells. WO 2009/051974 describes the use of C-type lectin-like molecule-1 (CLL-1)-specific antibodies, which are capable of inducing CDC against AML cells *in vitro* in the presence of rabbit complement. Bakker et al., 2004, however, does not envision that antibody-dependent cell-mediated cytotoxicity or complement-dependent cytotoxicity via CLL-1 will be an effective targeting mechanism, and proposes toxin-conjugated CLL-1 antibodies for use against AML. WO 2012/098407 discloses the use of antibodies specific for IL1RAP for inducing antibody-dependent cell-mediated cytotoxicity against AML cells, by recruiting NK cells which induce AML cell death. Hence, the above described prior art focuses on the use of antibodies in order to activate other immune cells, which may not provide the desired results in immune compromised individuals such as AML patients who have undergone chemotherapy with or without an allogeneic SCT.

WO 2013/071068 describes anti-CXCR4 antibodies that were raised in transgenic transchromosomic mice expressing human antibody genes. The obtained antibodies bind a broad range of hematopoietic cells and are capable of inhibiting growth of an AML cell line *in vitro.* These anti-CXCR4 antibodies induce apoptosis of various AML cell lines.

Anti-CD33 monoclonal antibody therapy against AML has also been described (Walther et al., 2012). For instance, CD33-specific antibodies coupled to a drug conjugate are described.

WO 2010/102244 describes a humaneered anti-EphA3 antibody that is capable of inducing apoptosis of AML cells. Furthermore, Biernacki et al., 2010 and Wu et al., 2000 describe antibodies obtained from AML patients that are specific for intracellular antigens such as RAB38, TBCE, DUSP12 and RAFTK.

Zhang et al. (1998), Proc.Natl.Acad.Sci. USA, Vol. 95, pages 6290-6295 describe an anti-Porimin antibody that has been obtained by immunizing mice with apoptotic Jurkat cells. This antibody induces cell death of Jurkat cells by a pathway similar to oncosis.

In conclusion, several publications describe AML antibodies that bind intracellular antigens. Such antibodies are not a first choice for combating living, intact AML cells *in vivo* due to the unreachability of such intracellular targets when the AML cells are intact. Other publications describe antibodies that bind a broad range of hematopoietic cells, for instance through CD33, CXCR4, CD47 or CLL-1. Antibodies which bind a broad range of (hematopoietic) cells involve the risk of severe side-effects.

Several antibodies described in the art act by inducing phagocytosis, ADCC or CDC, meaning that other immune cells or complement components are required for counteracting AML cells. The use of such antibodies is, therefore, limited in immune compromised individuals. Moreover, some of the described antibodies are not human, which involves a high risk of side effects. Other publications (such as WO 2013/071068 and WO 2010/102244) describe antibodies that induce apoptosis of AML cells.

In contrast to the above-mentioned publications, the present inventors have taken a different approach. Instead of artificially producing antibodies against a component that is present on, amongst other things, AML cells, the inventors have isolated antibodies from human AML patients who, after receiving an allogeneic SCT, mounted a potent graft versus leukemia response as they maintained in complete remission. These antibodies are capable of specifically binding intact AML cells. Hence, instead of using artificially developed antibodies, the present inventors have elegantly taken advantage of the natural immune defenses elicited in human AML patients after receiving an allogeneic SCT. The humoral response found in nature results in a very adequate selection and outgrowth of B-cells that produce effective antibodies *in vivo.* The antibodies according to the present invention, or functional parts or functional derivatives thereof, are therefore particularly suitable for binding AML cells in AML patients. The fact that the antibodies are specific for intact AML cells, instead of intracellular antigens, makes them particularly suitable for AML therapy.

As described, an antibody or functional part according to the invention can be coupled to a toxic moiety. Such toxic moiety will then be directed to AML cells *in vivo* through the binding of the antibody to AML cells. One embodiment of the present invention provides human antibodies according to the claims, or functional parts thereof, which are able to bind intact AML cells and which are able to diminish proliferation of AML cells. Such antibody obviates the need of coupling the antibody to an additional, toxic component (although the use of a toxic moiety can still be useful for providing additional anti-AML effects). In one particularly preferred embodiment, human antibodies, or functional parts thereof, according to the invention are provided which are able to bind intact AML cells and which are able to diminish proliferation of AML cells independently from antibody-dependent cell-mediated cytotoxicity (ADCC), complement-dependent cytotoxicity (CDC) or phagocytosis by tumor-associated myeloid cells such as macrophages or dendritic cells. Preferably, such antibodies are capable of diminishing proliferation of AML cells independently from, or essentially independently from, other immune cells or complement components. The use of such antibodies, having a strong anti-AML activity, is preferred, especially in immune-compromised individuals. One preferred aspect of the present invention therefore provides an isolated, synthetic or recombinant human antibody according to the claims, or a functional part thereof, which is able to bind a cell surface component of acute myeloid leukemia (AML) cells and which is able to diminish proliferation of AML cells independently from, or essentially independently from, antibody-dependent cell-mediated cytotoxicity (ADCC), complement-dependent cytotoxicity (CDC) or phagocytosis by tumor-associated myeloid cells such as macrophages or dendritic cells. Preferably, such antibodies are capable of diminishing proliferation of AML cells (essentially) independently from any other immune cells or complement.

In a further preferred embodiment, human antibodies, or functional parts thereof, according to the invention are provided which are able to bind intact AML cells and which are able to diminish proliferation of AML cells independently from apoptosis. Further provided is therefore an isolated, synthetic or recombinant human antibody according to the claims, or a functional part thereof, which is able to bind a cell surface component of acute myeloid leukemia (AML) cells and which is able to diminish proliferation of AML cells independently from, or essentially independently from, apoptosis.

In a further preferred embodiment, human antibodies, or functional parts thereof, according to the invention are provided which are able to diminish proliferation of AML cells independently from both apoptosis and the above-mentioned immune cells and complement. Further provided is therefore an isolated, synthetic or recombinant human antibody according to the claims, or a functional part thereof, which is able to bind a cell surface component of acute myeloid leukemia (AML) cells and which is able to diminish proliferation of AML cells essentially independently from antibody-dependent cell-mediated cytotoxicity (ADCC), complement-dependent cytotoxicity (CDC), apoptosis, or phagocytosis by macrophages or dendritic cells.

An antibody or functional part according to the invention is preferably capable of binding intact AML cells. Preferably, such antibody or functional part is able to bind a cell surface component that is specific for AML cells. This typically means that non-hematopoietic cells or non-malignant hematopoietic cells, such as for instance hepatocytes, colon cells, fibroblasts, endothelial cells, healthy peripheral blood mononuclear cells (PBMC) and healthy bone marrow cells, are not, or to a significantly lesser extent, recognized as compared to the binding affinity of an antibody or functional part according to the invention for AML cells. This means that the binding of an antibody or functional part according to the invention to non-hematopoietic cells or non-malignant hematopoietic cells is typically in the same range as binding of an irrelevant control antibody to these cells (wherein the control antibody is not specific for said cells). Some reactivity towards other types of malignant hematopoietic cells is, however, embraced within the term "AML-specific".

As used herein, the term "AML cells" embraces natural AML cells, such as primary AML blasts that are present in AML patients, as well as AML cell lines such as for instance THP-1, Mono-Mac 6 and Molm13.

The term "antibody" as used herein, refers to an immunoglobulin protein comprising at least a heavy chain variable region (VH), paired with a light chain variable region (VL), that is specific for a target epitope.

A "functional part of an antibody" is defined herein as a part that has at least one shared property as said antibody in kind, not necessarily in amount. Said functional part is capable of binding the same antigen as said antibody, albeit not necessarily to the same extent. In one embodiment a functional part of an antibody comprises at least a heavy chain variable domain (VH). Non-limiting examples of a functional part of an antibody are a single domain antibody, a single chain antibody, a nanobody, an unibody, a single chain variable fragment (scFv), a Fab fragment and a F(ab')₂ fragment.

A "functional equivalent of an antibody" is defined herein as an artificial binding compound, comprising at least one CDR sequence of an antibody, preferably a heavy chain CDR3 sequence. Said functional equivalent preferably comprises the heavy chain CDR3 sequence of an antibody, as well as the light chain CDR3 sequence of said antibody. More preferably, said functional equivalent comprises the heavy chain CDR1, CDR2 and CDR3 sequences of an antibody, as well as the light chain CDR1, CDR2 and CDR3 sequences of said antibody. A functional equivalent of an antibody is for instance produced by altering an antibody such that at least an antigen-binding property of the resulting compound is essentially the same in kind, not necessarily in amount. This is done in many ways, for instance through conservative amino acid substitution, whereby an amino acid residue is substituted by another residue with generally similar properties (size, hydrophobicity, etc), such that the overall functioning of the antibody is essentially not affected.

As is well known by the skilled person, a heavy chain of an antibody is the larger of the two types of chains making up an immunoglobulin molecule. A heavy chain comprises a constant domain and a variable domain, which variable domain is involved in antigen binding. A light chain of an antibody is the smaller of the two types of chains making up an immunoglobulin molecule. A light chain comprises a constant domain and a variable domain. The variable domain is often, but not always, together with the variable domain of the heavy chain involved in antigen binding.

Complementary-determining regions (CDRs) are the hypervariable regions present in heavy chain variable domains and light chain variable domains. In case of whole antibodies, the CDRs 1-3 of a heavy chain and the CDRs 1-3 of the connected light chain together form the antigen-binding site.

As used herein, the term "an antibody or functional part according to the invention" is also referred to as "a binding compound according to the invention".

The term "cell surface component of AML cells" means any component that is at least in part present in or on the cell surface of AML cells, or a component that is attached to an AML cell surface. Non-limiting examples of cell surface components of AML cells are (trans)membrane proteins, glycoproteins, and any compound attached thereto.

The terms "specific for" and "capable of specifically binding" are used herein interchangeably and refer to the interaction between an antibody, or functional part or functional equivalent thereof, and its epitope. This means that said antibody, or functional part or functional equivalent thereof, preferentially binds to said epitope over other antigens or amino acid sequences. Thus, although the antibody, functional part or equivalent may non-specifically bind to other antigens or amino acid sequences, the binding affinity of said antibody or functional part or functional equivalent for its epitope is significantly higher than the non-specific binding affinity of said antibody or functional part or functional equivalent for other antigens or amino acid sequences.

An antibody or functional part according to the invention that is able to bind a particular epitope of AML cells can also be specific for other, non-AML cells if said epitope of AML cells is also present on other cells (for instance other leukemic cells, myeloma cells or lymphoma cells). In that case an antibody referred to herein as being specific for AML cells is also specific for said other cells comprising the same epitope. Preferably, human AML antibodies and functional parts thereof, as provided herewith, do not significantly bind non-hematopoietic cells and non-malignant hematopoietic cells.

"Binding affinity" refers to the strength of the total sum of the noncovalent interactions between a single binding site of an antibody or functional part or functional equivalent and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity can generally be represented by the equilibrium dissociation constant (K_{D}), which is calculated as the kₐ to k_{d} ratio, see, e.g., Chen, Y., et al., (1999) J. Mol Biol 293:865-881. Affinity can be measured by common methods known in the art, such as for instance a surface plasmon resonance (SPR) assay such as BiaCore or IBIS-iSPR instrument at IBIS Technologies BV (Hengelo, the Netherlands) or solution phase assays, such as Kinexa. Preferably an antibody according to the invention has a binding affinity for an epitope at or on the cell surface of AML cells characterized by a dissociation constant (K_{D}) of at most 100 nM, more preferably at most 50 nM, more preferably at most 25 nM, more preferably at most 10 nM, more preferably at most 5 nM, more preferably at most 2 nM, more preferably at most 1 nM, more preferably at most 0.5 nM, more preferably at most 0.3 nM, more preferably at most 0.1 nM.

The percentage of identity of an amino acid or nucleic acid sequence, or the term "% sequence identity", is defined herein as the percentage of residues in a candidate amino acid or nucleic acid sequence that is identical with the residues in a reference sequence after aligning the two sequences and introducing gaps, if necessary, to achieve the maximum percent identity. Methods and computer programs for the alignment are well known in the art, for example "Align 2".

In a particularly preferred embodiment, an antibody or functional part according to the invention is provided which is able to induce death of primary AML blasts. Since primary AML blasts are directly derived from an AML patient, as opposed to commercially available cell lines, the activity of an antibody against such AML blasts is even more indicative for an in *vivo* situation. In a particularly preferred ambodiment, an antibody or functional part according to the invention is provided which is able to induce death of primary AML blasts independently from, or essentially independently from, antibody-dependent cell-mediated cytotoxicity (ADCC), complement-dependent cytotoxicity (CDC), apoptosis, and/or phagocytosis by macrophages or dendritic cells. Preferably, such binding compounds are capable of inducing death of AML blasts essentially independently from other immune cells, or complement or apoptosis.

As used herein, the term "essentially independently from ADCC, CDC or phagocytosis by tumor-associated myeloid cells such as macrophages or dendritic cells" means that ADCC, CDC or phagocytosis by tumor-associated myeloid cells such as macrophages or dendritic cells are not required for an anti-AML effect induced by a binding compound according to the present invention, even though in an *in vivo* situation ADCC, CDC or phagocytosis by tumor-associated myeloid cells such as macrophages or dendritic cells may also occur. Hence, in this embodiment, ADCC, CDC and phagocytosis by tumor-associated myeloid cells such as macrophages or dendritic cells is not excluded, but not essential either. Likewise, the term "essentially independently from other immune cells or complement" means that a binding compound according to the invention is, in principle, capable of exerting an anti-AML effect without the presence of other immune cells or complement, even though in an *in vivo* situation other immune cells or complement may also have an anti-AML activity. The term "essentially independently from apoptosis" means that the anti-AML effect is exerted by a binding compound according to the present invention via a mechanism other than apoptosis. Preferably, said anti-AML effect is exerted via necrosis.

In a further preferred embodiment, an antibody or functional part according to the invention is provided which is able to diminish proliferation of AML cells *in vitro* within 7 days, preferably within 5 days, more preferably within 3 days and even more preferably within 1 day. This indicates a quick therapeutic effect.

The present invention provides isolated, synthetic and recombinant human antibodies according to the claims, and functional parts thereof, which are able to bind intact AML cells. Said AML cells preferably belong to a French-American-British (FAB) classification selected from the group consisting of M5, M0, M1, M2, M3 and M4. More preferably, said AML cells belong to the FAB classification M5 or M1 or M0 or M4, preferably M5. These are the FAB classifications commonly found in AML patients.

In a further preferred embodiment, isolated, synthetic and recombinant human antibodies according to the claims, or functional parts thereof, are provided which are able to bind different AML cells of at least two, preferably at least three, more preferably at least four different FAB classifications. Such antibodies are useful for different AML patients having different FAB classifications, so that these antibodies are broadly applicable.

In one particularly preferred embodiment, an antibody or functional part according to the invention is provided wherein said antibody is of the IgG isotype, preferably IgG1 or IgG3. This is beneficial for medical applications in humans.

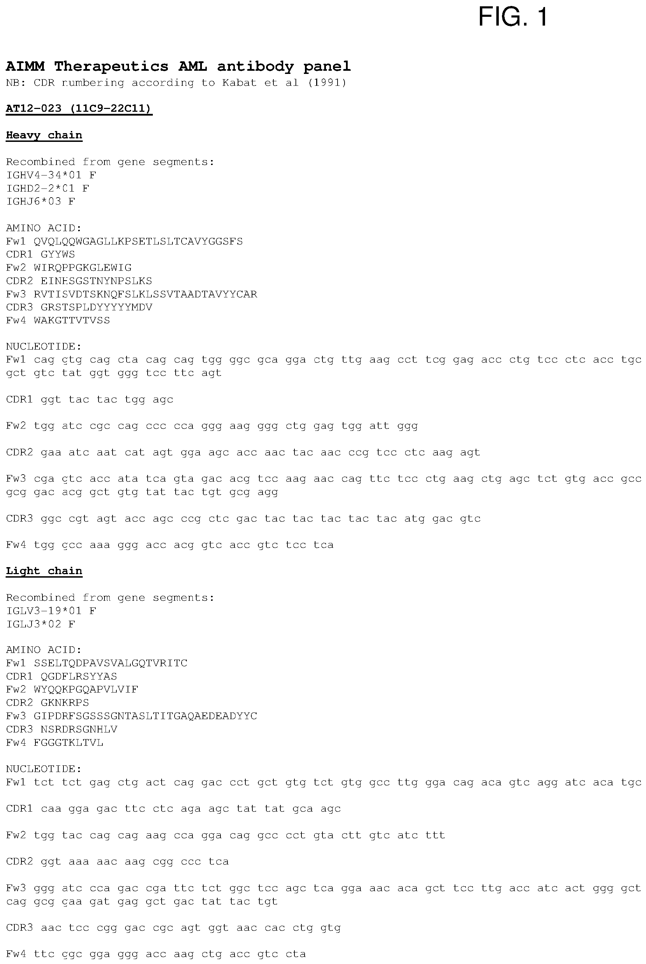

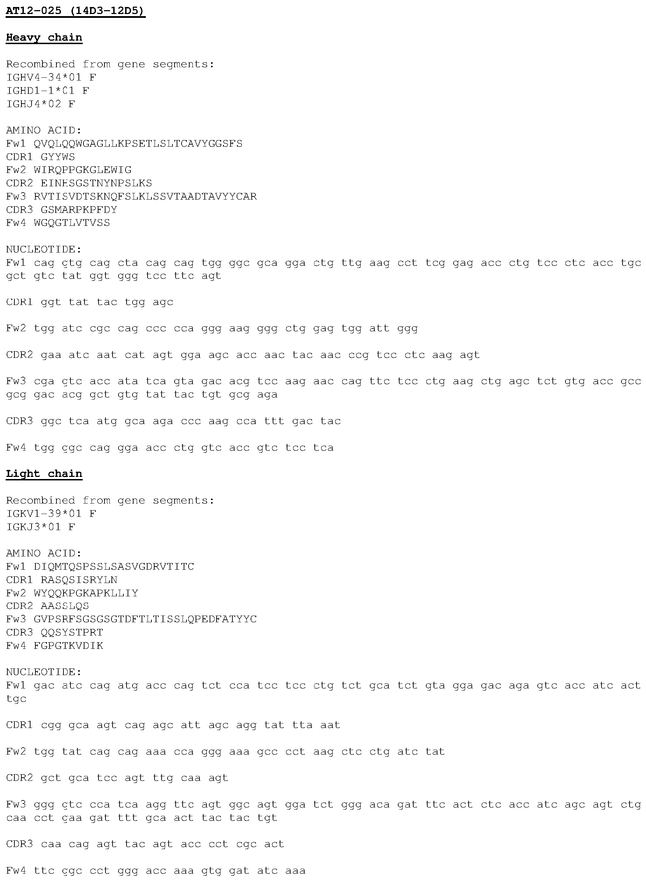

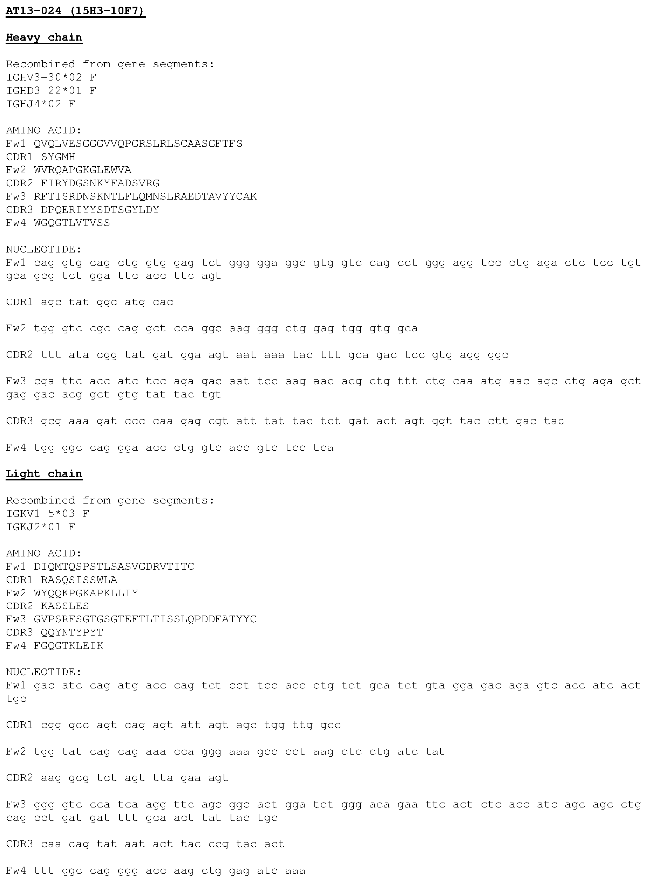

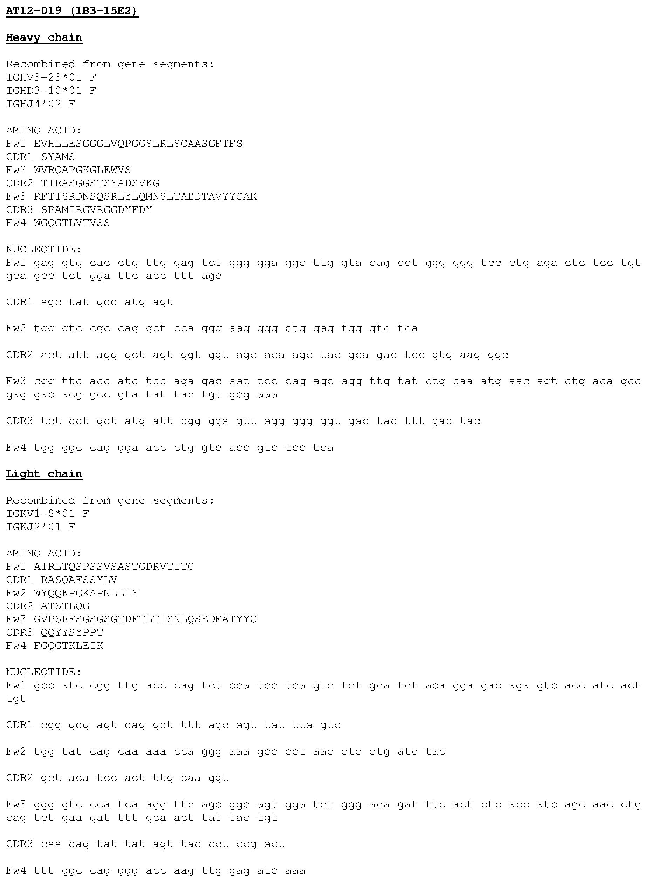

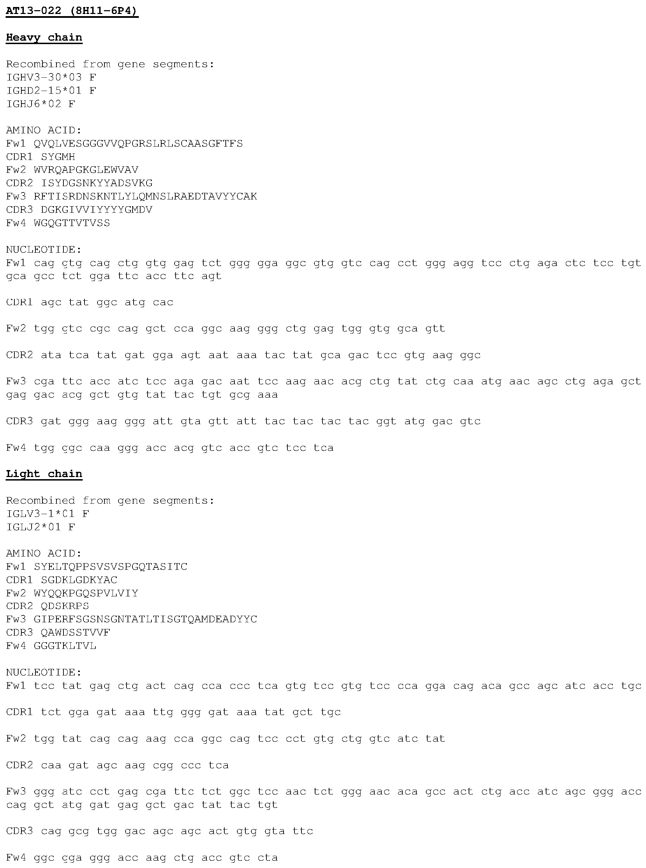

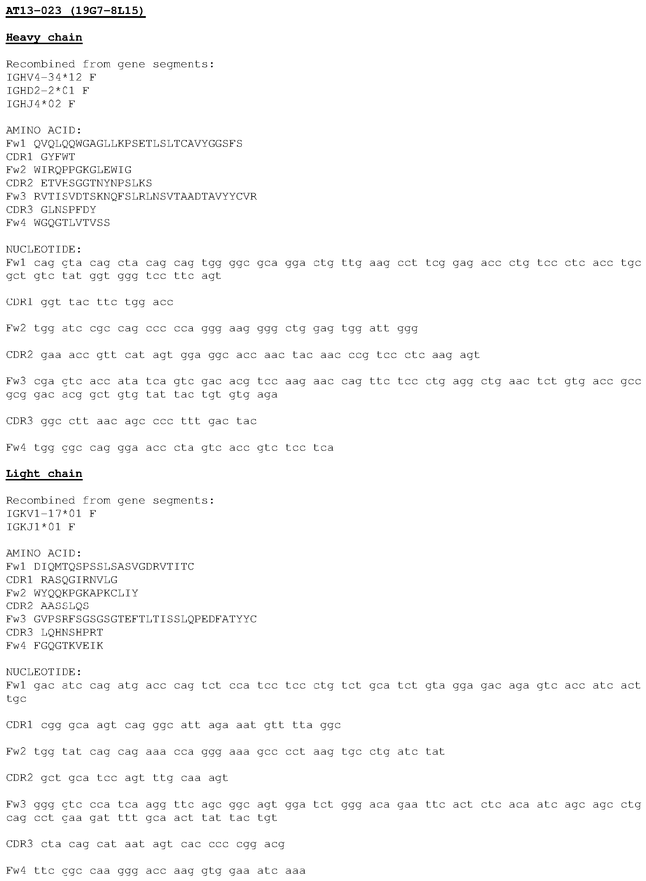

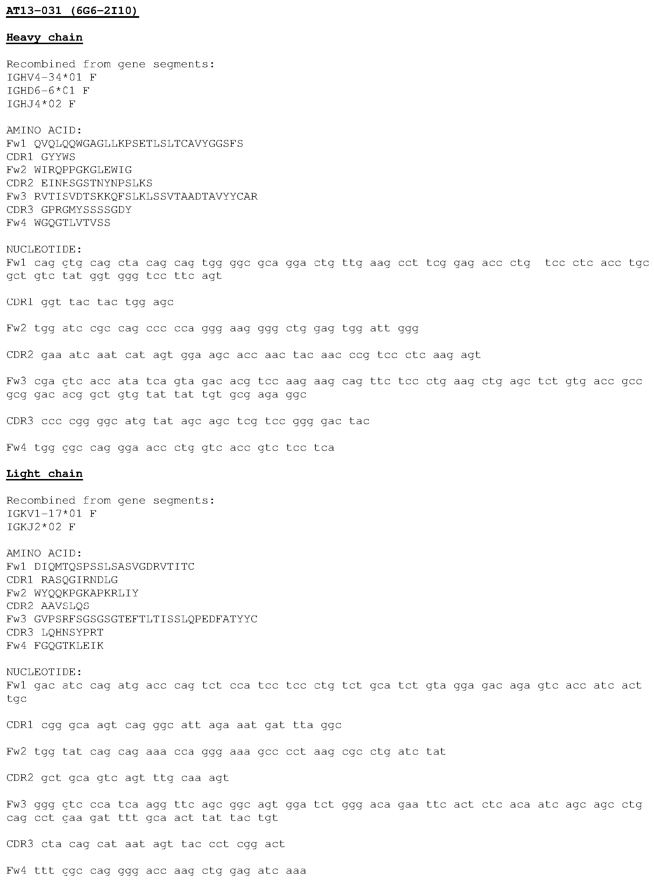

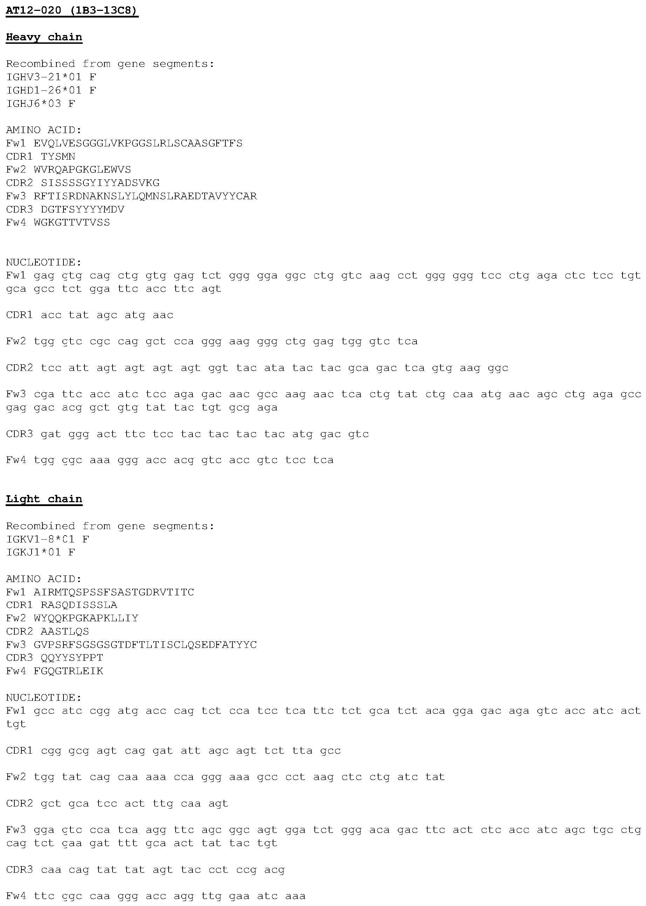

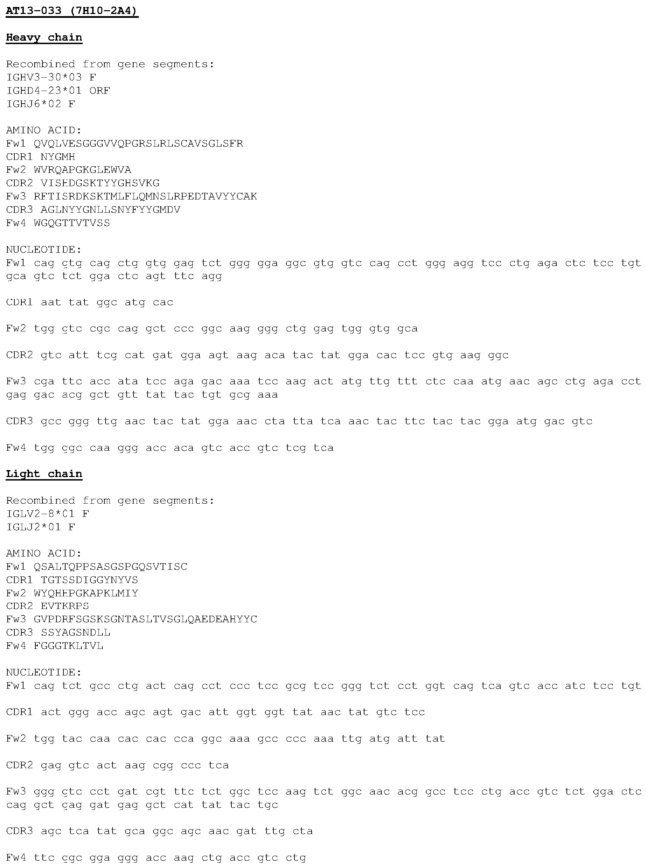

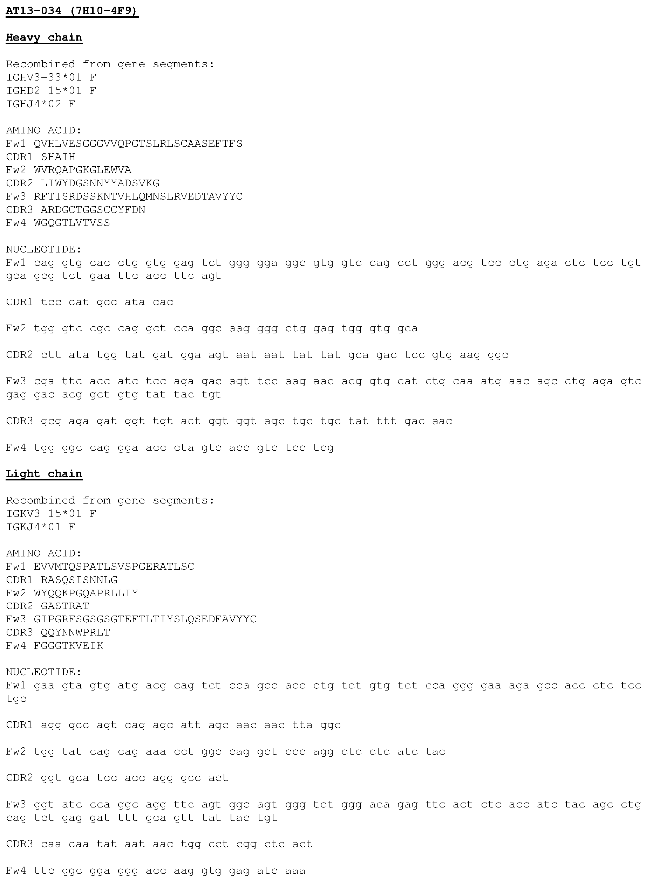

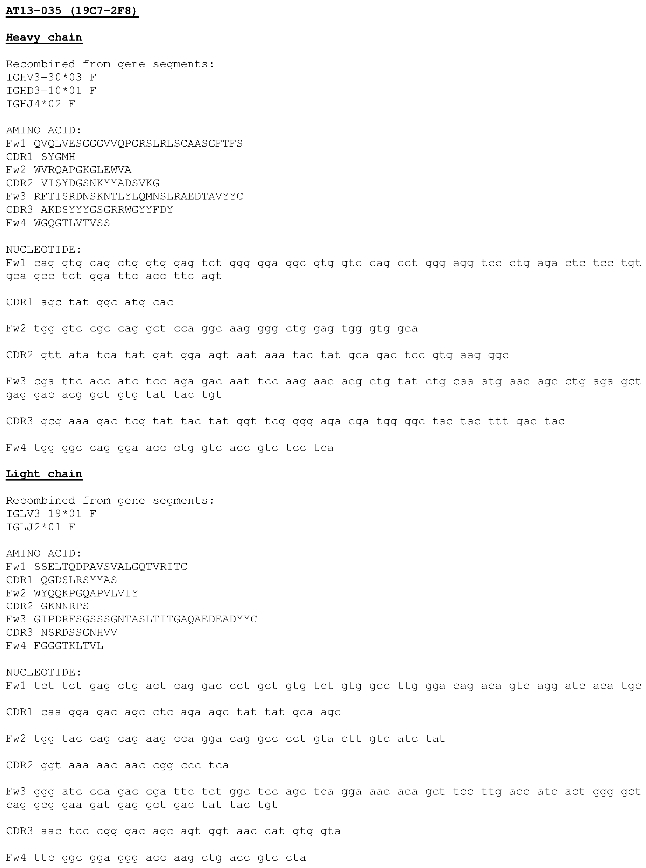

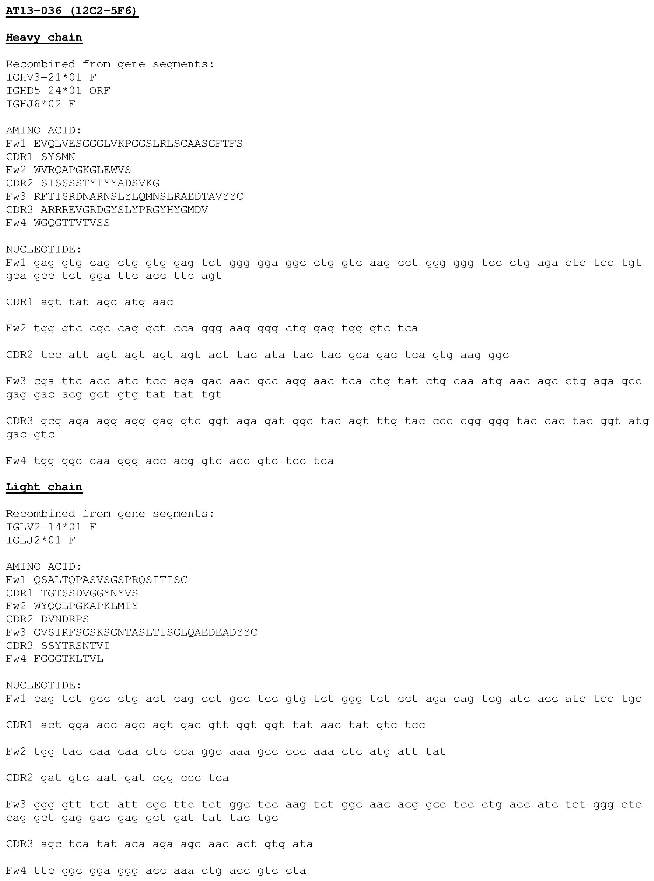

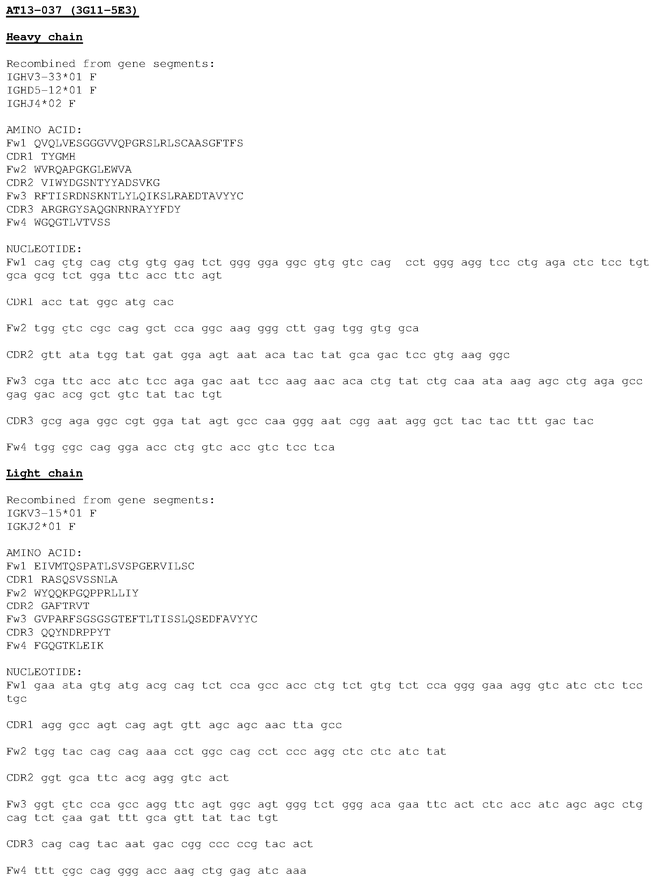

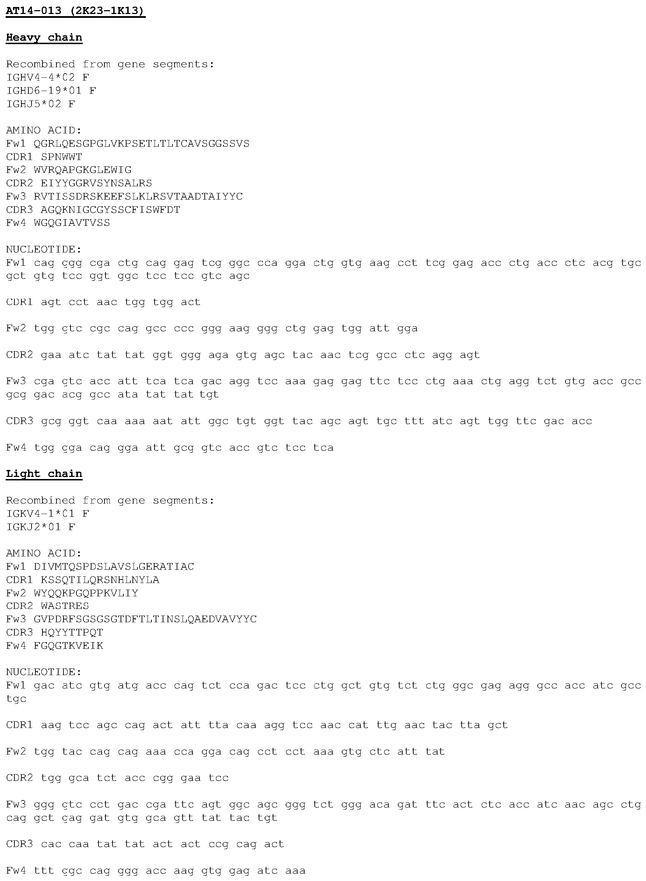

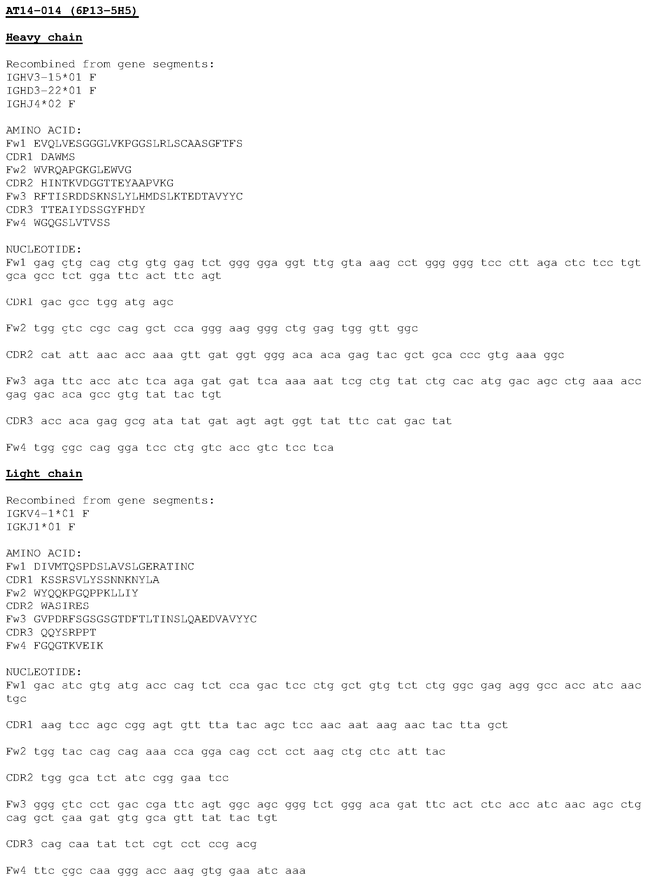

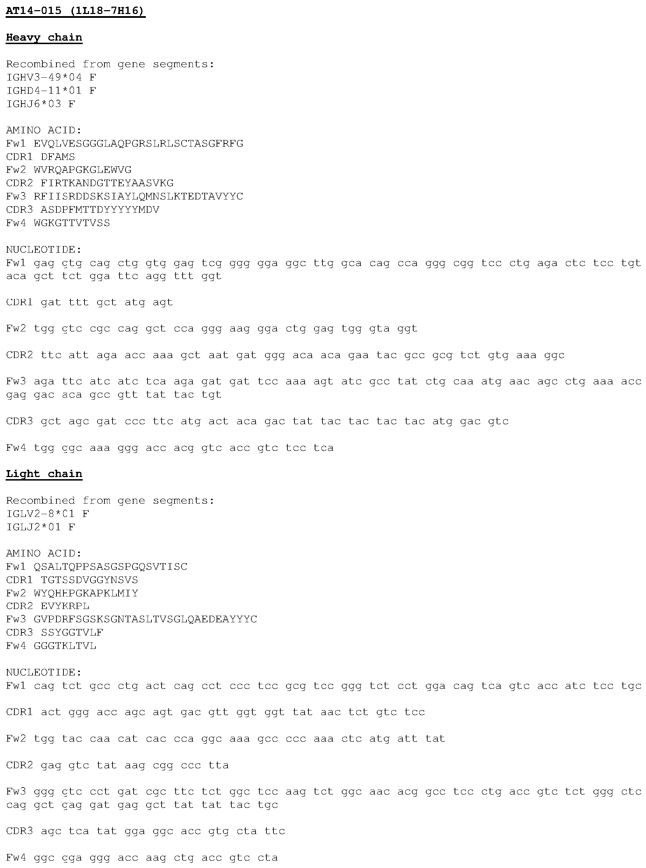

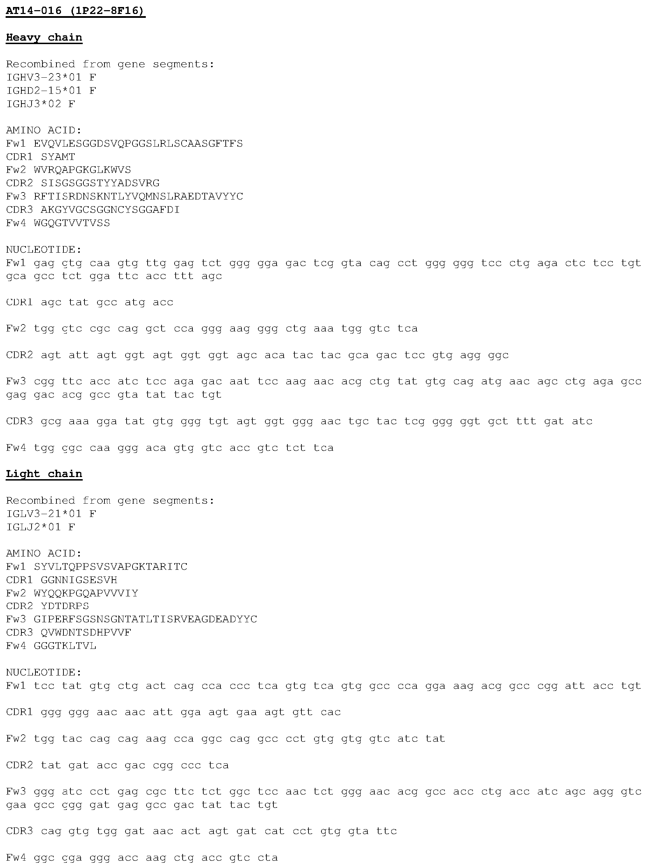

Tables 1A and 1B, and Figure 1, provide an overview of the variable heavy and light chain sequences, as well as the individual CDR sequences, of antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016. AT14-013 is according to the invention, obtained from a human AML patient. The terms "AT12-023", "AT12-025", "AT13-024", "AT12-019", "AT13-022", "AT13-023", "AT13-031", "AT12-020", "AT13-033", "AT13-034", "AT13-035", "AT13-036", "AT13-037", "AT14-013", "AT14-014", "AT14-015" and "AT14-016" as used herein encompass all antibodies and functional parts having at least the heavy and light chain CDR1-3 regions, preferably the variable heavy chain and light chain sequences, of these antibodies as depicted in Tables 1A and 1B and Figure 1, such as for instance isolated and/or purified antibodies or recombinantly produced antibodies.

As used herein, any reference to "Table 1" includes a reference to Table 1A and/or Table 1B.

Based on the antibodies depicted in Table 1 and Figure 1, it is possible to produce an antibody or functional part or functional equivalent thereof comprising at least one CDR sequence of an antibody depicted in Table 1 or Figure 1, which is specific for AML cells.A binding compound according to the invention comprises all three heavy chain CDRs and all three light chain CDRs of AT14-013. Optionally, at least one of said CDR sequences is optimized, thereby generating a variant binding compound, preferably in order to improve binding efficacy, selectivity, or stability. This is for instance done by mutagenesis procedures where after the stability and/or binding efficacy of the resulting compounds are preferably tested and an improved AML-specific binding compound is selected. A skilled person is well capable of generating variants comprising at least one altered CDR sequence according to the invention. For instance, conservative amino acid substitution is applied. Examples of conservative amino acid substitution include the substitution of one hydrophobic residue such as isoleucine, valine, leucine or methionine for another hydrophobic residue, and the substitution of one polar residue for another polar residue, such as the substitution of arginine for lysine, glutamic acid for aspartic acid, or glutamine for asparagine.

Besides optimizing CDR sequences in order to improve binding efficacy or stability, at least one sequence in at least one of the framework regions can be optimized. This is preferably done in order to improve binding efficacy or stability. Framework sequences are for instance optimized by mutating a nucleic acid molecule encoding such framework sequence where after the characteristics of the resulting antibody - or functional part or functional equivalent - are preferably tested. This way, it is possible to obtain improved binding compounds. In a preferred embodiment, human germline sequences are used for framework regions in antibodies according to the invention. The use of human germline sequences minimizes the risk of immunogenicity of said antibodies, because these sequences are less likely to contain somatic alterations which are unique to individuals from which the framework regions are derived, and may cause an immunogenic response when applied to another human individual. Further provided is therefore a synthetic or recombinant antibody or functional part according to the invention, comprising at least one non-natural mutation in a framework region. Additionally, or alternatively, a synthetic or recombinant antibody or functional part according to the invention is provided that comprises at least one non-natural mutation in a constant region. By a "non-natural mutation" is meant that the resulting amino acid sequence does not occur in nature. Instead, it has been artificially produced.

A binding compound according to the present invention preferably comprises a human variable region. More preferably, said binding compound comprises a human constant region and a human variable region. Most preferably, said binding compound is a human antibody. The use of human AML-specific antibodies is advantageous over the use of non-human antibodies. The *in vivo* use of non-human antibodies for diagnosis and/or treatment of human diseases is hampered by a number of factors. In particular, the human body may recognize non-human antibodies as foreign, which will result in an immunogenic response against the non-human antibodies, resulting in adverse side effects and/or rapid clearance of the antibodies from the circulation. A human antibody diminishes the chance of side-effects when administered to a human individual and often results in a longer half-life in the circulation because of reduced clearance when compared to non-human antibodies. In another embodiment a binding compound according to the invention is a humanized antibody. In another embodiment a binding compound according to the invention is a chimeric antibody. In a chimeric antibody, sequences of interest, such as for instance an additional binding site of interest, are included into a binding compound according to the invention.

Further, binding compounds according to the invention are preferably monoclonal antibodies. A monoclonal antibody is an antibody consisting of a single molecular species. Monoclonal antibodies can be produced in large quantities by monoclonal antibody-producing cells or recombinant DNA technology.

Hence, variant binding compounds based on the preferred antibodies depicted in Table 1 and Figure 1 can also be generated, using techniques known in the art such as for instance mutagenesis. Typically, sequence variations between 80 and 99% are tolerated while maintaining a certain antigen specificity.

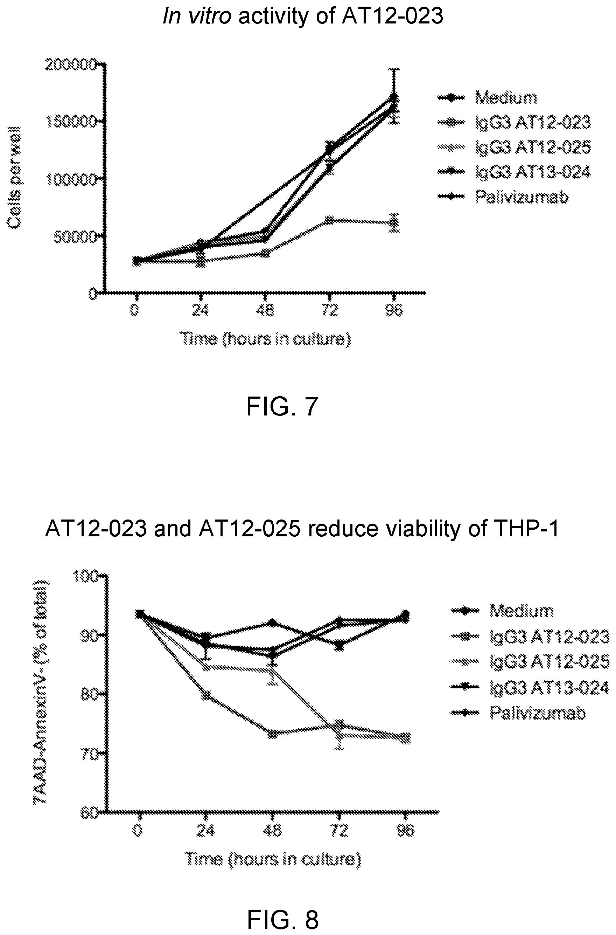

Disclosed herein is antibody AT12-023. This antibody is preferred because it is capable of efficiently binding and killing cells of the AML cell line THP-1, as shown in the Examples and Figures 7 and 8. This antibody is, therefore, particularly suitable for AML therapy and/or diagnosis. Interestingly, AT12-023 is of the IgG3 isotype and belongs to the VH4-34 family, which is a family of VH sequences known for their potential killing properties (Bhat et al, 1997). Antibody AT12-023 is also capable of efficiently binding primary AML blasts of at least four different FAB classifications (Table 3). The heavy chain CDR1, CDR2 and CDR3 sequences, and the light chain CDR1, CDR2 and CDR3 sequences of antibody AT12-023 are SEQ ID NOs 1, 14, 27, 40, 53 and 66, respectively, as depicted in Table 1. Further disclosed is therefore an isolated, synthetic or recombinant antibody, or a functional part or a functional equivalent thereof, which comprises:
- a heavy chain CDR1 sequence comprising a sequence which has at least 85% sequence identity with SEQ ID NO: 1; and
- a heavy chain CDR2 sequence comprising a sequence which has at least 85% sequence identity with SEQ ID NO: 14; and
- a heavy chain CDR3 sequence comprising a sequence which has at least 85% sequence identity with SEQ ID NO: 27; and
- a light chain CDR1 sequence comprising a sequence which has at least 85% sequence identity with SEQ ID NO: 40; and
- a light chain CDR2 sequence comprising a sequence which has at least 85% sequence identity with SEQ ID NO: 53; and
- a light chain CDR3 sequence comprising a sequence which has at least 85% sequence identity with SEQ ID NO: 66. Preferably, said sequence identities are at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described herein before, at least 1, 2 or 3 amino acid residues in the recited CDR sequences may vary while retaining the same kind of binding activity (in kind, not necessarily in amount). Hence, said heavy and light chain CDR 1, 2 and 3 sequences preferably contain antibody AT12-023-derived CDR sequences that deviate in no more than three, preferably no more than two, more preferably no more than one amino acid from the recited AT12-023 CDR sequences.

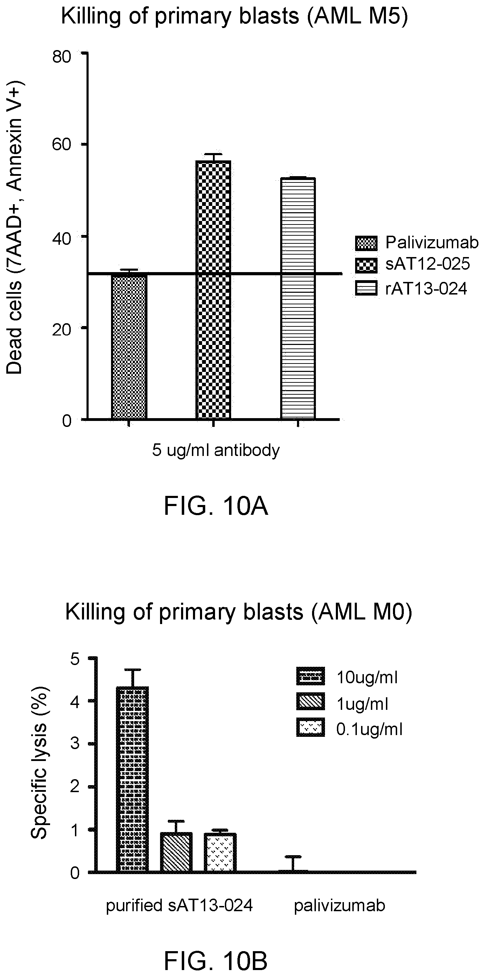

Also disclosed herein is antibody AT12-025. This antibody is preferred because it is capable of efficiently binding and killing cells of the AML cell line THP-1, as well as patient-derived primary AML blasts, as shown in the Examples and Figures 8 and 10. This antibody is, therefore, particularly suitable for AML therapy and/or diagnosis. Interestingly, AT12-025 is of the IgG3 isotype and belongs to the VH4-34 family, which is a family of VH sequences known for their potential killing properties (Bhat et al, 1997). The heavy chain CDR1, CDR2 and CDR3 sequences and the light chain CDR1, CDR2 and CDR3 sequences of antibody AT12-025 are SEQ ID NOs 2, 15, 28, 41, 54 and 67, respectively, as depicted in Table 1. Further disclosed is therefore an isolated, synthetic or recombinant antibody, or a functional part or a functional equivalent thereof, which comprises:
- a heavy chain CDR1 sequence comprising a sequence which has at least 80% sequence identity with SEQ ID NO: 2; and
- a heavy chain CDR2 sequence comprising a sequence which has at least 80% sequence identity with SEQ ID NO: 15; and
- a heavy chain CDR3 sequence comprising a sequence which has at least 80% sequence identity with SEQ ID NO: 28; and
- a light chain CDR1 sequence comprising a sequence which has at least 80% sequence identity with SEQ ID NO: 41; and
- a light chain CDR2 sequence comprising a sequence which has at least 80% sequence identity with SEQ ID NO: 54; and
- a light chain CDR3 sequence comprising a sequence which has at least 80% sequence identity with SEQ ID NO: 67. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT12-025 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT12-025 CDR sequences.

Also disclosed herein is antibody AT13-024. This antibody is preferred because it is capable of binding and killing patient-derived primary AML blasts, as shown in the Examples and Figure 10. This antibody is, therefore, particularly suitable for AML therapy and/or diagnosis. Interestingly, AT13-024 is of the IgG3 isotype and belongs to the VH3-30 family. The heavy chain CDR 1, CDR2 and CDR3 sequences and the light chain CDR1, CDR2 and CDR3 sequences of antibody AT13-024 are SEQ ID NOs 3, 16 29, 42, 55 and 68, respectively, as depicted in Table 1. Further disclosed is therefore an isolated, synthetic or recombinant antibody, or a functional part or a functional equivalent thereof, which comprises:
- a heavy chain CDR1 sequence comprising a sequence which has at least 80% sequence identity with SEQ ID NO: 3; and
- a heavy chain CDR2 sequence comprising a sequence which has at least 80% sequence identity with SEQ ID NO: 16; and
- a heavy chain CDR3 sequence comprising a sequence which has at least 80% sequence identity with SEQ ID NO: 29; and
- a light chain CDR1 sequence comprising a sequence which has at least 80% sequence identity with SEQ ID NO: 42; and
- a light chain CDR2 sequence comprising a sequence which has at least 80% sequence identity with SEQ ID NO: 55; and
- a light chain CDR3 sequence comprising a sequence which has at least 80% sequence identity with SEQ ID NO: 68. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-024 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-024 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 4; and
- a heavy chain CDR2 sequence comprising a sequence which has at least 80% sequence identity with SEQ ID NO: 17; and
- a heavy chain CDR3 sequence comprising a sequence which has at least 80% sequence identity with SEQ ID NO: 30; and
- a light chain CDR1 sequence comprising a sequence which has at least 80% sequence identity with SEQ ID NO: 43; and
- a light chain CDR2 sequence comprising a sequence which has at least 80% sequence identity with SEQ ID NO: 56; and
- a light chain CDR3 sequence comprising a sequence which has at least 80% sequence identity with SEQ ID NO: 69. These are the CDR sequences of antibody AT12-019. Antibody AT12-019, derived from a human AML patient in complete remission, is capable of efficiently binding intact AML cells, which makes a binding compound containing AT12-019-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT12-019 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT12-019 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 5; and
- a heavy chain CDR2 sequence comprising a sequence which has at least 80% sequence identity with SEQ ID NO: 18; and
- a heavy chain CDR3 sequence comprising a sequence which has at least 80% sequence identity with SEQ ID NO: 31; and
- a light chain CDR1 sequence comprising a sequence which has at least 80% sequence identity with SEQ ID NO: 44; and
- a light chain CDR2 sequence comprising a sequence which has at least 80% sequence identity with SEQ ID NO: 57; and
- a light chain CDR3 sequence comprising a sequence which has at least 80% sequence identity with SEQ ID NO: 70. These are the CDR sequences of antibody AT13-022. Interestingly, antibody AT13-022 is of the IgG3 isotype. Antibody AT13-022, derived from a human AML patient in complete remission, is capable of specifically binding intact AML cells, which makes a binding compound containing AT13-022-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-022CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-022 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 6; and
- a heavy chain CDR2 sequence comprising a sequence which has at least 80% sequence identity with SEQ ID NO: 19; and
- a heavy chain CDR3 sequence comprising a sequence which has at least 80% sequence identity with SEQ ID NO: 32; and
- a light chain CDR1 sequence comprising a sequence which has at least 80% sequence identity with SEQ ID NO: 45; and
- a light chain CDR2 sequence comprising a sequence which has at least 80% sequence identity with SEQ ID NO: 58; and
- a light chain CDR3 sequence comprising a sequence which has at least 80% sequence identity with SEQ ID NO: 71. These are the CDR sequences of antibody AT13-023. Interestingly, AT13-023 belongs to the VH4-34 family, which is a family of VH sequences known for their potential killing properties (Bhat et al, 1997). Antibody AT13-023, derived from a human AML patient in complete remission, is capable of specifically binding intact AML cells, which makes a binding compound containing AT13-023-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-023 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-023 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 7; and
- a heavy chain CDR2 sequence comprising a sequence which has at least 80% sequence identity with SEQ ID NO: 20; and
- a heavy chain CDR3 sequence comprising a sequence which has at least 80% sequence identity with SEQ ID NO: 33; and
- a light chain CDR1 sequence comprising a sequence which has at least 80% sequence identity with SEQ ID NO: 46; and
- a light chain CDR2 sequence comprising a sequence which has at least 80% sequence identity with SEQ ID NO: 59; and
- a light chain CDR3 sequence comprising a sequence which has at least 80% sequence identity with SEQ ID NO: 72. These are the CDR sequences of antibody AT13-031. Interestingly, AT13-031 belongs to the VH4-34 family, which is a family of VH sequences known for their potential killing properties (Bhat et al, 1997). Antibody AT13-031, derived from a human AML patient in complete remission, is capable of specifically binding intact AML cells. Moreover, this antibody is preferred because it is capable of efficiently binding and killing cells of the AML cell line THP-1. Hence, a binding compound containing AT13-031-derived CDR sequences is particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-031 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-031 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 8; and
- a heavy chain CDR2 sequence comprising a sequence which has at least 80% sequence identity with SEQ ID NO: 21; and
- a heavy chain CDR3 sequence comprising a sequence which has at least 80% sequence identity with SEQ ID NO: 34; and
- a light chain CDR1 sequence comprising a sequence which has at least 80% sequence identity with SEQ ID NO: 47; and
- a light chain CDR2 sequence comprising a sequence which has at least 80% sequence identity with SEQ ID NO: 60; and
- a light chain CDR3 sequence comprising a sequence which has at least 80% sequence identity with SEQ ID NO: 73. These are the CDR sequences of antibody AT12-020. Interestingly, antibody AT12-020 is of the IgG3 isotype. Antibody AT12-020, derived from a human AML patient in complete remission, is capable of specifically binding intact AML cells, which makes a binding compound containing AT12-020-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT12-020 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT12-020 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 9; and
- a heavy chain CDR2 sequence comprising a sequence which has at least 80% sequence identity with SEQ ID NO: 22; and
- a heavy chain CDR3 sequence comprising a sequence which has at least 80% sequence identity with SEQ ID NO: 35; and
- a light chain CDR1 sequence comprising a sequence which has at least 80% sequence identity with SEQ ID NO: 48; and
- a light chain CDR2 sequence comprising a sequence which has at least 80% sequence identity with SEQ ID NO: 61; and
- a light chain CDR3 sequence comprising a sequence which has at least 80% sequence identity with SEQ ID NO: 74. These are the CDR sequences of antibody AT13-033. Antibody AT13-033, derived from a human AML patient in complete remission, is capable of specifically binding intact AML cells. Moreover, this antibody is preferred because it is capable of efficiently binding and killing cells of the AML cell line THP-1. Hence, a binding compound containing AT13-033-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-033 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-033 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 10; and
- a heavy chain CDR2 sequence comprising a sequence which has at least 80% sequence identity with SEQ ID NO: 23; and
- a heavy chain CDR3 sequence comprising a sequence which has at least 80% sequence identity with SEQ ID NO: 36; and
- a light chain CDR1 sequence comprising a sequence which has at least 80% sequence identity with SEQ ID NO: 49; and
- a light chain CDR2 sequence comprising a sequence which has at least 80% sequence identity with SEQ ID NO: 62; and
- a light chain CDR3 sequence comprising a sequence which has at least 80% sequence identity with SEQ ID NO: 75. These are the CDR sequences of antibody AT13-034. Antibody AT13-034, derived from a human AML patient in complete remission, is capable of specifically binding intact AML cells, which makes a binding compound containing AT13-034-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-034 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-034 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 11; and
- a heavy chain CDR2 sequence comprising a sequence which has at least 80% sequence identity with SEQ ID NO: 24; and
- a heavy chain CDR3 sequence comprising a sequence which has at least 80% sequence identity with SEQ ID NO: 37; and
- a light chain CDR1 sequence comprising a sequence which has at least 80% sequence identity with SEQ ID NO: 50; and
- a light chain CDR2 sequence comprising a sequence which has at least 80% sequence identity with SEQ ID NO: 63; and
- a light chain CDR3 sequence comprising a sequence which has at least 80% sequence identity with SEQ ID NO: 76. These are the CDR sequences of antibody AT13-035. Antibody AT13-035, derived from a human AML patient in complete remission, is capable of specifically binding intact AML cells. Moreover, this antibody is preferred because it is capable of efficiently binding and killing cells of the AML cell line THP-1. Hence, a binding compound containing AT13-035-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-035 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-035 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 12; and
- a heavy chain CDR2 sequence comprising a sequence which has at least 80% sequence identity with SEQ ID NO: 25; and
- a heavy chain CDR3 sequence comprising a sequence which has at least 80% sequence identity with SEQ ID NO: 38; and
- a light chain CDR1 sequence comprising a sequence which has at least 80% sequence identity with SEQ ID NO: 51; and
- a light chain CDR2 sequence comprising a sequence which has at least 80% sequence identity with SEQ ID NO: 64; and
- a light chain CDR3 sequence comprising a sequence which has at least 80% sequence identity with SEQ ID NO: 77. These are the CDR sequences of antibody AT13-036. Antibody AT13-036, derived from a human AML patient in complete remission, is capable of specifically binding intact AML cells. Moreover, this antibody is preferred because it is capable of efficiently binding and killing cells of the AML cell line THP-1. Hence, a binding compound containing AT13-036-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-036 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-036 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 13; and
- a heavy chain CDR2 sequence comprising a sequence which has at least 80% sequence identity with SEQ ID NO: 26; and
- a heavy chain CDR3 sequence comprising a sequence which has at least 80% sequence identity with SEQ ID NO: 39; and
- a light chain CDR1 sequence comprising a sequence which has at least 80% sequence identity with SEQ ID NO: 52; and
- a light chain CDR2 sequence comprising a sequence which has at least 80% sequence identity with SEQ ID NO: 65; and
- a light chain CDR3 sequence comprising a sequence which has at least 80% sequence identity with SEQ ID NO: 78. These are the CDR sequences of antibody AT13-037. Antibody AT13-037, derived from a human AML patient in complete remission, is capable of specifically binding intact AML cells. Moreover, this antibody is preferred because it is capable of efficiently binding and killing cells of the AML cell line THP-1, as well as patient-derived primary AML blasts, as shown in the Examples. Hence, a binding compound containing AT13-037-derived CDR sequences particularly suitable for AML therapy and/or diagnosis. Such binding compound is for instance used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT13-037 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT13-037 CDR sequences.

The invention provides an isolated, synthetic or recombinant antibody according to the claims, or a functional part thereof, which comprises:
- a heavy chain CDR1 sequence as defined by SEQ ID NO: 209; and
- a heavy chain CDR2 sequence as defined by SEQ ID NO: 213; and
- a heavy chain CDR3 sequence as defined by SEQ ID NO: 217; and
- a light chain CDR1 sequence as defined by SEQ ID NO: 221; and
- a light chain CDR2 sequence as defined by SEQ ID NO: 225; and
- a light chain CDR3 sequence as defined by SEQ ID NO: 229. These are the CDR sequences of antibody AT14-013. Antibody AT14-013, derived from a human AML patient in complete remission, is capable of specifically binding primary AML blasts of at least three different FAB classifications, which makes a binding compound containing AT14-013-derived CDR sequences particularly suitable for AML therapy and/or diagnosis. Such binding compound is for instance used for diminishing proliferation of AML cells, preferably independently from apoptosis and/or independently from ADCC, CDC or phagocytosis by macrophages or dendritic cells. Preferably, said binding compound is used for inducing death of AML cells. In some embodiments, such binding compound is used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 210; and
- a heavy chain CDR2 sequence comprising a sequence which has at least 80% sequence identity with SEQ ID NO: 214; and
- a heavy chain CDR3 sequence comprising a sequence which has at least 80% sequence identity with SEQ ID NO: 218; and
- a light chain CDR1 sequence comprising a sequence which has at least 80% sequence identity with SEQ ID NO: 222; and
- a light chain CDR2 sequence comprising a sequence which has at least 80% sequence identity with SEQ ID NO: 226; and
- a light chain CDR3 sequence comprising a sequence which has at least 80% sequence identity with SEQ ID NO: 230. These are the CDR sequences of antibody AT14-014. Interestingly, antibody AT14-014 is of the IgG3 isotype. Antibody AT14-014, derived from a human AML patient in complete remission, is capable of specifically binding intact AML cells, which makes a binding compound containing AT14-014-derived CDR sequences particularly suitable for AML therapy and/or diagnosis. Such binding compound is for instance used for diminishing proliferation of AML cells, preferably independently from apoptosis and/or independently from ADCC, CDC or phagocytosis by macrophages or dendritic cells. Preferably, said binding compound is used for inducing death of AML cells. In some embodiments, such binding compound is used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT14-014 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT14-014 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 211; and
- a heavy chain CDR2 sequence comprising a sequence which has at least 80% sequence identity with SEQ ID NO: 215; and
- a heavy chain CDR3 sequence comprising a sequence which has at least 80% sequence identity with SEQ ID NO: 219; and
- a light chain CDR1 sequence comprising a sequence which has at least 80% sequence identity with SEQ ID NO: 223; and
- a light chain CDR2 sequence comprising a sequence which has at least 80% sequence identity with SEQ ID NO: 227; and
- a light chain CDR3 sequence comprising a sequence which has at least 80% sequence identity with SEQ ID NO: 231. These are the CDR sequences of antibody AT14-015. Interestingly, antibody AT14-015 is of the IgG3 isotype. Antibody AT14-015, derived from a human AML patient in complete remission, is capable of specifically binding intact AML cells, which makes a binding compound containing AT14-015-derived CDR sequences particularly suitable for AML therapy and/or diagnosis. Such binding compound is for instance used for diminishing proliferation of AML cells, preferably independently from apoptosis and/or independently from ADCC, CDC or phagocytosis by macrophages or dendritic cells. Preferably, said binding compound is used for inducing death of AML cells. In some embodiments, such binding compound is used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT14-015 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT14-015 CDR sequences.

Further disclosed is an isolated, synthetic or recombinant antibody, or a functional part or a functional equivalent thereof, which comprises:
- a heavy chain CDR1 sequence comprising a sequence which has at least 80% sequence identity with SEQ ID NO: 212; and
- a heavy chain CDR2 sequence comprising a sequence which has at least 80% sequence identity with SEQ ID NO: 216; and
- a heavy chain CDR3 sequence comprising a sequence which has at least 80% sequence identity with SEQ ID NO: 220; and
- a light chain CDR1 sequence comprising a sequence which has at least 80% sequence identity with SEQ ID NO: 224; and
- a light chain CDR2 sequence comprising a sequence which has at least 80% sequence identity with SEQ ID NO: 228; and
- a light chain CDR3 sequence comprising a sequence which has at least 80% sequence identity with SEQ ID NO: 232. These are the CDR sequences of antibody AT14-016. Interestingly, antibody AT14-016 is of the IgG3 isotype. Antibody AT14-016, derived from a human AML patient in complete remission, is capable of specifically binding intact AML cells, which makes a binding compound containing AT14-016-derived CDR sequences particularly suitable for AML therapy and/or diagnosis. Such binding compound is for instance used for diminishing proliferation of AML cells, preferably independently from apoptosis and/or independently from ADCC, CDC or phagocytosis by macrophages or dendritic cells. Preferably, said binding compound is used for inducing death of AML cells. In some embodiments, such binding compound is used as an antibody-drug conjugate (ADC), such that a toxic compound will be directed to AML cells, or it is used by inducing CDC and/or ADCC. Alternatively, or additionally, such binding compound can be used to mark AML cells for specific phagocytosis by tumor-associated myeloid cells such as macrophages or dendritic cells, which cells can subsequently induce an AML specific immune response. Again, said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%. As described above, at least 1, 2 or 3 amino acid residues in the recited AT14-016 CDR sequences may vary while retaining the same kind of binding activity (in kind, not necessarily in amount). Hence, the above mentioned heavy and light chain CDR 1, 2 and 3 sequences preferably contain CDR sequences that deviate in no more than three, preferably no more than two, more preferably no more than one amino acid from the recited AT14-016 CDR sequences.

Preferably, an antibody according to the invention comprises a variable heavy chain sequence and/or a variable light chain sequence of AT14-013 as depicted in Table 1 or Figure 1, or a sequence which has at least 90% sequence identity thereto. As shown in Table 1, the variable heavy chain sequences of antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016 are SEQ ID NOs 79-91 and 233-236, respectively. The variable light chain sequences of antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016 are SEQ ID NOs 92-104 and 237-240, respectively. Also provided is, therefore, an antibody or functional part thereof according to the invention, comprising a variable heavy chain sequence having at least 90% sequence identity with a sequence comprising SEQ ID NO: 233, and/or comprising a variable light chain sequence having at least 90% sequence identity with a sequence comprising SEQ ID NO: 237, or sequences that are at least 91%, more preferably at least 92%, more preferably at least 93%, more preferably at least 94%, more preferably at least 95%, more preferably at least 96%, more preferably at least 97%, more preferably at least 98%, more preferably at least 99%, or even 100% identical to these heavy chain or light chain sequences. The higher the identity, the more closely an antibody resembles AT14-013.

Antibody AT14-013 has the variable heavy chain sequence of SEQ ID NO: 233 and the variable light chain sequence of SEQ ID NO: 237 as depicted in Table 1. Further provided is therefore a binding compound according to the present invention, having a variable heavy chain sequence having at least 90%, or at least 91%, or at least 92%, or at least 93%, or at least 94%, or at least 95%, or at least 96%, or at least 97%, or at least 98%, or at least 99%, or 100% sequence identity with SEQ ID NO: 233. Preferably, said binding compound also comprises a variable light chain sequence having at least 90%, or at least 91%, or at least 92%, or at least 93%, or at least 94%, or at least 95%, or at least 96%, or at least 97%, or at least 98%, or at least 99%, sequence identity with SEQ ID NO: 237.

Further disclosed is an isolated, synthetic or recombinant nucleic acid molecule with a length of at least 15 nucleotides, or a functional equivalent thereof, encoding at least one CDR sequence of an antibody or functional part according to the invention. Preferably a nucleic acid molecule as disclosed herein has a length of at least 30 nucleotides, more preferably at least 50 nucleotides, more preferably at least 75 nucleotides. A nucleic acid molecule according to the disclosure is for instance isolated from a B-cell which is capable of producing an antibody according to the invention. Said B-cell preferably produces antibody AT12-023, AT12-025, AT13-024, AT12-019, AT13-022, AT13-023, AT13-031, AT12-020, AT13-033, AT13-034, AT13-035, AT13-036, AT13-037 AT14-013, AT14-014, AT14-015, or AT14-016. In a preferred aspect a nucleic acid molecule encoding at least the heavy chain CDR3 sequence and the light chain CDR3 sequence of an antibody according to the invention is disclosed.

As used herein the term "an isolated, synthetic or recombinant nucleic acid molecule with a length of at least 15 nucleotides, or a functional equivalent thereof, encoding at least one CDR sequence of an antibody or functional part or functional equivalent according to the disclosure" is herein also referred to as "a nucleic acid molecule or functional equivalent according to the disclosure".

As used herein, a nucleic acid molecule or nucleic acid sequence of the invention preferably comprises a chain of nucleotides, more preferably DNA, cDNA or RNA. In other embodiments a nucleic acid molecule or nucleic acid sequence of the invention comprises other kinds of nucleic acid structures such as for instance a DNA/RNA helix, peptide nucleic acid (PNA), locked nucleic acid (LNA) and/or a ribozyme. Such other nucleic acid structures are referred to as functional equivalents of a nucleic acid sequence. The term "functional equivalent of a nucleic acid molecule" thus encompasses a chain comprising non-natural nucleotides, modified nucleotides and/or non-nucleotide building blocks which exhibit the same function as natural nucleotides.

Nucleic acid sequences encoding heavy chain and light chain CDRs of antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016 are depicted in Table 1. Nucleic acid molecules encoding a heavy or light chain CDR of an antibody according to the invention which differ from the CDR nucleic acid sequences depicted in Table 1 but have nucleic acid codons which encode the same amino acids of said heavy or light chain CDR are also encompassed by the invention. Such nucleic acid molecules for instance comprise nucleic acid sequences that have been codon optimized for a producer cell, such as for instance *E.coli* or Chinese hamster ovary (CHO) cells, NSO cells (a mouse myeloma) or 293(T) cells, enabling high scale production of binding compounds according to the invention. It should be noted that antibody production can be done by any recombinant antibody production system; the four producer cell systems mentioned here are only a few examples of the many systems that are available to date. As used herein, the term "codon" means a triplet of nucleotides (or functional equivalents thereof) that encode a specific amino acid residue. The term "codon optimized" means that one or more codons from the original, human nucleic acid sequence are replaced by one or more codons that are preferred by a certain antibody production system. These replacement codons preferably encode the same amino acid residue as the original human codon that has been replaced. Alternatively, one or more replacement codons encode a different amino acid residue. This preferably results in conservative amino acid substitution, although this is not necessary. Typically, in constant regions and framework regions one or more amino acid substitutions are generally allowed. In CDR regions, codons are used that encode the same amino acid residue as the original human codon that has been replaced.

A nucleic acid molecule or vector(s) according to the invention encode(s) at least the variable heavy chain sequence and/or the variable light chain sequence of an antibody or functional part according to the invention. Preferably, said nucleic acid molecule(s) or vector(s) encode(s) at least the variable heavy chain sequence and the variable light chain sequence of an antibody or functional part according to the invention. In one embodiment, a nucleic acid molecule or one or more vectors according to the invention are provided which comprise(s) a sequence which has at least 80% sequence identity with a sequence comprising SEQ ID NO 265 and/or a sequence which has at least 80% sequence identity with a sequence comprising SEQ ID NO 269. Said sequence identities are preferably at least 85%, more preferably at least 86%, more preferably at least 87%, more preferably at least 88%, more preferably at least 89%, more preferably at least 90%, more preferably at least 91%, more preferably at least 92%, more preferably at least 93%, more preferably at least 94%, more preferably at least 95%, more preferably at least 96%, more preferably at least 97%, more preferably at least 98%, more preferably at least 99%, more preferably 100%.

In some embodiments, nucleic acid molecules and vectors are provided that encode an antibody or functional part according to the invention. Further provided is therefore a nucleic acid molecule, or one or more vectors, that encode(s) antibody AT14-013, or functional part thereof. In some embodiments, said nucleic acid molecule or vector(s) is/are codon optimized for a non-human recombinant expression system.

Further provided is a vector comprising a nucleic acid molecule according to the invention. As used herein "a vector comprising a nucleic acid molecule according to the invention" is also referred to as "a vector according to the invention". These terms encompass one or more vector(s) according to the invention, comprising one or more nucleic acid molecule(s) according to the invention. As used herein, the singular term "a" encompasses the term "one or more".

Methods for constructing vectors comprising one or more nucleic acid molecule(s) according to the invention are well known in the art. Non-limiting examples of vectors suitable for generating a vector of the invention are retroviral and lentiviral vectors. Such vectors are suitable for a variety of applications. For instance, one or more vectors of the invention comprising a therapeutically beneficial nucleic acid sequence according to the invention is/are suitable for prophylactic or therapeutic applications against AML. Administration of such vector(s) to an individual, preferably a human, in need thereof results in expression of said prophylactic or therapeutic nucleic acid sequence *in vivo* resulting in at least partial treatment or prophylaxis against AML. Said vector(s) can also be used in applications involving *in vitro* expression of a nucleic acid molecule of interest, for instance for (commercial) production of antibodies according to the invention. Also provided is therefore an isolated or recombinant cell, or a non-human animal, comprising at least one nucleic acid molecule, or at least one vector, according to the invention.

A nucleic acid molecule or a vector according to the invention is particularly useful for generating antibodies or functional parts according to the invention, which are specific for AML. This is for instance done by introducing such nucleic acid molecule or vector(s) into a cell so that the cell's nucleic acid translation machinery will produce the encoded antibodies or functional parts. In one embodiment, a nucleic acid molecule or vector encoding a heavy and/or light chain according to the invention is expressed in so called producer cells, such as for instance E.coli, CHO, NSO or 293(T) cells, some of which are adapted to commercial antibody production. Of note, any recombinant antibody production system is suitable; these four producer cell systems mentioned are only a few examples of the many systems that are available to date. As described herein before, in such cases it is preferred to use nucleic acid molecules wherein the original human sequences as provided herein are codon optimized for the producer cell. Proliferation of said producer cells results in a producer cell line capable of producing binding compounds according to the invention. Preferably, said producer cell line is suitable for producing antibodies for use in humans. Hence, said producer cell line is preferably free of pathogenic agents such as pathogenic microorganisms. Most preferably, antibodies consisting of human sequences are generated using at least one nucleic acid molecule or vector according to the invention.

An isolated or recombinant antibody producing cell capable of producing a binding compound according to the invention is therefore also provided. Such cell typically comprises at least one nucleic acid molecule or vector according to the invention, which preferably contains a nucleic acid sequence that is codon optimized for said cell. An antibody producing cell is defined herein as a cell which is capable of producing and/or secreting antibodies or functional parts or functional equivalents thereof, and/or which is capable of developing into a cell which is capable of producing and/or secreting antibodies or functional parts or functional equivalents thereof. An antibody producing cell according to the invention is preferably a producer cell which is adapted to commercial antibody production. As explained above, said producer cell is preferably suitable for producing antibodies for use in humans. A method for producing an antibody or functional part according to the invention is therefore also provided, said method comprising providing a cell, preferably an antibody producing cell, with a nucleic acid molecule or a vector according to the invention, and allowing said cell to translate said nucleic acid molecule or vector, thereby producing said antibody or functional part according to the invention. A method according to the invention preferably further comprises a step of harvesting, purifying and/or isolating said antibody or functional part according to the invention. Obtained binding compounds according to the invention are preferably used in human therapy, optionally after additional purifying, isolation or processing steps.

Another aspect of the invention provides an antibody or functional part according to the invention, which is coupled to another compound. In one embodiment, a binding compound according to the invention is coupled to another therapeutic moiety, such as a chemotherapeutic drug or other toxic compound or radioactive compound, to form a so called "antibody-drug conjugate". In another embodiment, a moiety that is coupled to a binding compound according to the invention is an immunomodulatory molecule such as for instance a CD3-specific antibody. Such CD3-specific antibody is capable of binding T cells and, if coupled to a binding compound according to the invention, it will target T cells to AML cells, thereby enhancing an anti-leukemic T-cell response. This provides an even stronger anti-AML effect. One preferred embodiment of the invention therefore provides a bispecific or multispecific binding compound, comprising an AML-specific binding compound according to the present invention and an immunomodulatory molecule, preferably a CD3-specific binding compound. Another preferred embodiment provides an anti-AML compound, said compound comprising a binding compound according to the present invention, which is specific for AML cells, and a toxic moiety. In some other embodiments, a binding compound according to the present invention is coupled to a label. This allows detection of myeloproliferative cells, such as AML cells, using such labeled binding compound. Other embodiments provide a binding compound according to the invention that is coupled to another AML-binding compound. In some embodiments, such other AML-binding compound is also a binding compound according to the present invention. Provided is therefore a compound comprising two binding compounds according to the invention that are coupled to each other. This is, however, not necessary since a binding compound according to the invention can also be coupled to other AML-binding compounds, such as currently known antibodies that bind AML cells. Bispecific compounds according to the invention allow, for instance, for increased binding of AML cells, especially when the two coupled binding compounds are specific for different epitopes on AML cells. Such bispecific compound is thus very suitable for therapeutic or diagnostic applications. It is also possible to use bispecific compounds according to the invention in assays wherein different AML cells are bound to the same bispecific binding compound.

In one embodiment, a synthetic or recombinant antibody is provided, or a functional part thereof, which comprises one Fab fragment of an antibody according to the present invention, and one Fab fragment of another antibody according to the invention. The resulting binding compound is specific for AML cells, but each Fab arm will typically bind its own epitope. In some embodiments, the epitopes recognized by the Fab fragments are different from each other. In another embodiment, the epitopes are the same. The Fab arms may bind the epitopes with different affinity. Alternatively, the Fab arms bind their epitopes with essentially the same affinity, meaning that the K_{D} of the Fab arms differ no more than 30%, preferably no more than 20% or no more than 10% from each other.

Said other moiety, for example a chemotherapeutic agent or a CD3-specific antibody, is preferably coupled to a binding compound according to the invention via a linker such as for instance an acid-labile hydrazone linker, or via a peptide linker like citruline-valine, or through a thioether linkage, or by sortase catalized transamidation, which is described in detail in WO 2010/087994.

Sortase catalized transamidation involves engineering of a sortase recognition site (LPETGG) on the heavy chain of an antibody, preferably on the C-terminal part of the heavy chain, and on the moiety to be coupled to said antibody. The antibody and the moiety further typically contain a GGGGS sequence and a tag for purification purposes, such as a HIS tag. Subsequently sortase mediated transamidation is performed followed by click chemistry linkage. In a sortase catalized transaminidation, "click chemistry linkage" typically involves chemical coupling of, for instance, an alkyne-containing reagent and, for instance, an azide-containing reagent which are added by sortase through addition of glycines to the sortase motif on the heavy chain of the antibody and to a sortase motif on the moiety (such as a protein, peptide or antibody) to be coupled to the antibody. In one embodiment, the invention therefore provides an antibody according to the invention wherein a sortase recognition site (LPETGG) is engineered on the heavy chain of the antibody, preferably on the C-terminal part of the heavy chain, the antibody preferably further containing a GGGGS sequence and a purification tag, such as a HIS tag.

In another embodiment a binding compound according to the invention is coupled to another moiety via a thioether linkage. In such case, one or more cysteines are preferably incorporated into a binding compound according to the invention. Cysteines contain a thiol group and, therefore, incorporation of one or more cysteines into a binding compound according to the invention, or replacement of one or more amino acids by one or more cysteines of a binding compound according to the invention, enable coupling of said binding compound to another moiety. Said one or more cysteines are preferably introduced into a binding compound according to the invention at a position where it does not significantly influence folding of said binding compound, and does not significantly alter antigen binding or effector function. The invention therefore also provides a binding compound according to the invention wherein at least one amino acid other than cysteine has been replaced by a cysteine.

In one embodiment, an AML-specific binding compound according to the present invention is coupled to at least one other AML-specific binding compound according to the present invention. Such bispecific or multispecific binding compound provides a stronger anti-AML effect.

Binding compounds according to the present invention are suitable for use against myeloproliferative disorders such as AML, or acute leukemia's that developed from myelodysplastic syndrome, chronic myeloid leukemia, myelofibrosis or other, non-malignant myeloproliferative syndromes. As some of the antibodies also bind to non-myeloid, lymphoproliferative malignancies such as multiple myeloma and B-non Hodgkin lymphoma (B-NHL), they are suitable for use against these disorders as well. Binding compounds according to the present invention are therefore particularly suitable for use as a medicine or prophylactic agent. Preferably, binding compounds according to the invention are used which consist of human sequences, in order to reduce the chance of adverse side effects when human individuals are treated. Such human sequences can be isolated from a human or synthetically or recombinantly produced based on the sequence of human antibodies, optionally using codon optimized nucleic acid sequences that encode the same amino acids as the original human nucleic acid sequence. Provided is therefore an antibody or functional part according to the invention for use as a medicament and/or prophylactic agent. Said antibody preferably comprises antibody AT14-013. Also provided is a nucleic acid molecule according to the invention or a vector according to the invention comprising such nucleic acid, or a cell according to the invention, for use as a medicament and/or prophylactic agent. When (a vector comprising) one or more nucleic acid molecule(s) according to the invention is/are administered, the nucleic acid molecule(s) will be translated in *situ* by the host's machinery into a binding compound according to the invention. Produced binding compounds according to the invention are capable of preventing and/or counteracting myeloproliferative disorders such as AML and lymphoproliferative disorders such as for instance lymphoma, B-NHL and multiple myeloma. Further provided is therefore an antibody or functional part according to the invention, or a nucleic acid molecule according to the invention, or a vector or a cell according to the invention, for use in a method for at least in part treating or preventing AML or B-NHL. As described herein before, such disorder can be treated or prevented by using cytotoxic binding compounds according to the invention.

In some embodiments, a binding compound according to the invention is coupled to a therapeutic moiety, such as a chemotherapeutic drug or other toxic compound or a radioactive compound or an immunomodulatory molecule such as for instance a CD3-specific antibody, to form a so called "antibody-drug conjugate" or a "chimeric antigen receptor (CAR) T cell", respectively, which is able to counteract a myeloproliferative or lymphoproliferative disorder.

A binding compound according to the invention, or a nucleic acid molecule according to the invention, or at least one vector or cell according to the invention, is preferably used for at least in part treating and/or preventing AML. As used herein the term "at least in part treating and/or preventing AML" includes counteracting AML tumor growth and/or alleviating symptoms resulting from the presence of AML cells in a patient. Also provided is therefore a use of an antibody or functional part according to the invention, or of a nucleic acid molecule according to the invention, or of at least one vector or cell according to the invention, for the preparation of a medicament and/or prophylactic agent for at least in part treating and/or preventing AML. Further provided is an antibody or functional part according to the invention, or a nucleic acid molecule according to the invention, or at least one vector or cell according to the invention, for use in a method for at least in part treating and/or preventing AML.

Preferred antibodies for use in any of the recited methods are antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016.

The invention further provides a composition comprising an antibody or functional part according to the invention. A composition comprising a nucleic acid molecule according to the invention is also provided, as well as a composition comprising a vector or a cell according to the invention. In some embodiments a composition according to the invention comprises at least two antibodies, functional parts according to the invention.

A composition according to the present invention preferably comprises a pharmaceutical composition. Said pharmaceutical composition preferably also comprises a pharmaceutical acceptable carrier, diluent and/or excipient. Non-limiting examples of suitable carriers for instance comprise keyhole limpet haemocyanin (KLH), serum albumin (e.g. BSA or RSA) and ovalbumin. In one preferred embodiment said suitable carrier comprises a solution, like for example saline. A pharmaceutical composition according to the invention is preferably suitable for human use.

Further disclosed is a method for at least in part treating and/or preventing a myeloproliferative or lymphoproliferative disorder, comprising administering to an individual in need thereof a therapeutically effective amount of an antibody or functional part or functional equivalent according to the invention, and/or a nucleic acid molecule or functional equivalent thereof according to the invention, and/or a vector or cell according to the invention, and/or a composition according to the invention. As used herein, an "individual" or "subject" is a human or an animal, preferably a human AML patient. Said composition is preferably a pharmaceutical composition according to the invention.

A binding compound and/or composition according to the present invention is particularly suitable for administering to immune compromised individuals with an increased risk of complications, such as individuals that have undergone chemotherapy, particularly infants and elderly people. A binding compound, or a nucleic acid molecule, or a vector, and/or a pharmaceutical composition according to the invention is preferably administered via one or more injections. Typical doses of administration of a binding compound according to the invention are between 0.1 and 10 mg per kg body weight.

A binding compound according to the invention is also particularly suitable for diagnostic use. For instance, if an individual, preferably a human, is suspected of suffering from a myeloproliferative disorder such as AML or a lymphoproliferative disorder such as B-NHL or multiple myeloma, a sample, such as a blood or tissue sample, from said individual can be tested for the presence of myeloproliferative cells or lymphoproliferative cells, using a binding compound according to the invention. Preferably, said sample is mixed with a binding compound according to the invention, which will specifically bind to myeloproliferative cells. Myeloproliferative cells or lymphoproliferative cells bound to a binding compound according to the invention can be isolated from the sample and/or detected using any method known in the art, for example, but not limited to, isolation using magnetic beads, streptavidin-coated beads, or isolation through the use of secondary antibodies immobilized on a column. Alternatively, or additionally, a binding compound according to the invention is labeled in order to be able to detect said antibody, for instance, but not limited to, fluorescently labeled, enzymatically labeled, or radioactively labeled. Alternatively, a binding compound according to the invention is detected using a labeled secondary antibody which is directed against said binding compound. If binding of said antibody is detected, it is indicative for the presence of myeloproliferative or lymphoproliferative cells. The invention therefore provides an antibody or functional part according to the invention, or a nucleic acid molecule according to the invention, or a vector or a cell according to the invention, for use in detectingAML or B-NHL. Also provided is an antibody or functional part according to the invention, or a nucleic acid molecule according to the invention, or a vector or a cell according to the invention, for use in diagnosis ofAML or B-NHL.

A use of an antibody or functional part according to the invention, or use of a nucleic acid molecule according to the invention, or use of a vector or a cell according to the invention, for determining whether a sample comprises AML cells or B-NHL cells is also provided, as well as a method for detecting AML cells or B-NHL cells using an antibody or functional part according to the invention. The invention further provides a method for determining whether AML cells or B-NHL cells are present in a sample comprising:
- contacting said sample with an antibody or functional part according to the invention, and
- allowing said antibody or functional part to bind myeloproliferative cells or lymphoproliferative cells, if present, and
- determining whether or not AML cells or B-NHL cells are bound to said antibody or functional part, thereby determining whether or not AML cells orB-NHL cells are present in said sample.

In a further embodiment it is determined whether an individual is suffering fromAML or B-NHL. Also provided is therefore a method for determining whether an individual is suffering fromAML or B-NHL, comprising:
- contacting a sample from said individual with an antibody or functional part according to the invention, and
- allowing said antibody or functional part to bind AML cells or B-NHL cells, if present, and
- determining whether or not AML cells or B-NHL cells are bound to said antibody or functional part, thereby determining whether or nor said individual is suffering fromAML or B-NHL. Preferably said individual is a human.

Screening assays as provided herein can be performed using methods such as for instance enzyme-linked immunosorbent assays (ELISA), radio-immuno assays (RIA), Western Blot assays and immunohistochemical staining assays. These assays are well known in the art and therefore need no further explanation. Variations or adaptations of ELISA, RIA, Western blot assay and immunohistochemical staining assay are also known in the art.

In a method according to the invention, said myeloproliferative disorder is preferably AML and said myeloproliferative cells are preferably AML cells. Moreover, said lymphoproliferative disorder is preferably B- non-Hodgkin lymphoma and said lymphoproliferative cells are preferablyB- non-Hodgkin lymphoma cells.

The above-mentioned procedures for detecting myeloproliferative or lymphoproliferative cells using binding compounds according to the present invention are for instance particularly suitable for determining whether a patient suffering from a myeloproliferative disorder or lymphoproliferative disorder who has received medical treatment, such as for instance an AML patient who has been treated against AML, for instance an AML patient who has received immunotherapy such as a stem cell transplantation or donor-lymphocyte infusion, has a GvL response. Until to date, there are no diagnostic tools to test for the presence of a potent GvL response in a treated patient. Such diagnostic tool is much needed, for instance because: 1) It will allow early identification of allogeneic SCT recipients at high risk for relapse, at a time-point before relapse has occurred thereby allowing earlier interventions such as tapering of immunosuppressants or donor-lymphocyte infusions; 2) It will allow titrating such donor lymphocyte infusions until anti-leukemia antibodies do appear; and 3) It will offer hope for allogeneic SCT recipients at a time they often suffer from one of many SCT-related complications when the presence of a potent GvL response can be demonstrated. Nowadays patients have to wait and see whether or not a relapse occurs, and there is no way to predict relapse of disease. The availability of a test for determing whether a patient has a GvL response will therefore greatly improve the clinical care of SCT patients, affecting prognosis and quality of life. Further provided is, therefore, a use of a binding compound according to the invention for determining whether a sample is indicative for a GvL response. Also provided is a use of a binding compound according to the invention for determining whether or not an AML patient has a GvL response, and a use of a binding compound according to the invention for determining whether anti-AML therapy or anti-B-NHL therapy is efficaceous. This is for instance done by determining whether a sample (for instance a blood or tissue sample) from a patient (that has for instance received a SCT or DLI or any other form of immunotherapy) contains AML cells or B-NHL cells. The absence of myeloproliferative or lymphoproliferative cells indicates that said patient has a GvL response. Further disclosed is, therefore, a method for determining whether a patient suffering from a myeloproliferative disorder or a lymphoproliferative disorder who has received immunotherapy against said disorder has a GvL response, the method comprising contacting a sample from said patient with an antibody or functional part according to the invention, and allowing said antibody or functional part to bind myeloproliferative or lymphoproliferative cells, if present, and determining whether or not myeloproliferative cells or lymphoproliferative cells are bound to said antibody or functional part, thereby determining whether or not said individual has a GvL response, whereby the absence of myeloproliferative or lymphoproliferative cells is indicative for a GvL response and the presence of myeloproliferative or lymphoproliferative cells is indicative for a lack of, or insufficient (ineffective), GvL response. In some embodiments, said lymphoproliferative disorder is lymphoma, B-NHL, or multiple myeloma. In some embodiments, said myeloproliferative disorder is AML. Also provided is, therefore, a method for determining whether an AML patient that has received anti-AML immunotherapy has a GvL response, the method comprising contacting a sample from said AML patient with an antibody or functional part according to the invention, and allowing said antibody or functional part to bind AML cells, if present, and determining whether or not AML cells are bound to said antibody or functional part, thereby determining whether or nor said individual has a GvL response, whereby the absence of AML cells is indicative for a GvL response and the presence of AML response is indicative for a lack of, or insufficient (ineffective), GvL response. Preferably said individual is a human. Alternatively, binding compounds according to the present invention that are labeled with a detectable moiety, such as for instance with a copper compound, are administered to an AML patient that has received anti-AML immunotherapy such as a SCT or DLI, and it is subsequently determined whether said labeled antibody is bound to AML cells of said patient *in vivo,* for instance using a PET scan. The absence of bound binding compounds is indicative for a GvL response and the presence of bound binding compounds is indicative for a lack of, or insufficient (ineffective), GvL response.

It is also possible to determine the amount of antibody belonging to the VH4-34 family before and after anti-AML immunotherapy. If the amount of antibody belonging to the VH4-34 family, which is a family of VH sequences known for their potential killing properties (Bhat et al, 1997), is significantly raised after immunotherapy, it indicates that a GvL response is present. Further disclosed is, therefore, a method for determining whether an AML patient that has received anti-AML immunotherapy has a GvL response, the method comprising determining the amount of antibody belonging to the VH4-34 family before and after anti-AML immunotherapy, and determining whether the amount of antibody belonging to the VH4-34 family is significantly raised after immunotherapy. If this is the case, it is concluded that a GvL response is present in said patient. If this is not the case, it is concluded that a GvL response is lacking, or not sufficient.

The invention is further explained in the following examples. These examples do not limit the scope of the invention, but merely serve to clarify the invention.

### Brief description of the drawings

Figure 1. Sequences of antibodies AT12-023, AT12-025, AT13-024, AT12-019, AT13-022, AT13-023, AT13-031, AT12-020, AT13-033, AT13-034, AT13-035, AT13-036, AT13-037, AT14-013, AT14-014, AT14-015 and AT14-016.

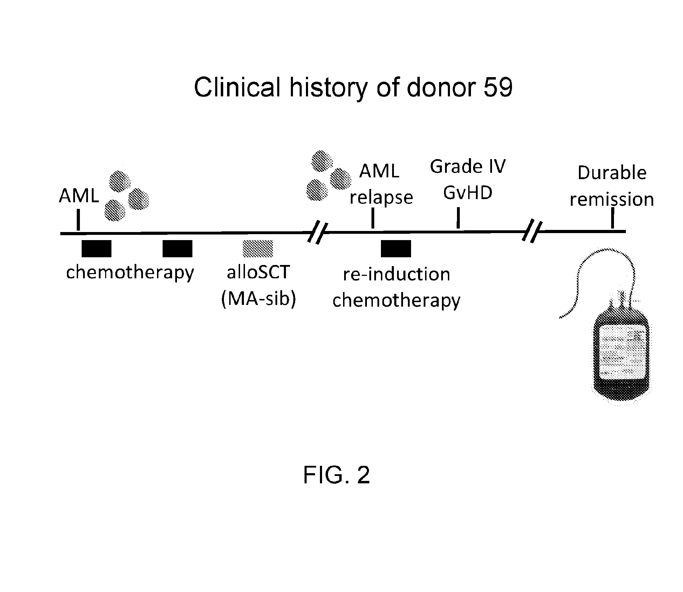

Figure 2. 36 year old patient with AML, who obtained complete remission after the first cycle of induction chemotherapy and received a myeloablative (MA) allogeneic stem cell transplantation (SCT) from her HLA-matched brother (sib) as consolidation therapy. AML relapse occurred however 5 months post-transplantation. Re-induction chemotherapy resulted again in complete remission and in severe, grade IV graft versus host disease (GvHD) of the liver and grade I GvHD of the skin. The observation that she has remained in complete remission for more than three years now implies that she has developed a potent graft versus leukemia response. B lymphocytes were isolated from a phlebotomy product obtained about 1,5 years after the one cycle of re-induction chemotherapy.

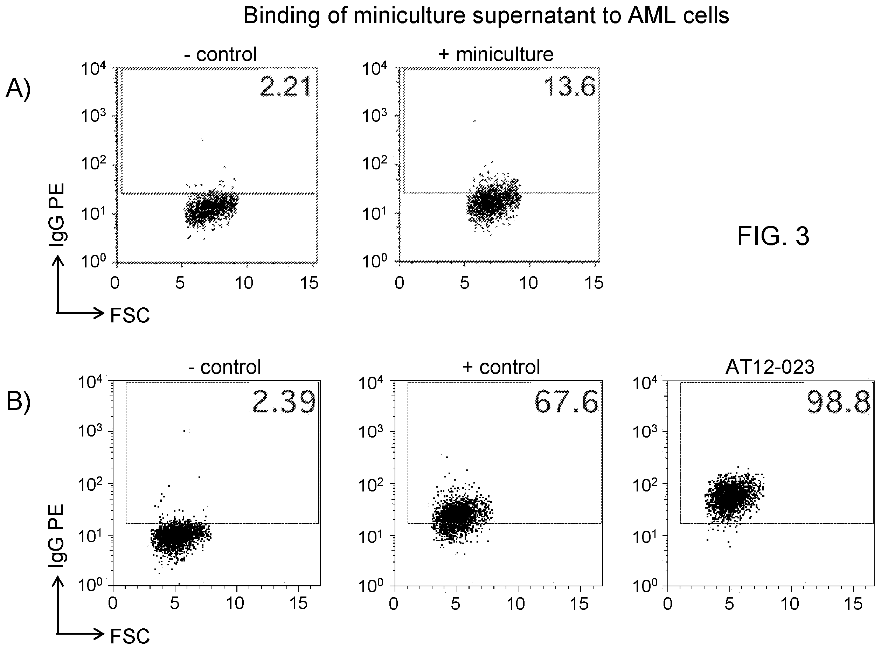

Figure 3. a) Supernatant of one of the minicultures (20 or 40 cells per well) binding to the AML cell line THP-1. b) B cells from this miniculture were plated into 1 cell/well solutions and supernatants again screened for binding to THP-1. THP-1 express HLA-DR, that was used in this screening experiment as a positive control. As a negative control an influenza-specific monoclonal antibody was used.

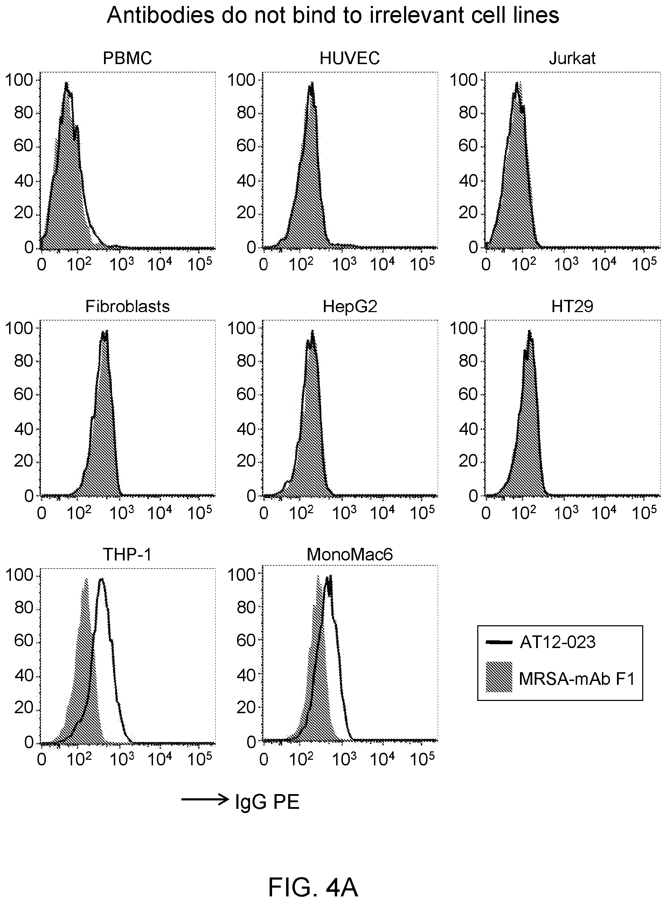

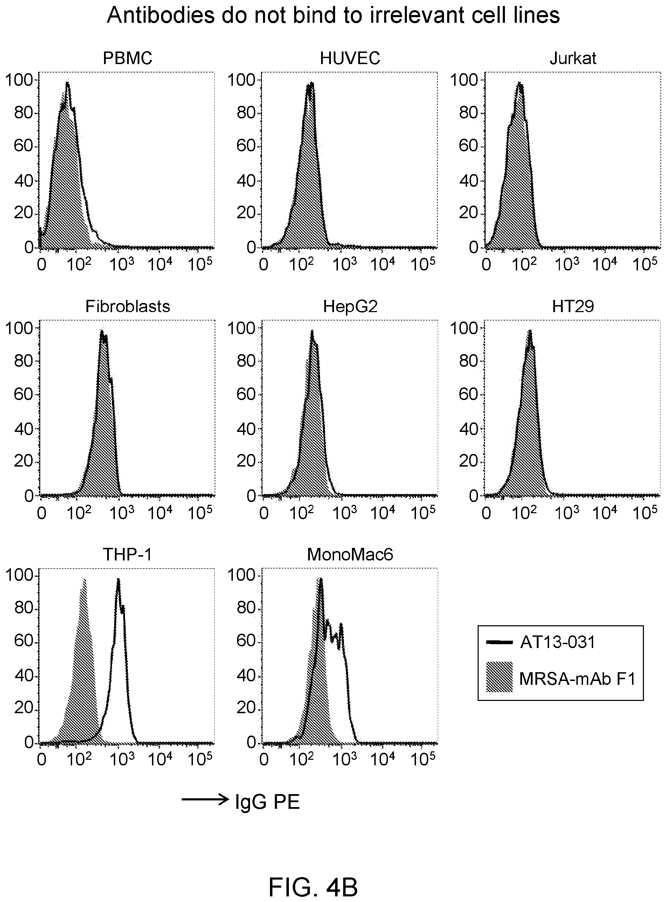

Figure 4. Two examples of the AML specific antibodies identified. AT12-023 (a) and AT13-031 (b) bind to AML cell lines THP-1 and MonoMac6, but not to healthy peripheral blood mononuclear cells (PBMC), endothelial cells (human umbilical vein endothelial cells: HUVEC), the T cell line Jurkat, primary fibroblasts, hepatocytes (liver hepatocellular carcinoma cell line: HepG2) or the colon adenocarcinoma cell line HT-29. The MRSA-mAb F1 is an in-house generated human IgG3 mAb that is used as control antibody.

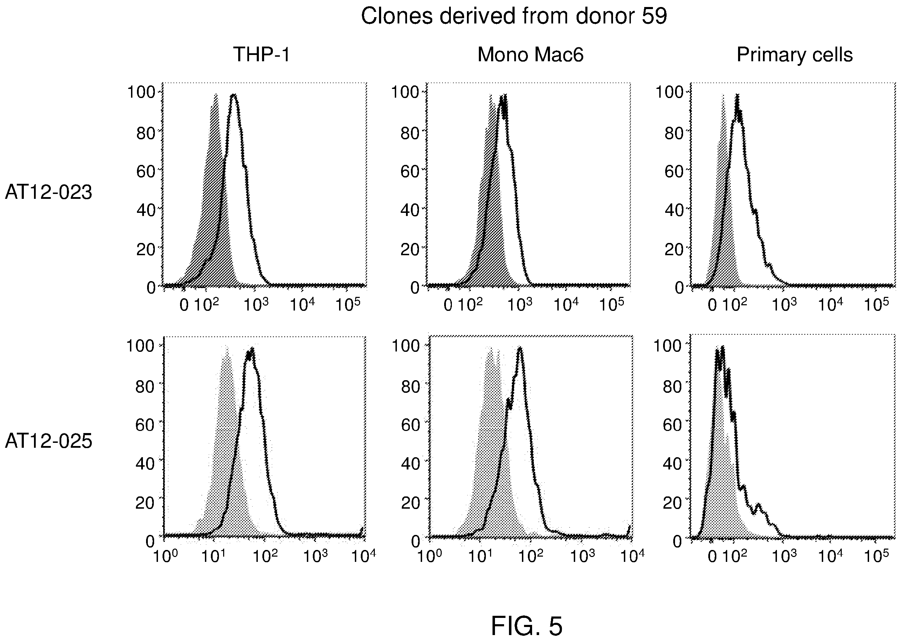

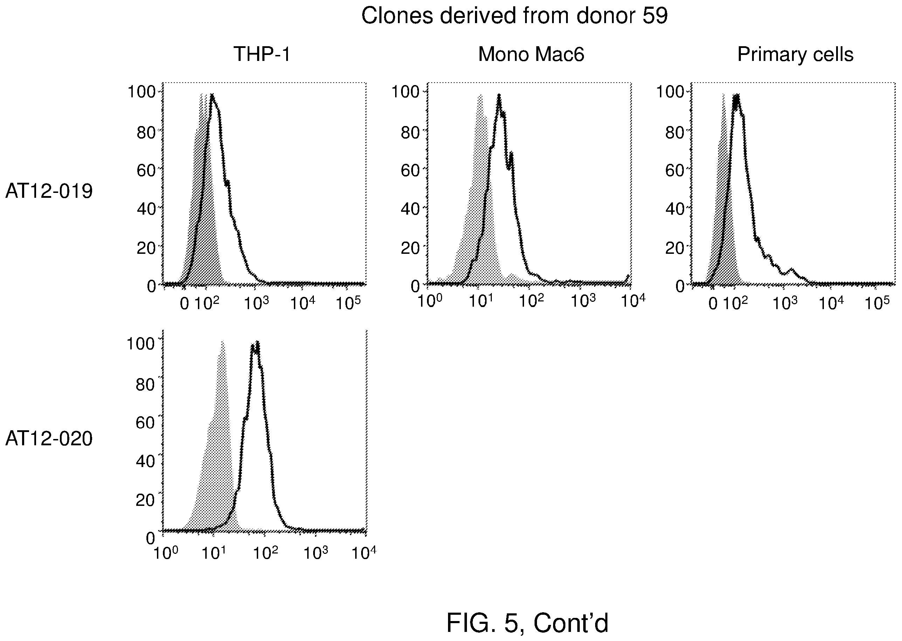

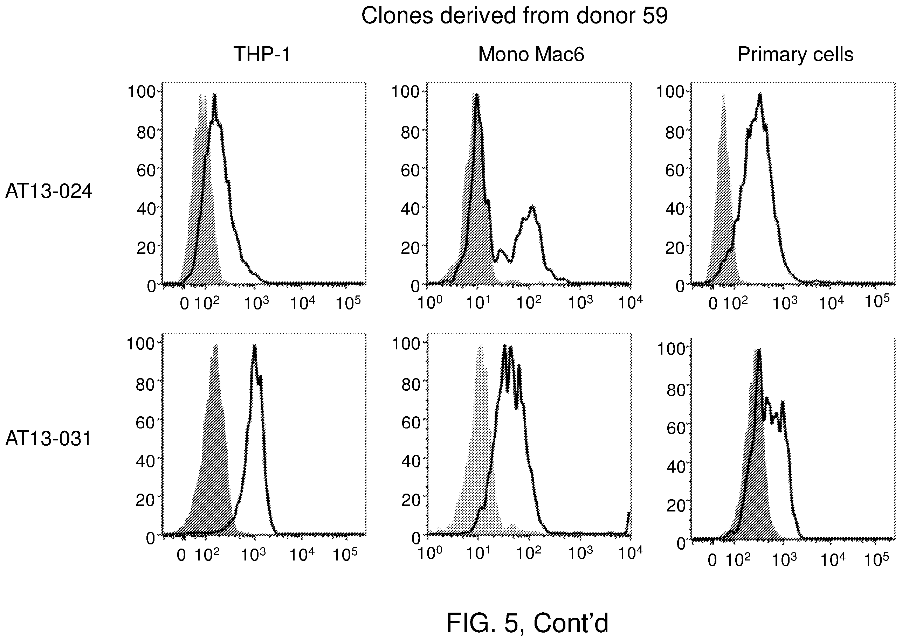

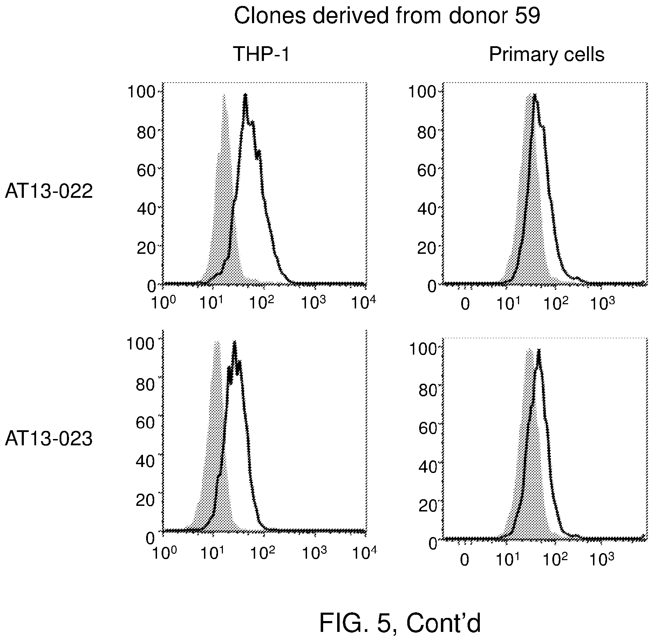

Figure 5. Antibodies derived from donor 59 bind to the AML cell lines THP-1 and MonoMac6, and to primary leukemic blasts isolated from newly diagnosed AML patients (ranging from FAB classification M0-M4). See also table 3 for a more detailed overview.

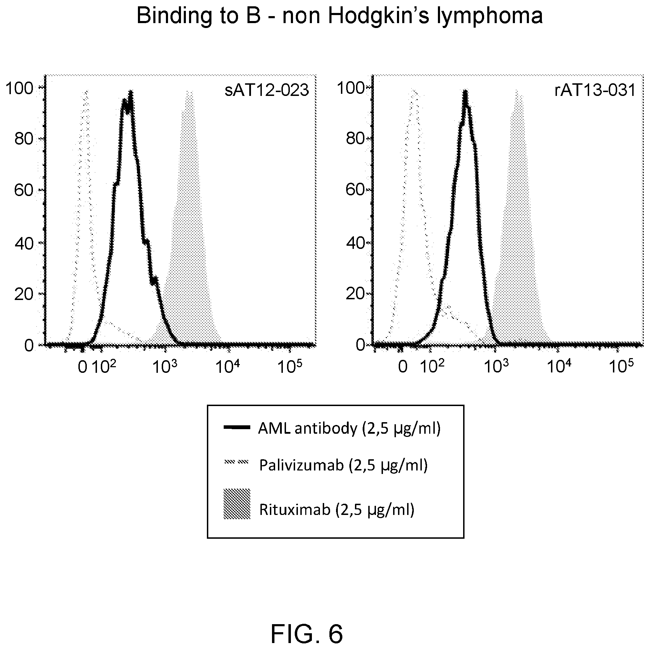

Figure 6. Some of the antibodies also bind to other hematological malignancies. Here, binding of AT12-023 (antibodies isolated from supernatant of B cell clone) and AT13-031 (recombinant antibody) to B-non Hodgkin lymphoma (B-NHL) cells that were freshly isolated from a lymphoma patient is shown. The antibodies did not bind to non-malignant B cells (not shown in this experiment). The RSV antibody Palivizumab was used as a negative control, and Rituximab, a CD20-antibody specifically binding B-cell lymphoma, as a positive control. See for more details of antibody binding to other hematologic malignancies table 4.
Figure 7. THP-1 cells were cultered in medium alone or with antibodies added at a concentration of 5 µg/ml at day 0. In the presence of AT12-023, THP-1 cells showed significant growth inhibition. Depicted are total cell numbers in culture.
Figure 8. When added at day 0 of the culture, antibodies AT12-023 and AT12-025 strongly reduced the viability of THP-1 cells. Viability is depicted as the proportion of cells double-negative for cell-death markers Annexin V and 7AAD.

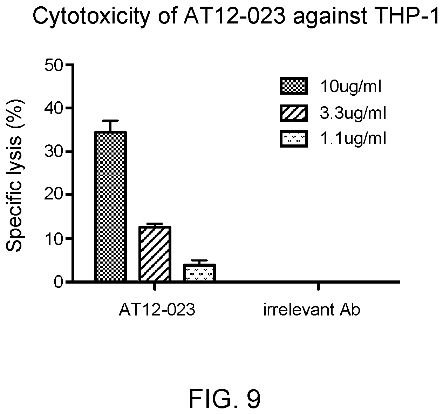

Figure 9. To quantify the amount of THP-1 cells specifically killed by AT12-023 we used a calcein AM release assay. Briefly, before THP-1 cells were incubated with AT12-023, the THP-1 cells were loaded with calcein AM, that is released when cells are dying and the cell membranbe becomes instable. Lysis was read out after 4 hours of incubation and was related to the background death of calcein-loaded cells as measured with an irrelevant antibody.
Figure 10. a) Besides killing of THP-1 cells, AT12-025 does also induce death of primary tumor cells, similar to AT13-024. Leukemic blasts, isolated from a patient with AML-M5 at diagnosis, were incubated with AT12-025 or AT13-024 (5 ug/ml) for 24 hours at 37°C, after which cells were stained with 7AAD and AnnexinV to determine the amount of dead cells (7AAD and Annexin V double-positive). b) In addition, Lactate dehydrogenase (LDH) can be used as a marker to determine dying cells. Leukemic blasts, isolated from a patient with AML-M0, were incubated with AT13-024 or the RSV antibody palivizumab for 18 hrs, after which LDH release was measured. These two experiments show that AT13-024, but not palivizumab, induced death of leukemic blasts and that this property was not restricted to one type of AML but includes different FAB classifications (M0 to M5).

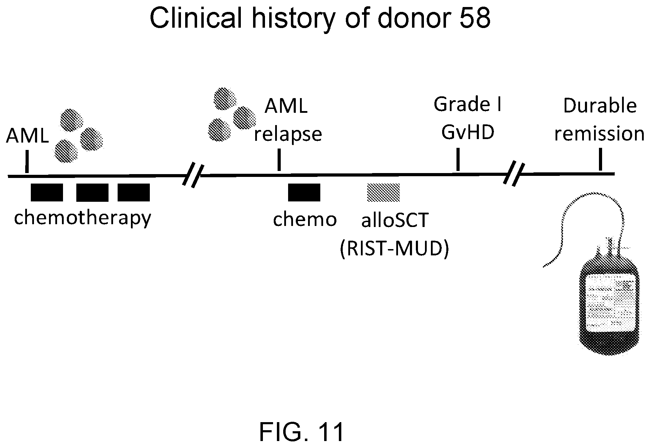

Figure 11. Donor 58 was diagnosed with AML-M5, for which she received three cycles of chemotherapy. She remained in complete remission for about a year, after which the AML relapsed. She received one cycle of re-induction chemotherapy, followed by an allogeneic reduced intensity stem cell transplantation (RIST) from a matched unrelated donor (MUD). She developed GvHD grade I of the liver and has remained in complete remission for almost 4 years now, implicating that she has developed a potent graft versus leukemia response. B cells were isolated from a phlebotomy product obtained from this patient about 3 years post-SCT.

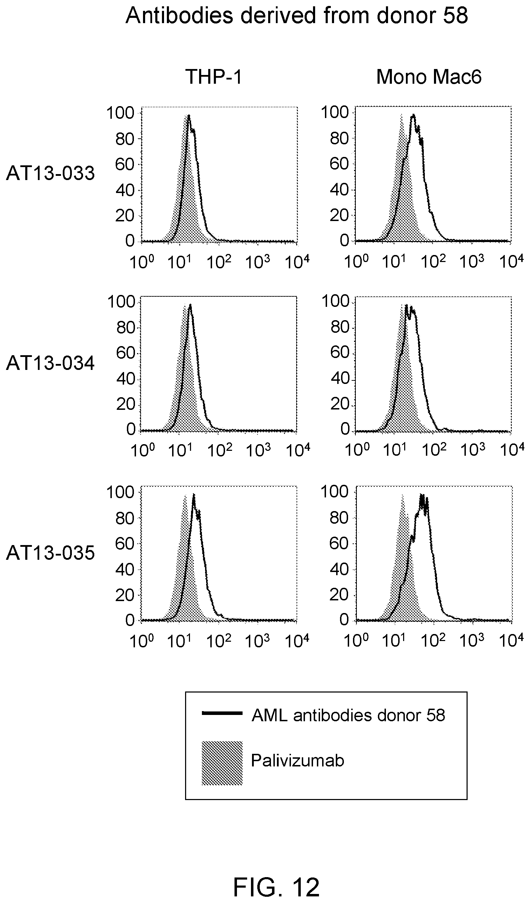

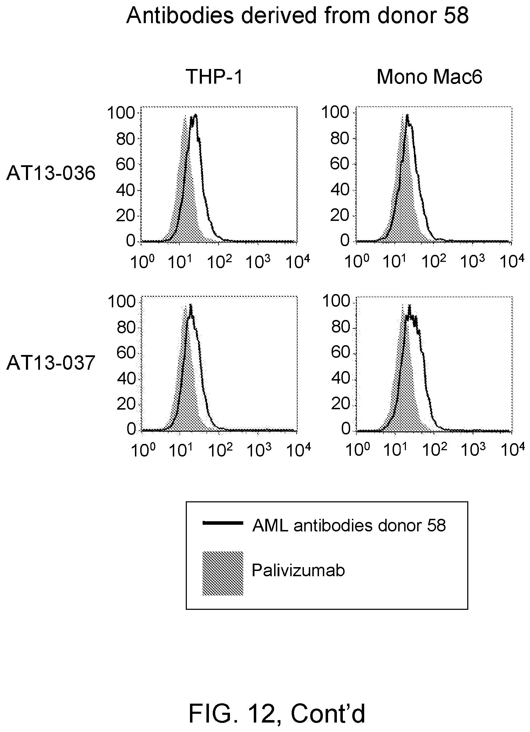

Figure 12. Antibodies derived from donor 58 bind to the AML cell lines THP-1 and MonoMac6. Palivizumab, the commercially available RSV specific antibody, was used as a negative control.

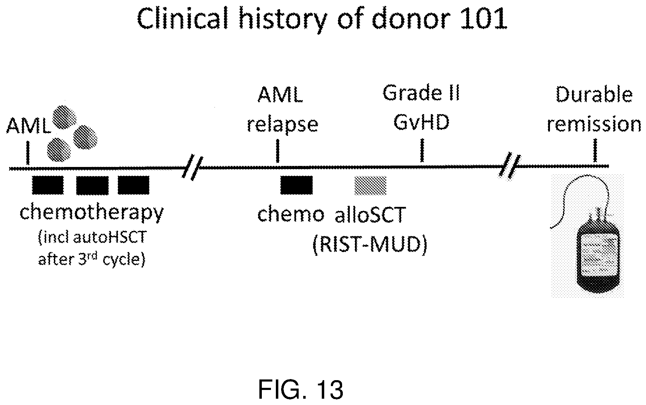

Figure 13. Clinical history of a third patient with a potent GvL response. Donor 101 was diagnosed with an intermediate-risk AML (no cytogenetic or molecular abnormalities; FAB classification AML-M5) at the age of 49 years. He received two courses of chemotherapy and one course of consolidation chemotherapy followed by an autologous HSCT, as there was no HLA-matched sibling stem cell donor available. Fourteen months after the first diagnosis his disease relapsed. He obtained complete remission after one cycle of high-dose cytarabine, after which he received a rediced-intensity allogeneic HSCT with a matched unrelated donor (RIST-MUD). Six weeks later he developed acute GvHD of skin, liver and intestine (stage 1; grade II) that responded well to corticosteroid therapy. B cells were isolated from a phlebotomy product obtained from this patient 38 months after the allogeneic HSCT. He has maintained durable remission until today.

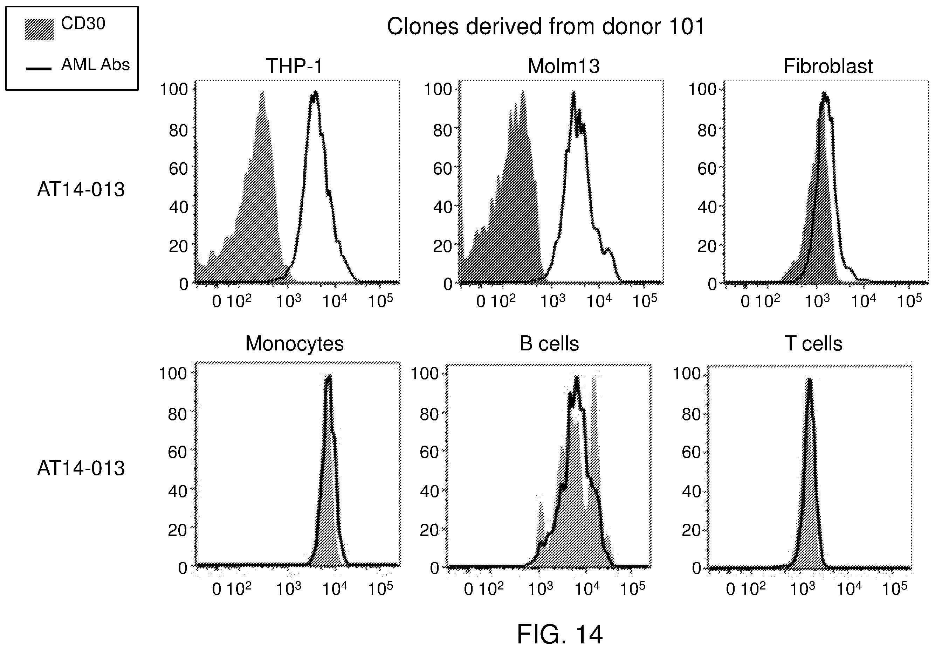

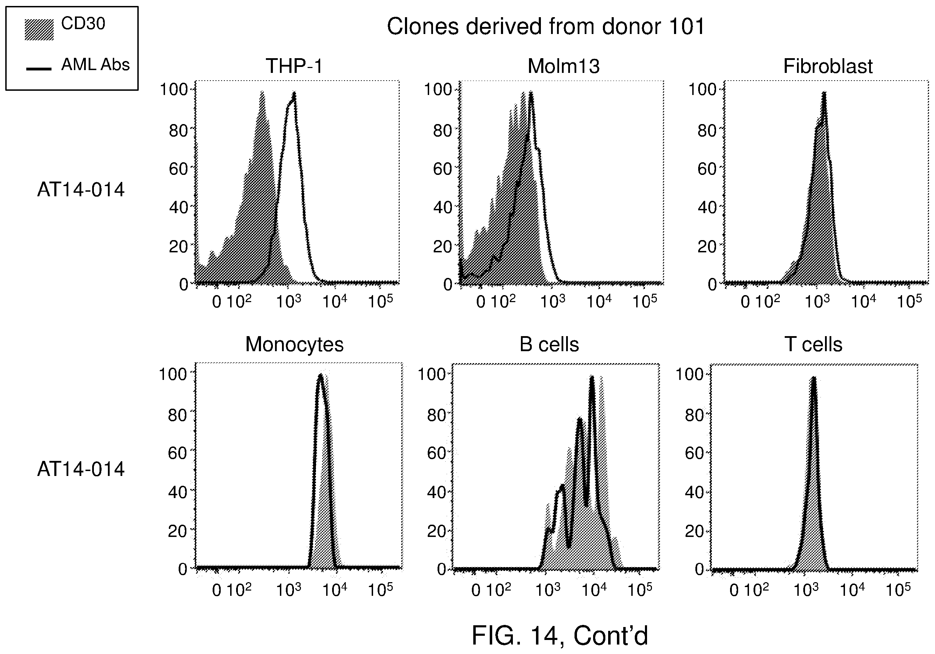

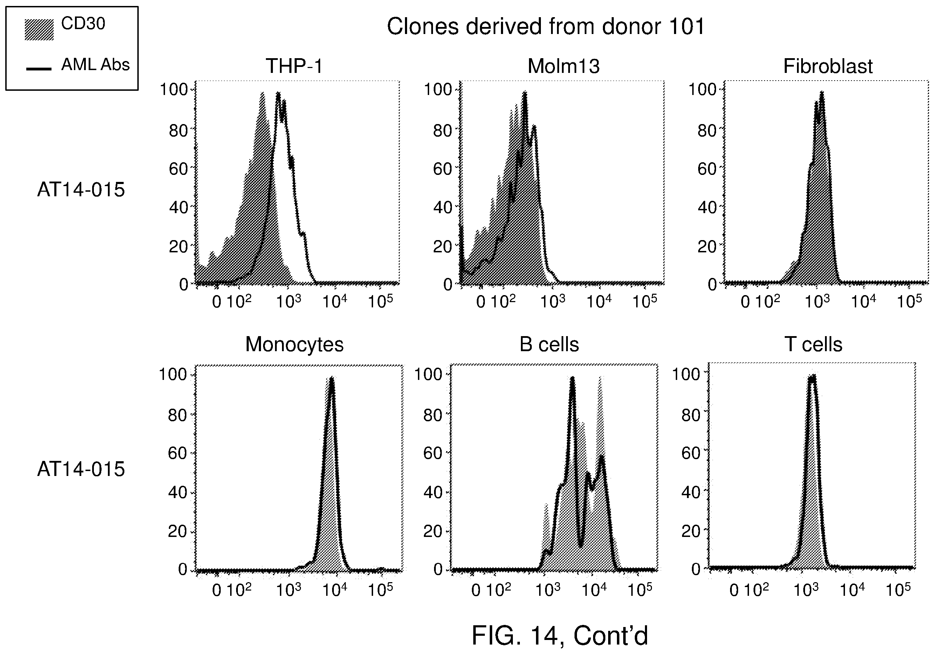

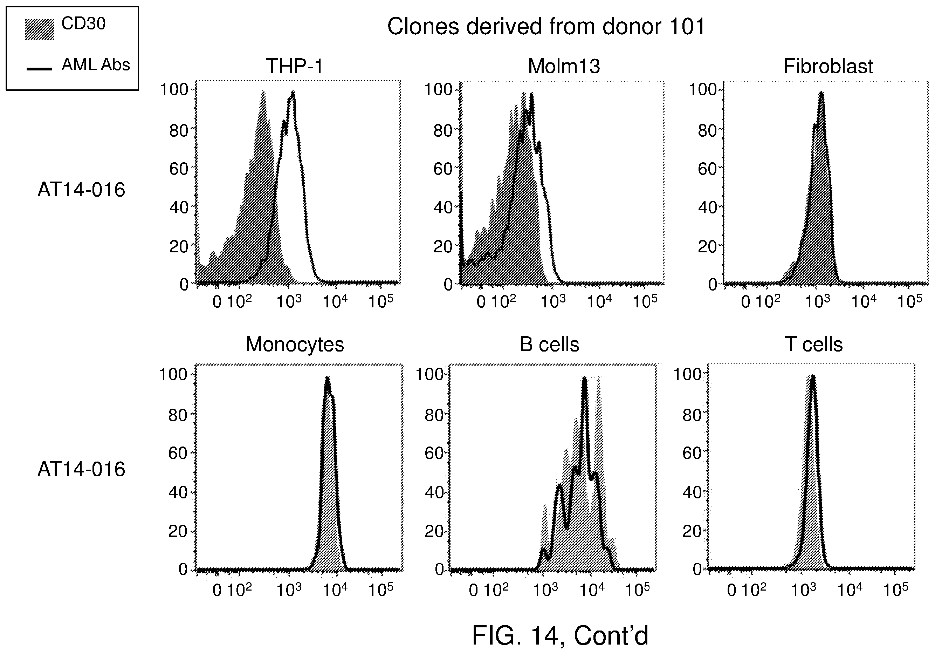

Figure 14. Antibodies derived from donor 101 bind to the AML cell lines THP-1 and Molm 13, but not to fibroblasts, monocytes, B cells and T cells. An in-house produced human antibody against CD30 was used as a negative control. AT14-013 showed minor reactivity with fibroblasts, compared to its high binding to the AML cell lines. See tables 7 and 8 for an overview of the binding capacities of the antibodies derived from donor 101.

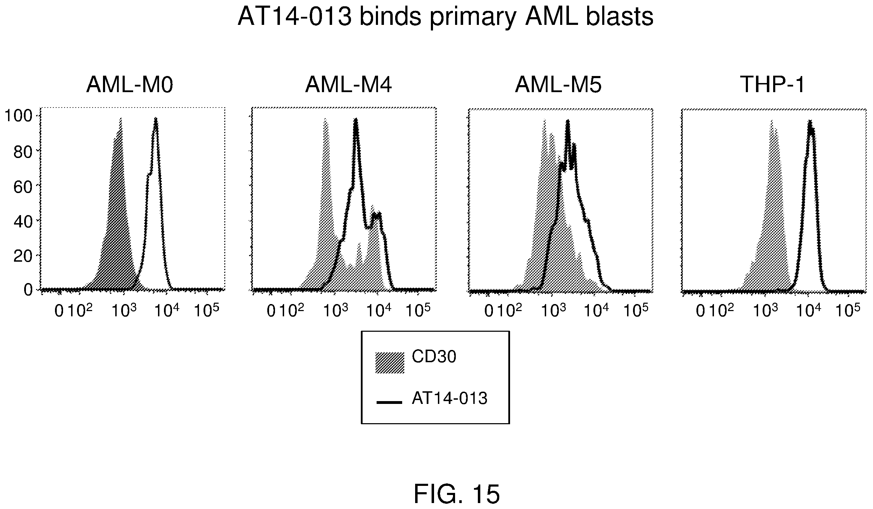

Figure 15. AML-specific antibody AT14-013 (donor 101) binds primary leukemic blasts isolated from newly diagnosed AML patients (FAB classifications M0-M5). Negative control: in-house generated CD30 antibody. See table 7 for an overview of the primary AML blast binding capacities of the antibodies derived from donor 101.

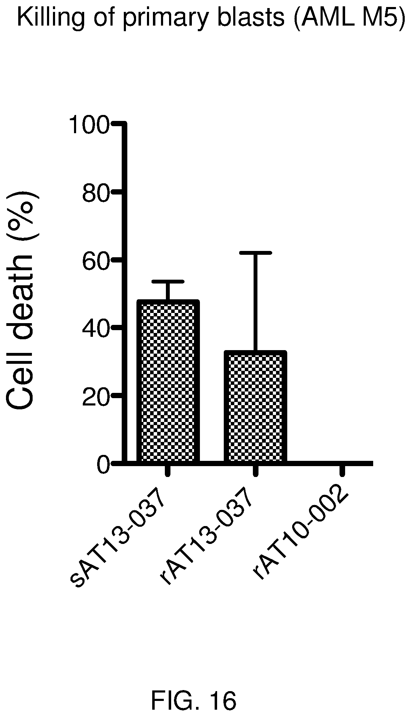

Figure 16. Antibody AT13-037 induces death of primary blasts. Leukemic blasts, isolated from an AML patient at diagnosis (BL-038; AML-M5), were incubated with antibodies purified from the supernatants of the parental B cell clone (sAT13-037) or with the recombinant antibody (rAT13-037) at a concentration of 5 ug/ml for 4 hours at 37°C, after which cells were stained with the death cell marker Dapi. To quantify death of cells a standard amount of beads was added. Recombinant influenza specific rAT10-002 was used as a negative control.

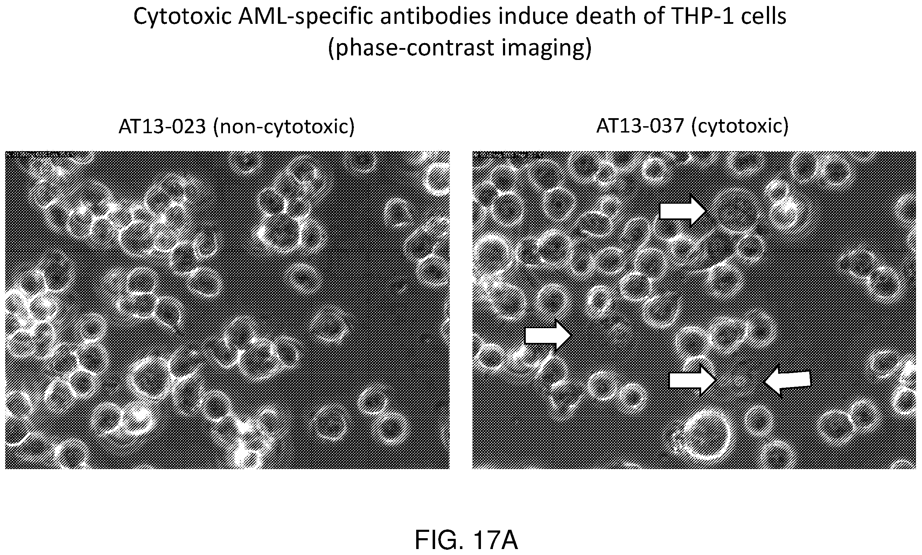

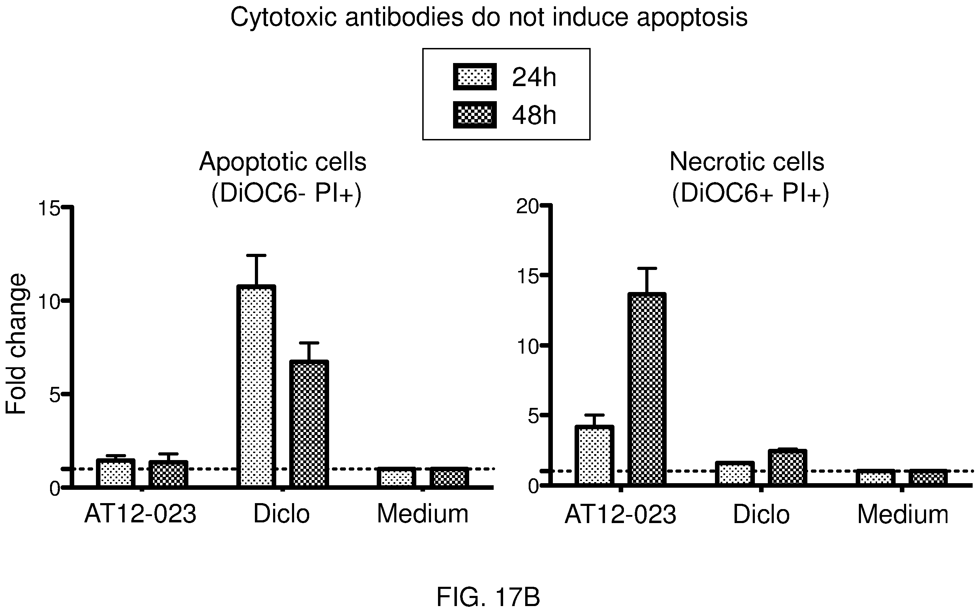

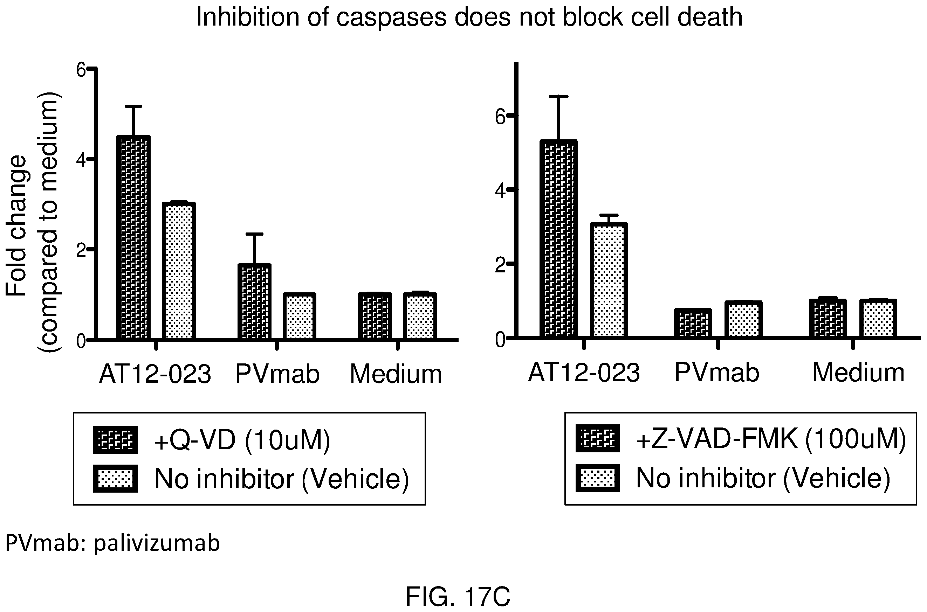

Figure 17. Cytotoxic antibodies induced a non-apoptotic death pathway. a) Phase contrast imaging of THP-1 cells. THP-1 cells were incubated with the non-cytotoxic AML-specific antibody AT13-023 (left panel) or the cytotoxic antibody AML-specific AT13-037 (right panel). Interaction was visualized using time-lapse imaging and demonstrated swelling of target cells after which cells died. Stills were taken after 4 hours of incubation, with blue arrows indicating large cells that are dead. b) Double staining with DiOC6 and PI showed that cytotoxic antibodies do not induce apoptosis. THP-1 cells were incubated with the cytotoxic AML-specific antibody AT12-023, with diclofenac (that induces apoptosis in THP-1 cells) or with medium only. Cells undergoing apoptosis first loose their mitochondrial membrane potential (loss of DiOC6 stain) after which they become permeable (PI positive), as can be seen after incubation of THP-1 cells with diclofenac. THP-1 cells incubated with AT12-023 showed increased membrane permeability (PI+) but maintained mitochondrial membrane potential (DioC6+), indicating the induction of a non-apoptotic cell death pathway. c) To confirm the non-apoptotic nature of the death pathway induced we tested the involvement of caspases in cell death induction. Cell death of THP-1 cells by AML-specific antibodies could not be prevented with the pan caspase inhibitors Q-VD-OPh (left panel) or Z-VAD-fmk (right panel).

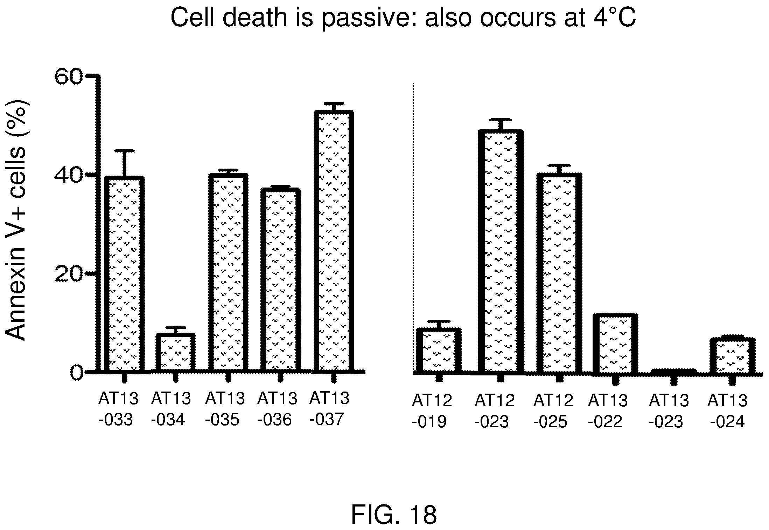

Figure 18. Cell death induced by AML-specific antibodies also occured at 4°C, suggesting the involvement of a passive process. This was true for all antibodies except AT13-031, which is cytotoxic at 37°C only (not shown). Negative controls included the AML-specific non-cytotoxic antibodies AT13-034, AT12-019, AT13-022, AT13-023 and AT13-024.

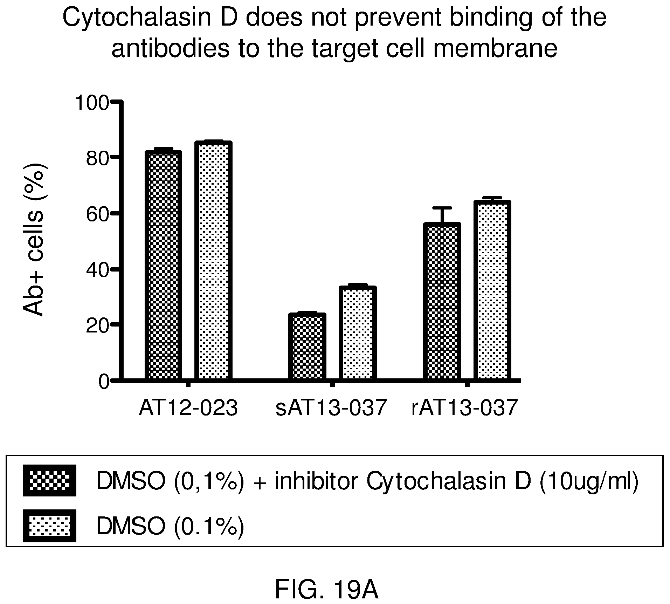

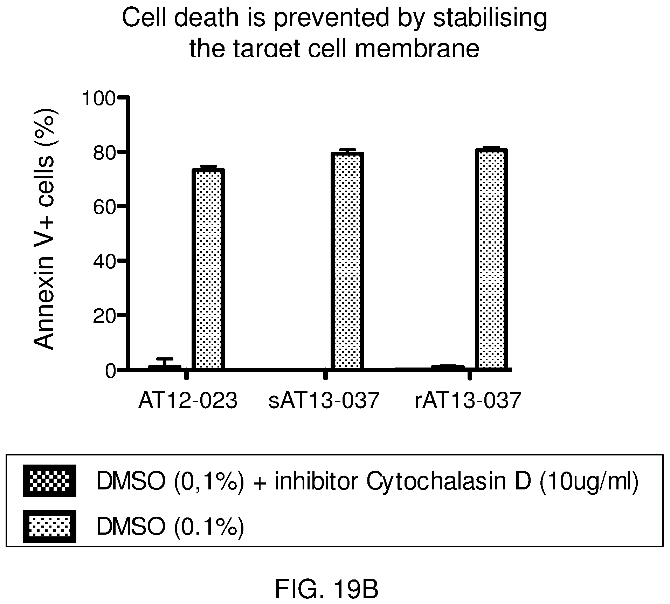

Figure 19. (a) Incubation of target cells with cytochalasin D did not inhibit binding of the antibodies. THP-1 cells were incubated with cytochalasin D, after which AML-specific antibodies AT12-023, AT13-031 and AT13-037 were added. (b) Preincubation of the target cells with the membrane stabilizing agent cytocholasin D did protect the THP-1 cells against cell death by the AML-specific cytotoxic antibodies.

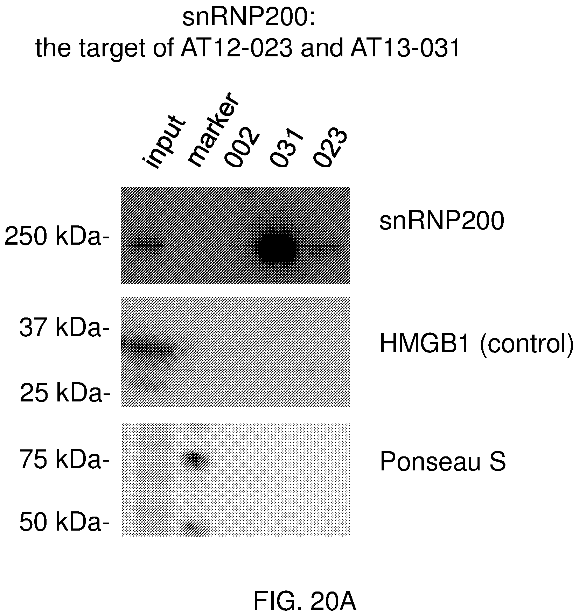

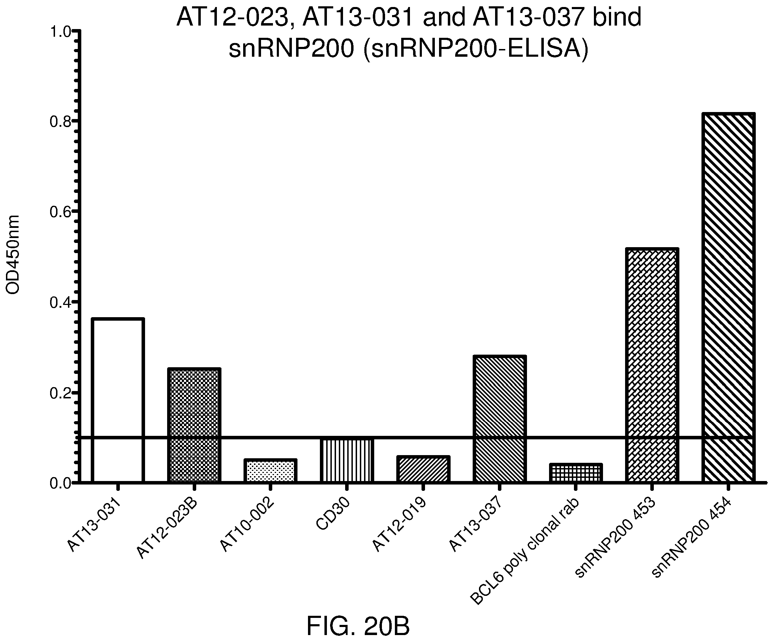

Figure 20. Target verification of AT12-023, AT13-031 and AT13-037. a) THP-1 membrane lysate was incubated with AT12-023 and AT13-031, with the influenza-specific antibody AT10-002 or with marker alone. Western blot analysis including mouse-anti-human snRNP200 (and HMGB1 as a negative control) revealed specific binding of AT12-023 and AT13-031 to snRNP200. b) AML specific antibodies AT12-019, AT12-023, AT13-031 and AT13-037 or commercially available snRNP200-specific antibodies snRNP200 453 and snRNP200 454 were coated on an ELISA plate, incubated with a snRNP200-flag construct for capturing and with anti-flag HRP for detection. AT12-023, AT13-031 and AT13-037 specifically bound snRNP200, whereas negative controls and for example AT12-019 did not.

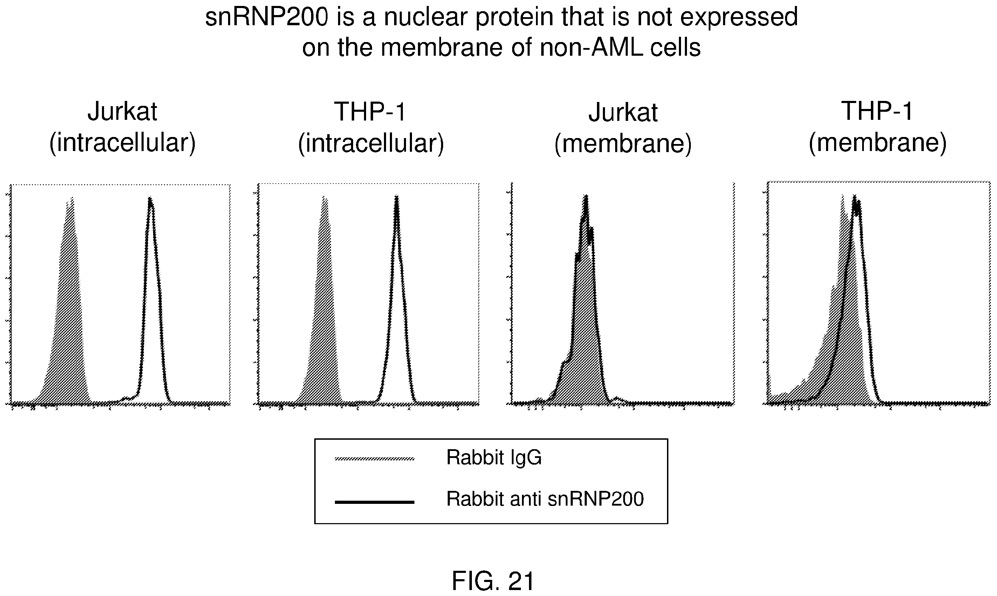

Figure 21. THP-1 cells express snRNP200 on their membrane. THP-1 cells and Jurkat cells were stained with a commercially available human-anti-mouse snRNP200 antibody. Intracellular staining of Jurkat cells and THP-1 cells showed nuclear staining, as expected (left panels). However, membrane staining of snRNP200 was restricted to THP-1 cells (right panels).

### Examples

### Example 1

### MATERIALS & METHODS

### Patient and healthy human materials

Study protocols were approved by the Medical Ethical Committee of the Academic Medical Centre. All participants signed informed consent. We selected two patients who had received an allogeneic stem cell transplantation (myeloablative sibling transplant for donor 59 and a matched unrelated donor- reduced intensity stem cell transplantation for donor 58) for AML and who, based on their clinical histories, can be assumed to have generated strong graft versus leukemia responses. These two SCT recipients donated 500 ml peripheral blood each, the products of one of many phlebotomies they underwent for post-transfusion hyperferritinemia. In addition, patients admitted to our clinic with newly diagnosed AML consented to donate 2-5 ml bone marrow or blood containing AML blasts to be used for antibody binding assays. Healthy bone marrow was donated by patients undergoing thoracotomy for cardiac surgery in our institute. B-non Hodgkin's lymphoma cells were obtained as rest material from biopsies of patients with newly diagnosed B-non Hodgkin's lymphoma. These B-non Hodgkin's lymphoma cells were used to test for binding of our antibodies as described below. Rest material of human umbilical vein endothelial cells (HUVECS), that were freshly isolated at the Department of Ophthalmology (AMC, Amsterdam, The Netherlands) were directly used for binding assays as described below. For purity, cells were co stained with and selected for anti human CD14+.

Mononuclear cells were isolated from peripheral blood and bone marrow of healthy individuals and AML patients by Ficoll separation. Extra markers like CD45, CD33, CD34, CD14, CD3 and CD19 were used to isolate specific cell populations (AML blasts, monocytes, T and B lymphocytes, respectively, obtained from blood bank donors).

### Isolation of B cells

We obtained B cells from peripheral blood by Ficoll separation and CD22 MACS microbeads (Miltenyi Biotech). Subsequently we sorted these cells for CD27- or CD27+ CD19+CD3-IgM-IgA- (naïve or memory IgG cells, respectively) and CD19+CD3-CD27+IgG-IgA- (memory IgM cells) on a FACSAria (Becton Dickinson).

### Cell culture

We maintained B cells (2×10⁵ cells/ml) in IMDM (Gibco) culture medium containing 8% FBS (HyClone) and penicillin/streptomycin (Roche), supplemented with recombinant mouse IL-21 (50 ng/ml, in house produced) and co-cultured them on γ-irradiated (50Gy) mouse L cell fibroblasts stably expressing CD40L (CD40L-L cells, 10⁵ cells/ml). The cultures were tested routinely for the presence of mycoplasma by PCR (data not shown).

### Retroviral transduction

Retroviral transduction was performed as described in Kwakkenbos ea, Nat Med 2010. Briefly, naive and memory IgG and memory IgM B cells were cultured and activated during 36 hours on CD40L-L cells in the presence of mIL-21. Then, BCL6 and Bcl-xL retroviral constructs that include the marker gene for GFP were used to transduce the B cells as described before (Diehl ea, J Immunol 2008), with the addition that we centrifuged cells and virus at room temperature for 60 min at 360 × *g* (1800 RPM). Transduction efficiency ranged from 69-90%.

### Culture of target cell lines

Target AML cell lines THP-1 (ATCC; density of 2×10⁵ cells/ml to 1×10⁶ cells/ml) and Mono-Mac6 (a kind gift from Dr. Hamann of the Experimental Immunology department, density of 2×10⁵ cells/ml to 2×10⁶ cells/ml) were maintained in RPMI 1640 (Gibco) culture medium, containing 10% FBS (HyClone) and penicillin/streptomycin (Roche). Culture medium of Mono-Mac6 was enriched with non-essential amino acids (Invitrogen), 1 mM sodium pyruvate (Invitrogen) and 10 µg/ml human insulin (Sigma). The AML cell line Molm13 was maintained at a density of 5×10⁵ cells/ml to 1,5×10⁶ cells/ml in RPMI 1640 (Gibco) culture medium, containing 20% FBS (HyClone) and penicillin/streptomycin (Roche). The liver cell lines HepG2 and Huh7 and the colon cell line HT29 (a kind gift from the Tytgat Institute, AMC, Amsterdam, The Netherlands) were cultured in DMEM (Gibco) culture medium, containing 8% FBS (HyClone) and penicillin/streptomycin (Roche). The acute T-cell leukemia cell line Jurkat (ATCC) was maintained in RPMI 1640 (Gibco) culture medium, containing 10% FBS (HyClone) and penicillin/streptomycin (Roche) at a density of 1×10⁵ cells/ml to 2×10⁶ cells/ml. Skin primary fibroblasts (a kind gift of the University of Leiden, The Netherlands) were cultured twice a week in DMEM (Gibco) culture medium, containing 10% FBS (HyClone) and penicillin/streptomycin (Roche). Cells were passaged no more then 10 times. The diffuse large cell B cell lymphoma (DLBCL) cell lines OCI-Ly1 and OCI-Ly7 (DSMZ and ATCC, respectively) were maintained in IMDM (Gibco) culture medium containing 8% FBS (HyClone) and penicillin/streptomycin (Roche). The cultures were maintained at 0,5-2×10⁶ cells/ml. Finally, the multiple myeloma (MM) cell lines U266 and NCI-H929 were maintained and cultured as described for THP-1 cells.

### Generation of AML-specific clones

Transduced naïve and memory IgG and IgM B cells of each patient were seeded at concentrations of 20 or 40 cells per well (hereafter named microcultures) and expanded with IL-21 and CD40L. Supernatants of expanded B cell micro cultures were then screened for antibody binding to leukemia cell lines (THP-1, MonoMac6 and the cell lines depicted in Table 4), to liver- and colon cell lines, and some supernatants also to the primary blasts isolated from patients with AML- M0, M1, M4 and M5, by FACS, using human-IgG-PE (Southern Biotech) or human IgG H+L AF647 (Life Technologies) as a secondary antibody. Several in-house generated antibodies were used as negative control antibodies, such as anti-CD30 (expressed on activated B and T lymphocytes), anti-CD33 (expressed on monocytes, myeloid progenitor cells and myeloid leukaemias), D25 (against RSV; described in WO 2008/147196), AT10-002, AT10-004 (against influenza; described in WO 2013/081463), and F1 (against MRSA; described in WO 2011/008092). In addition, some commercially available antibodies were used, such as Rituximab (anti-CD20), Palivizumab (anti-RSV), Panitumumab (anti-EGFR) and HLA-DR. Microcultures binding to AML cell lines but not to liver- and colon cell lines were selected and seeded at a concentration of 1 cell/well and their supernatants tested again for specificity for AML cell lines. Clones with supernatants specifically binding AML cell lines and not liver- or colon cell lines, or healthy PBMC and bone marrow were selected for sequencing. Clones were expanded under normal culture conditions in the presence of FBS IgG low serum (Hyclone) and antibodies purified from the supernatants of these cultures as described below for the recombinant antibodies.

### Cloning of AML-specific antibodies

To produce recombinant antibodies we isolated total RNA with the RNeasy^{®} mini kit (Qiagen), generated cDNA, performed PCR and cloned the heavy and light chain variable regions into the pCR2.1 TA cloning vector (Invitrogen). To rule out reverse transcriptase or DNA polymerase induced mutations, we performed several independent cloning experiments. To produce recombinant mAb we cloned heavy and light variable regions of each antibody in frame with human IgG1 or IgG3 and Kappa constant regions into a pcDNA3.1 (Invitrogen) based vector and transiently transfected 293T cells. We purified recombinant antibodies from the culture supernatant with Protein A or G, depending on the Ig subtype of the clone.

### In vitro activity of AML-specific antibodies

To measure the *in vitro* effect of AML-specific antibodies on AML cells we used several approaches. First, THP-1 cells (2x10⁴) were seeded in flat bottom 96 well plates (Costar). AML or control -antibodies were added on day 1, in a concentration of 5-10 µg/ml. Numbers of cells per well were counted daily. Induction of cell death on both THP-1 cells and primary leukemic cells by AML-specific antibodies was measured in parallel by costaining an aliquot of cells for Annexin V (BD Pharmingen) and 7- Aminoactinomycin D (7-AAD; Beckman Coulter), double-negative cells being the viable cells. In addition, we used a specific lysis assay. Target cells (THP-1) were labeled with Calcein AM (Becton Dickinson), a green fluorescent dye that is released from the cytoplasm when cells die. Briefly, 2 million THP-1 cells were incubated with 2 ml of 2 µM Calcein AM for 30 minutes at 37°C. AML or control antibodies were added for 4 hours, after which green fluorescence was measured using a fluoresence plate reader. The proportion of specific lysis was calculated as (experimental value-low control) / (high control-low control) x 100, where low control means the spontaneous release of Calcein AM by unaffected cells and high control means the maximum amount of released Calcein AM when all cells are lysed. In addition to the Calcein AM release assay we applied the lactate dehydrogenase (LDH) release assay to measure the killing activity of AML specific antibodies. LDH is released by damaged cells, from the cytosol. Leukemic blasts, isolated at diagnosis from a patient with AML-M0, were incubated with AT13-024 at a maximum concentration of 10 µg/ml per 10.000 blasts. LDH release is measured after adding Reaction mixture and Stop Solution (Roche Diagnostics/Applied Science) according to the manufacturer's protocol on an ELISA reader. The percent cytotoxicity is calculated as the (experimental value-low control) / (high control-low control) x 100, where low control means the spontaneous release of LDH by unaffected cells and high control means the maximum amount of LDH when all cells are lysed. To quantify death of target cells induced by AML-specific antibodies we used a FACS-based leukemia cell lysis assay as previously described (Schmiedel et al, 2013). In brief, FACS calibration beads (Accudrop Fluorescent Beads, BD Biosciences) were added to the cells in a 50/50 ratio after which a standard amount of beads was acquired with FACS. As an equal assay volume was ascertained by the calibration beads, the amount of dead or vanished cells can then be calculated as followes: 100 - ((Dapi negative cells in respective treatment/Dapi negative cells in control) × 100).

Finally, to discern between apoptotic and non-apoptotic pathways of cell death induced by our antibodies we co-stained target cells with DiOC6 and propidium iodide (PI). DiOC6 (3,3-dihexyloxacabocyanine iodide) is a cell-permeant, green-fluorescent, lipophilic dye that is selective for the mitochondria of live cells, when used at low concentrations. Apoptotic cells will lose their mitochondrial membrane potential (loss of DiOC6 stain) before the cell membrane becomes permeable (PI positive); necrotic cells become PI positive before they loose their mitochondrial membrane potential (and become DiOC6-negative). The methods used for staining were adapted from Hugh J. M et al., 2004. In brief, THP-1 cells were stained with 40nM of DiOC6 (Invitrogen) for 20 minutes at 37°C. PI was added after which the samples were analyzed immediately by flow cytometry.

### Flow cytometry

Stained cells were analyzed on FACSAria (BD), FACSCanto (BD), FACS LSRFortessa X-20 (BD) and Guava (Millipore) flowcytometers and flow cytometry data were processed with FlowJo software (Tree Star).

### Phase contrast imaging

THP-1 cells were added to a two chambered Cover glass system (LabTek). Using a phase contrast-imaging microscope (in-house manufactured), four fields of interest per chamber were chosen. The software was set to take a picture of every field of interest every other minute. Just prior to the run, the THP-1 binding, non-cytotoxic antibody AT13-023 and the THP-1 binding, cytotoxic antibody AT13-037 were added to their respective wells at a concentration of 10 ug/ml.

### Target verification: Immunoprecipitation and flow cytometry

THP-1 cells were lysed and precleared with an irrelevant antibody (in-house generated RSV antibody D25) and beads to remove non specific binding proteins. Precleared lysate was then incubated with bead-labeled AML-specific antibodies or with the influenza specific antibody AT10-002 as a negative control (3 hrs at 4°C). Antibody-incubated lysates were washed five times, bound proteins were eluted from the THP-1 lysate and then run on an SDS-PAGE gel. Proteins were blotted and stained with Ponseau S to reveal total protein. The blot was blocked with BSA and incubated with mouse-anti-snRNP200 (Millipore, clone 3B6.1) or Rabbit-anti-HMGB1 (Abcam) for Western blot analysis. Intracelllar staining for snRNP200 was performed after fixing and permeabilizing THP-1 cells with methanol (Sigma) and permeabilization buffer containing triton X-100 (Sigma), EDTA (Gibco) and BSA (Roche), followed by incubation with rabbit-anti-human snRNP200 antibody (Sigma) overnight at 4°C.

### Target conformation: snRNP200 ELISA

HEK 293T cells were transfected with an expression vector containing the full-length open reading frame of snRNP200 with an N-terminal FLAG tag. At 2 days after transfection cells were harvested and lysed in lysis buffer containing protease inhibitors. This lysate was cleared and protein concentration was measured. AML specific antibodies AT12-019, AT12-023, AT13-031 and AT13-037 or commercially available snRNP200 specific antibodies (Bethyl labs) were then coated on an ELISA plate. The lysate was added at a concentration of 3 µg/ml for capturing. After extensive washing, captured snRNP200-flag was detected with a monoclonal mouse anti-flag HRP antibody (Sigma-Aldrich, clone M2).

### RESULTS

### Generation of AML-specific antibodies

In our project we use a unique technology that was recently developed in our laboratory (WO 2007/067046) that allows for selection of naturally occurring leukemia-specific B cells and the antibodies produced by these cells. We employed this technology to generate leukemia-specific B cell lines from two AML patients who developed potent GvL responses in parallel with severe GvHD of the liver and skin. With our B cell immortalization technique, it became possible to identify B cell responses of leukemia patients and to create leukemia-specific B cell lines that allow functional studies of the antibodies these cell lines produce and allow generation of larger numbers of antibodies, as compared to other methods known in the art, with diagnostic and therapeutic potential.

First, we applied our technique as described in WO 2007/067046 to perform a set of pilot experiments, where we isolated and immortalized B lymphocytes from a carefully selected patient (donor 59) who developed a potent GvL response following extensive GvHD of liver and skin. This patient was diagnosed with AML at the age of 36 years. She obtained complete remission after the first of two courses induction chemotherapy after which she received a myeloablative, HLA-matched sibling donor SCT as consolidation therapy. Only 5 months after SCT, shortly after immunosuppressive therapy had been tapered, her AML relapsed. With one cycle of high-dose cytarabine she obtained complete remission again, after which she spontaneously, before the scheduled donor-lymphocyte infusion, developed stage III GvHD of the liver and stage II GvHD of the skin. She was successfully treated with high-dose corticosteroid therapy but during tapering of therapy hepatic GvHD relapsed twice. In the end, corticosteroid therapy was successfully phased out about 6 months before the current experiments were started (1,5 yrs after AML relapse) and she has remained in complete remission from her leukemia for more than three years now (Figure 2).

From this patient, we generated B cell clones using our technique to immortalize B cells. Briefly, PBMC were isolated from the peripheral blood by ficoll centrifugation, and B lymphocytes sorted by FACS. Isolated B lymphocytes were then cultured with IL21 and CD40L and transduced with BCL6 and BCL-xL, with a transduction efficiency of around 70%. Using this technique, unique B cell clones that concurrently express the B cell receptor and secrete monoclonal antibodies were generated. Immortalized CD27- naive and CD27+ memory IgG+ B cells were seeded at concentrations of 20 or 40 cells per well and expanded with IL21 and CD40L. Supernatants of expanded B cell micro cultures were then screened for antibody binding to leukemia-, liver- and colon cell lines by FACS, using human-IgG-PE as a secondairy detection antibody.

As the patient had been diagnosed with AML-M5 according to the French-American-British (FAB) classification (Bennett et al., 1976), we selected the THP-1 cell line that morphologically and phenotypically is roughly similar to AML-M5 for these screening essays (Tsuchiya et al., 1988). In addition, because the patient suffered from extensive GvHD of the liver we screened for the identification of liver-binding B cell clones using the liver cell line HepG2. As a negative control, and to select out cross-binding B cell clones we used the colon cell lines HT-29 and LSTR. Micro cultures with the highest binding to either of these cell lines were then sorted and single cells were deposited in 96 well formats, expanded and the supernatants of these expanded clones were again screened for binding against leukemia cell lines (Figure 3).

### Properties of AML-specific antibodies

With this approach we generated eight B cell lines from this one patient, producing antibodies that bind to THP-1 (Figure 3) but not liver or colon cell lines, fibroblasts, endothelial cells (human umbilical vein endothelial cells or HUVEC), healthy PBMC or bone marrow (Table 2 and Figure 4). These are antibodies AT12-019, AT12-023, AT12-025, AT13-022, AT13-023, AT13-024, AT13-031 and AT12-020.

The heavy and light chain variable regions of these antibodies are depicted in Table 1 and Figure 1.

Interestingly, the antibodies are of the IgG1 and IgG3 isotypes, and several of the DNA sequences showed somatic hypermutations. Antibodies AT12-023, AT12-025, AT13-023 and AT13-031 belong to the VH4-34 family, which is a family of VH sequences known for their potential killing properties (Bhat et al, 1997). The antibodies are of donor-origin, as confirmed by microchimerism analyses.

We then determined the breadth of binding within the spectrum of AML subtypes, and tested the generated AML-specific B cell clones for binding to other AML FAB classification types. Selected leukemia-specific clones also bound to the AML-M5 cell line MonoMac6 (Table 3) (see for review of AML cell lines (Drexler and Minowada, 1998)).

Our AML-specific antibodies also bound to freshly isolated AML blasts of newly diagnosed patients (Table 3 and Figure 5).

Furthermore, some antibodies also bound other hematopoietic malignancy -cell lines, such as the diffuse large cell B cell lymphoma cell lines OCI-Ly1 and OCI-Ly7 and the multiple myeloma cell lines U266 and NCI-H929, and/or patient-derived hematologic tumor cells (Table 4 and Figure 6).

### In vitro activity of AML specific antibodies

While testing the specificity of the AML-binding antibodies, we made a striking observation. Three of the eight antibodies spontaneously induced death of the leukemic cells they were binding to. THP-1 cells were killed by AT12-023 and AT12-025, and primary AML blasts isolated from patients diagnosed with AML were killed by AT12-025 and AT12-024 (Table 5 and Figures 7-10).

Figure 7 shows rapid killing of THP-1 cells by AT12-023. Dying blasts expressed 7-AAD and Annexin V. Strikingly, while co-culture of THP-1 with AT12-025 at first did not affect total cell numbers in the cultures (Figure 7), this antibody appeared to also induce death of THP-1 cells, albeit with a little delay compared to AT12-023 (Figure 8). The observation that some of the antibodies show direct killing activity against leukemic cells is highly exciting and has to the best of our knowledge not been reported before.

### Confirmation of our findings by generating AML-specific B cell lines from a second patient

About 0.05 to 0.1% of the B cells that we screened from the first patient were found to be AML-specific. To confirm our findings, we selected a second patient with relapsed AML who obtained durable remission after an allogeneic SCT (donor 58; Figure 11). Also this patient developed GvHD of the skin and liver for which she was treated with oral steroids. After steroids were phased out, about one year after the SCT, PBMC were isolated, B cells immortalized, and B cell lines screened for binding to AML cell lines as described above. Five B cell clones have been generated from this patient that bind to the AML cell lines THP-1 and MonoMac6 (Figure 12), but not to liver or gut cell lines, fibroblasts or healthy PBMC (Table 6). These clones produce antibodies AT13-033, AT13-034, AT13-035, AT13-036 and AT13-037. Interestingly, all these antibodies are of the IgG3 isotype. The sequences of the heavy and light chain variable regions of these antibodies are also depicted in Table 1 and Figure 1. These data confirm the achievability of our project; despite the low frequencies of the B cells we are aiming at, we were able to isolate them and to generate robust antibody-producing B cell lines that were specific for AML.

### Discussion

These preliminary date are highly exciting, as - to the best of our knowledge - we are the first to generate several human, non-chimeric, leukemia-specific B lymphocyte clones from patients that developed a robust, lasting GvL response against AML. The observation that some of these antibodies spontaneously show killing activity against leukemic blasts *in vitro* is highly exciting and very promising. In addition, the observation that some of the antibodies have a germline sequence and are of the IgG3 subclass, a type of antibody that can be induced without T cell help, warrants further attention as these findings demonstrate that 'natural' antibodies generated in a T-cell-independent way affect GvL responses in SCT patients. Importantly, these data offer proof of concept that using this technique, we can select leukemia-specific B cell clones.

### Example 2

### AML-specific antibodies from a third patient

To further confirm our approach and to show this anti-AML response is not only reserved for female patients, we selected a male patient as well. This patient (Figure 13), donor 101, was diagnosed with an intermediate-risk AML (no cytogenetic or molecular abnormalities; FAB classification AML-M5) at the age of 49 years. He received two courses of chemotherapy (cytarabine, idarubicine, amsacrine) and one course of consolidation chemotherapy (busulphan, cyclophosphamide) followed by an autologous HSCT, as there was no HLA-matched sibling stem cell donor available. Fourteen months after the first diagnosis his disease relapsed. He obtained complete remission after one cycle of high-dose cytarabine, after which he received a reduced intensity allogeneic HSCT of a matched, unrelated donor (RIST-MUD). Six weeks later he developed acute GvHD of skin, liver and intestine (stage 1; grade II) that responded well to corticosteroid therapy. B cells were isolated from a phlebotomy product obtained from this patient 38 months post-SCT.

Using the same methods as described in Example 1, four B cell clones have been generated from this patient that bind to the AML cell lines THP-1 and/or Molm13 (Figure 14 and Table 7) but not to (fetal) liver or gut cell lines, fibroblasts or healthy PBMC (Table 8). These clones produce antibodies AT14-013 (IgG1), AT14-014 (IgG3), AT14-015 (IgG3) and AT14-016 (IgG3). Interestingly, again the majority of the antibodies is of the IgG3 isotype. The sequences of the heavy and light chain variable regions of these antibodies show high amounts of somatic hypermutations and are depicted in Table 1B and Figure 1. Clone AT14-013 shows very minor reactivity with the hepatocellular carcinoma cell line Huh7, but not to the hepatocellular carcinoma cell line HepG2 or healthy fetal liver cells. Also minor reactivity to fibroblasts is observed, however, the binding of this antibody to these tissue specific cells, compaired to the binding to primary leukemic blasts is negligible (Figure 14).

It was also tested whether the four antibodies of donor 101 could bind primary AML blasts (Materials & Methods: see Example 1). Figure 15 and Table 7 show that antibody AT14-013 appears to bind primary blasts of at least three different FAB classifications (AML-M0, M4 and M5). Antibodies AT14-015 and AT14-016 are able to bind primary AML blasts of the M4 classification.

### Example 3

### In vitro activity of AML-specific antibodies of donors 59, 58 and 101

In Example 1, the breadth of binding of the antibodies of donor 59 within the spectrum of AML subtypes was determined (Table 3). Using the same methods, this has also been determined for the antibodies of donor 58. The results are shown in Table 9A: all five antibodies of donor 58 (AT13-033, AT13-034, AT13-035, AT13-036 and AT13-037) bound to the AML-M5 cell line THP-1, as well as to the AML-M5 cell line MonoMac6. These AML-specific antibodies did not significantly bind to freshly isolated AML blasts of newly diagnosed patients of FAB classification M0, M1 or M4. AT13-034 could only weakly bind M0 blasts. This indicates that these antibodies have a specificity for (at least) AML M5 blasts.

In Example 1, it was also determined that antibodies AT12-023 and AT12-025 of donor 59 killed THP-1 cells (Table 5 and Figures 7-9). Subsequently, we have tested whether the other antibodies of donor 59, as well as the antibodies of donor 58, could also kill THP-1 cells, using the same Materials & Methods as in Example 1. As shown in Tables 9B-10, in addition to AT12-023 and AT12-025 also antibody AT13-031 (donor 59), as well as antibodies AT13-033, AT13-035, AT13-036 and AT13-037 (donor 58) appear to be able to kill THP-1 cells.

The capability of inducing death of primary AML blasts was also tested (Materials & Methods: see Example 1). Similar to some of the antibodies obtained from donor 59, some of the antibodies derived from donor 58 are also capable of inducing cell death of primary AML cells. This is shown in Figure 16 for antibody AT13-037, in both forms (purified B cell supernatant (sAT13-037) and recombinant produced antibody (rAT13-037); both in the IgG3 confirmation). The leukemic cells are derived from the bone marrow of a newly diagnosed AML patient, FAB classification AML-M5.

### Example 4 (for reference only)

### In vitro activity of AML antibodies is rapid and involves a non-apoptotic cell death pathway

To investigate the pathway through which several of the above-mentioned antibodies induced death of their target cells, we visualized death of target cells with time-lapse microscopy (Figure 17a). We observed that within a few minutes after incubation with antibody AT13-037, cells started to swell and then died. This suggested that antibodies according to the invention activated pathways other than the classical apoptosis pathways, as apoptotic cells shrink rather than swell. To further investigate this we performed double staining with the cell death markers DiOC6 and propidiumiodide (PI). Cells engaged to undergo apoptosis first loose mitochondrial charge (which can be visualized by the loss of DiOC6 binding) before membrane permeability increases (visualized by propidium iodide (PI) staining), while cells undergoing necrosis loose their membrane integrity (PI positive) but not immediately the mitochondrial membrane potential. As diclofenac is known to induce apoptosis of THP-1 cells, we used diclofenac as a positive control. Diclofenac-treated, apoptotic THP-1 cells became DiOC6-negative (lost mitochondrial membrane potential), but maintained membrane integrity. Upon incubation with the AML-specific cytotoxic antibody AT12-023 mitochondrial membrane potential was maintained (DiOC6-positive) while the membrane permeability of THP-1 cells increased (PI-positive). Thus, AML-antibody treated THP-1 cells became PI-positive while maintaining mitochondrial membrane potential (Figure 17b, right panel) indicating the activation of a non-apoptotic death pathway. Indeed, death of THP-1 cells by cytotoxic AML-specific antibody treatment could not be prevented by incubating the cells with the pan caspase inhibitors Z-VAD-fmk or QVD-OPh (Figure 17c).

### Cell death mediated through interference with the target cell membrane

Non-apoptotic death pathways include necroptosis and oncosis. Necroptosis is, similar to apoptosis, an active cell death pathway that depends on the activation of defined molecular pathways including the activation of receptor-interacting protein kinase 3 (RIPK3). To investigate whether AML-antibody-induced cell death was dependent on the activation of intracellular molecular pathways we tested whether antibody-induced cytotoxicity was dependent of temperature. We found that under these experimental conditions the cytotoxic activities of at least antibodies AT13-033, AT13-035, AT13-036, AT13-037, AT12-023 and AT12-025 were equally potent at 4°C as compared to their activities at 37°C (Figure 18), suggesting that at least these antibodies induced cell death by a passive process. Of note, the cytotoxic activity of antibody AT13-031 was significantly more potent at 37°C as compared to 4°C. However, since antibody AT13-031 does induce death of AML cells in the presence of apoptosis inhibitors (such as the pan caspase inhibitors Q-VD-Oph or Z-VAD-fmk), it is clear that antibody AT13-031 is also able to diminish proliferation of AML cells independently from apoptosis.

Oncosis is a mode of cell death characterized by swelling of the cell, through selective membrane injury, resulting in increased membrane permeability and ultimately cell death. Oncosis-inducing antibodies have been described that mediate cell death by forming large pores in the membrane through destabilization of the membrane (Hernandez et al, 2011). Cytocholasin D is an actin polymerization inhibitor that can stabilize the cytoskeleton. Treatment of the target cell line THP-1 with cytochalasin D did not prevent antibody binding to these cells (Figure 19a), however, it did prevent death of the target cells (Figure 19b ). Thus, membrane stabilisation protects the target cells against the cytotoxic activity of our antibodies.

### Example 5 (for reference only)

### One of the targets recognized by our antibodies is snRNP200

We then set out to determine the target recognized by several AML-specific antibodies. For this we used immunoprecipitation of cell membrane lysates of THP-1 cells with AT12-023 and AT13-031. We found a clear protein band at 250 kD that was sent for mass-spectometry analysis which revealed snRNP200 as the target antigen which could be confirmed by Western blot analysis (Figure 20a). We developed an ELISA, to verify snRNP200 as the target of some of the AML antibodies. With this ELISA we found that in addition to AT12-023 and AT13-031, AT13-037 specifically recognizes snRNP200 (Figure 20b). Other AML specific antibodies like AT12-019 did not recognize snRNP200 as target antigen. snRNP200 is part of the spliceosome in all eukaryotic cells and is therefore expected to be located in the nucleus. To confirm that AML cells express this protein on their cell surface we stained the cell membrane of THP-1 cells with a commercially available, anti-snRNP200 antibody. Figure 21 shows that the anti snRNP200 antibody is indeed binding to the membrane of the AML cells, but not to the Jurkat cell line. As expected, as snRNP200 is a nuclear protein, it did bind intracellularly to the Jurkat and THP-1 cell lines (Figure 21).

All eukaryotic cells have snRNP200 (also known as U5-snRNP) protein in the nucleus, as part of the spliceosome (Kattah, 2010). The spliceosome consists of a number of proteins. At least one of these proteins, U1-snRNP, has been described to be expressed on apoptotic cells in patients with systemic lupus erythematosus (SLE) and mixed connective tissue disease (MCTD) leading to auto-immune responses (Kattah, 2010).

We propose that snRNP200 expression by AML cells has triggered an allo-immune response (graft vs leukemia response) that has kept the patients in durable remission. Therefore, this protein can now be used as a new AML target. Moreover, since antibodies AT12-023 and AT13-031 specifically recognize snRNP200 and also recognize B-NHL cells, snRNP200 can now also be used as a target for B-NHL.

**Table 1A. Preferred anti-AML antibodies according to the invention (CDR numbering according to Kabat et al. 1991)**

| **SEQ ID NO.** | **Antibody** | **Identity** | **Sequence** |
|---|---|---|---|
| 1 | AT12-023 | Heavy chain CDR1 | GYYWS |
| 2 | AT12-025 | Heavy chain CDR1 | GYYWS |
| 3 | AT13-024 | Heavy chain CDR1 | SYGMH |
| 4 | AT12-019 | Heavy chain CDR1 | SYAMS |
| 5 | AT13-022 | Heavy chain CDR1 | SYGMH |
| 6 | AT13-023 | Heavy chain CDR1 | GYFWT |
| 7 | AT13-031 | Heavy chain CDR1 | GYYWS |
| 8 | AT12-020 | Heavy chain CDR1 | TYSMN |
| 9 | AT13-033 | Heavy chain CDR1 | NYGMH |
| 10 | AT13-034 | Heavy chain CDR1 | SHAIH |
| 11 | AT13-035 | Heavy chain CDR1 | SYGMH |
| 12 | AT13-036 | Heavy chain CDR1 | SYSMN |
| 13 | AT13-037 | Heavy chain CDR1 | TYGMH |
| 14 | AT12-023 | Heavy chain CDR2 | EINHSGSTNYNPSLKS |
| 15 | AT12-025 | Heavy chain CDR2 | EINHSGSTNYNPSLKS |
| 16 | AT13-024 | Heavy chain CDR2 | FIRYDGSNKYFADSVRG |
| 17 | AT12-019 | Heavy chain CDR2 | TIRASGGSTSYADSVKG |
| 18 | AT13-022 | Heavy chain CDR2 | ISYDGSNKYYADSVKG |
| 19 | AT13-023 | Heavy chain CDR2 | ETVHSGGTNYNPSLKS |
| 20 | AT13-031 | Heavy chain CDR2 | EINHSGSTNYNPSLKS |
| 21 | AT12-020 | Heavy chain CDR2 | SISSSSGYIYYADSVKG |
| 22 | AT13-033 | Heavy chain CDR2 | VISHDGSKTYYGHSVKG |
| 23 | AT13-034 | Heavy chain CDR2 | LIWYDGSNNYYADSVKG |
| 24 | AT13-035 | Heavy chain CDR2 | VISYDGSNKYYADSVKG |
| 25 | AT13-036 | Heavy chain CDR2 | SISSSSTYIYYADSVKG |
| 26 | AT13-037 | Heavy chain CDR2 | VIWYDGSNTYYADSVKG |
| 27 | AT12-023 | Heavy chain CDR3 | GRSTSPLDYYYYYMDV |
| 28 | AT12-025 | Heavy chain CDR3 | GSMARPKPFDY |
| 29 | AT13-024 | Heavy chain CDR3 | DPQERIYYSDTSGYLDY |
| 30 | AT12-019 | Heavy chain CDR3 | SPAMIRGVRGGDYFDY |
| 31 | AT13-022 | Heavy chain CDR3 | DGKGIVVIYYYYGMDV |
| 32 | AT13-023 | Heavy chain CDR3 | GLNSPFDY |
| 33 | AT13-031 | Heavy chain CDR3 | GPRGMYSSSSGDY |
| 34 | AT12-020 | Heavy chain CDR3 | DGTFSYYYYMDV |
| 35 | AT13-033 | Heavy chain CDR3 | AGLNYYGNLLSNYFYYGMDV |
| 36 | AT13-034 | Heavy chain CDR3 | ARDGCTGGSCCYFDN |
| 37 | AT13-035 | Heavy chain CDR3 | AKDSYYYGSGRRWGYYFDY |
| 38 | AT13-036 | Heavy chain CDR3 | ARRREVGRDGYSLYPRGYHYGMDV |
| 39 | AT13-037 | Heavy chain CDR3 | ARGRGYSAQGNRNRAYYFDY |
| 40 | AT12-023 | Light chain CDR1 | QGDFLRSYYAS |
| 41 | AT12-025 | Light chain CDR1 | RASQSISRYLN |
| 42 | AT13-024 | Light chain CDR1 | RASQSISSWLA |
| 43 | AT12-019 | Light chain CDR1 | RASQAFSSYLV |
| 44 | AT13-022 | Light chain CDR1 | SGDKLGDKYAC |
| 45 | AT13-023 | Light chain CDR1 | RASQGIRNVLG |
| 46 | AT13-031 | Light chain CDR1 | RASQGIRNDLG |
| 47 | AT12-020 | Light chain CDR1 | RASQDISSSLA |
| 48 | AT13-033 | Light chain CDR1 | TGTSSDIGGYNYVS |
| 49 | AT13-034 | Light chain CDR1 | RASQSISNNLG |
| 50 | AT13-035 | Light chain CDR1 | QGDSLRSYYAS |
| 51 | AT13-036 | Light chain CDR1 | TGTSSDVGGYNYVS |
| 52 | AT13-037 | Light chain CDR1 | RASQSVSSNLA |
| 53 | AT12-023 | Light chain CDR2 | GKNKRPS |
| 54 | AT12-025 | Light chain CDR2 | AASSLQS |
| 55 | AT13-024 | Light chain CDR2 | KASSLES |
| 56 | AT12-019 | Light chain CDR2 | ATSTLQG |
| 57 | AT13-022 | Light chain CDR2 | QDSKRPS |
| 58 | AT13-023 | Light chain CDR2 | AASSLQS |
| 59 | AT13-031 | Light chain CDR2 | AAVSLQS |
| 60 | AT12-020 | Light chain CDR2 | AASTLQS |
| 61 | AT13-033 | Light chain CDR2 | EVTKRPS |
| 62 | AT13-034 | Light chain CDR2 | GASTRAT |
| 63 | AT13-035 | Light chain CDR2 | GKNNRPS |
| 64 | AT13-036 | Light chain CDR2 | DVNDRPS |
| 65 | AT13-037 | Light chain CDR2 | GAFTRVT |
| 66 | AT12-023 | Light chain CDR3 | NSRDRSGNHLV |
| 67 | AT12-025 | Light chain CDR3 | QQSYSTPRT |
| 68 | AT13-024 | Light chain CDR3 | QQYNTYPYT |
| 69 | AT12-019 | Light chain CDR3 | QQYYSYPPT |
| 70 | AT13-022 | Light chain CDR3 | QAWDSSTVVF |
| 71 | AT13-023 | Light chain CDR3 | LQHNSHPRT |
| 72 | AT13-031 | Light chain CDR3 | LQHNSYPRT |
| 73 | AT12-020 | Light chain CDR3 | QQYYSYPPT |
| 74 | AT13-033 | Light chain CDR3 | SSYAGSNDLL |
| 75 | AT13-034 | Light chain CDR3 | QQYNNWPRLT |
| 76 | AT13-035 | Light chain CDR3 | NSRDSSGNHVV |
| 77 | AT13-036 | Light chain CDR3 | SSYTRSNTVI |
| 78 | AT13-037 | Light chain CDR3 | QQYNDRPPYT |
| 79 | AT12-023 | Heavy chain | QVQLQQWGAGLLKPSETLSLTCAVYGGSFSGYYWSWIRQPPGKGLEWIGEINHSGSTNYNPSLKSRVTISV DTSKNQFSLKLSSVTAADTAVYYCARGRSTSPLDYYYYYMDVWAKGTTVTVSS |
| 80 | AT12-025 | Heavy chain | QVQLQQWGAGLLKPSETLSLTCAVYGGSFSGYYWSWIRQPPGKGLEWIGEINHSGSTNYNPSLKSRVTISV DTSKNQFSLKLSSVTAADTAVYYCARGSMARPKPFDYWGQGTLVTVSS |
| 81 | AT13-024 | Heavy chain | QVQLVESGGGVVQPGRSLRLSCAASGFTFSSYGMHWVRQAPGKGLEWVAFIRYDGSNKYFADSVRGRFTIS RDNSKNTLFLQMNSLRAEDTAVYYCAKDPQERIYYSDTSGYLDYWGQGTLVTVSS |
| 82 | AT12-019 | Heavy chain | EVHLLESGGGLVQPGGSLRLSCAASGFTFSSYAMSWVRQAPGKGLEWVSTIRASGGSTSYADSVKGRFTISR DNSQSRLYLQMNSLTAEDTAVYYCAKSPAMIRGVRGGDYFDYWGQGTLVTVSS |
| 83 | AT13-022 | Heavy chain | QVQLVESGGGVVQPGRSLRLSCAASGFTFSSYGMHWVRQAPGKGLEWVAVISYDGSNKYYADSVKGRFTIS RDNSKNTLYLQMNSLRAEDTAVYYCAKDGKGIVVIYYYYGMDVWGQGTTVTVSS |
| 84 | AT13-023 | Heavy chain | QVQLQQWGAGLLKPSETLSLTCAVYGGSFSGYFWTWIRQPPGKGLEWIGETVHSGGTNYNPSLKSRVTISV DTSKNQFSLRLNSVTAADTAVYYCVRGLNSPFDYWGQGTLVTVSS |
| 85 | AT13-031 | Heavy chain | QVQLQQWGAGLLKPSETLSLTCAVYGGSFSGYYWSWIRQPPGKGLEWIGEINHSGSTNYNPSLKSRVTISV DTSKKQFSLKLSSVTAADTAVYYCARGPRGMYSSSSGDYWGQGTLVTVSS |
| 86 | AT12-020 | Heavy chain | EVQLVESGGGLVKPGGSLRLSCAASGFTFSTYSMNWVRQAPGKGLEWVSSISSSSGYIYYADSVKGRFTISR DNAKNSLYLQMNSLRAEDTAVYYCARDGTFSYYYYMDVWGKGTTVTVSS |
| 87 | AT13-033 | Heavy chain | QVQLVESGGGVVQPGRSLRLSCAVSGLSFRNYGMHWVRQAPGKGLEWVAVISHDGSKTYYGHSVKGRFTI SRDKSKTMLFLQMNSLRPEDTAVYYCAKAGLNYYGNLLSNYFYYGMDVWGQGTTVTVSS |
| 88 | AT13-034 | Heavy chain | QVHLVESGGGVVQPGTSLRLSCAASEFTFSSHAIHWVRQAPGKGLEWVALIWYDGSNNYYADSVKGRFTIS RDSSKNTVHLQMNSLRVEDTAVYYCARDGCTGGSCCYFDNWGQGTLVTVSS |
| 89 | AT13-035 | Heavy chain | QVQLVESGGGVVQPGRSLRLSCAASGFTFSSYGMHWVRQAPGKGLEWVAVISYDGSNKYYADSVKGRFTIS RDNSKNTLYLQMNSLRAEDTAVYYCAKDSYYYGSGRRWGYYFDYWGQGTLVTVSS |
| 90 | AT13-036 | Heavy chain | EVQLVESGGGLVKPGGSLRLSCAASGFTFSSYSMNWVRQAPGKGLEWVSSISSSSTYIYYADSVKGRFTISR DNARNSLYLQMNSLRAEDTAVYYCARRREVGRDGYSLYPRGYHYGMDVWGQGTTVTVSS |
| 91 | AT13-037 | Heavy chain | QVQLVESGGGVVQPGRSLRLSCAASGFTFSTYGMHWVRQAPGKGLEWVAVIWYDGSNTYYADSVKGRFTI SRDNSKNTLYLQIKSLRAEDTAVYYCARGRGYSAQGNRNRAYYFDYWGQGTLVTVSS |
| 92 | AT12-023 | Light chain | SSELTQDPAVSVALGQTVRITCQGDFLRSYYASWYQQKPGQAPVLVIFGKNKRPSGIPDRFSGSSSGNTASL TITGAQAEDEADYYCNSRDRSGNHLVFGGGTKLTVL |
| 93 | AT12-025 | Light chain | DIQMTQSPSSLSASVGDRVTITCRASQSISRYLNWYQQKPGKAPKLLIYAASSLQSGVPSRFSGSGSGTDFTL TISSLQPEDFATYYCQQSYSTPRTFGPGTKVDIK |
| 94 | AT13-024 | Light chain | DIQMTQSPSTLSASVGDRVTITCRASQSISSWLAWYQQKPGKAPKLLIYKASSLESGVPSRFSGTGSGTEFTL TISSLQPDDFATYYCQQYNTYPYTFGQGTKLEIK |
| 95 | AT12-019 | Light chain | AIRLTQSPSSVSASTGDRVTITCRASQAFSSYLVWYQQKPGKAPNLLIYATSTLQGGVPSRFSGSGSGTDFTL TISNLQSEDFATYYCQQYYSYPPTFGQGTKLEIK |
| 96 | AT13-022 | Light chain | SYELTQPPSVSVSPGQTASITCSGDKLGDKYACWYQQKPGQSPVLVIYQDSKRPSGIPERFSGSNSGNTATL TISGTQAMDEADYYCQAWDSSTVVFGGGTKLTVL |
| 97 | AT13-023 | Light chain | DIQMTQSPSSLSASVGDRVTITCRASQGIRNVLGWYQQKPGKAPKCLIYAASSLQSGVPSRFSGSGSGTEFTL TISSLQPEDFATYYCLQHNSHPRTFGQGTKVEIK |
| 98 | AT13-031 | Light chain | DIQMTQSPSSLSASVGDRVTITCRASQGIRNDLGWYQQKPGKAPKRLIYAAVSLQSGVPSRFSGSGSGTEFT LTISSLQPEDFATYYCLQHNSYPRTFGQGTKLEIK |
| 99 | AT12-020 | Light chain | AIRMTQSPSSFSASTGDRVTITCRASQDISSSLAWYQQKPGKAPKLLIYAASTLQSGVPSRFSGSGSGTDFTL TISCLQSEDFATYYCQQYYSYPPTFGQGTRLEIK |
| 100 | AT13-033 | Light chain | QSALTQPPSASGSPGQSVTISCTGTSSDIGGYNYVSWYQHHPGKAPKLMIYEVTKRPSGVPDRFSGSKSGNT ASLTVSGLQAEDEAHYYCSSYAGSNDLLFGGGTKLTVL |
| 101 | AT13-034 | Light chain | EVVMTQSPATLSVSPGERATLSCRASQSISNNLGWYQQKPGQAPRLLIYGASTRATGIPGRFSGSGSGTEFT LTIYSLQSEDFAVYYCQQYNNWPRLTFGGGTKVEIK |
| 102 | AT13-035 | Light chain | SSELTQDPAVSVALGQTVRITCQGDSLRSYYASWYQQKPGQAPVLVIYGKNNRPSGIPDRFSGSSSGNTASL TITGAQAEDEADYYCNSRDSSGNHVVFGGGTKLTVL |
| 103 | AT13-036 | Light chain | QSALTQPASVSGSPRQSITISCTGTSSDVGGYNYVSWYQQLPGKAPKLMIYDVNDRPSGVSIRFSGSKSGNT ASLTISGLQAEDEADYYCSSYTRSNTVIFGGGTKLTVL |
| 104 | AT13-037 | Light chain | EIVMTQSPATLSVSPGERVILSCRASQSVSSNLAWYQQKPGQPPRLLIYGAFTRVTGVPARFSGSGSGTEFTL TISSLQSEDFAVYYCQQYNDRPPYTFGQGTKLEIK |
| 105 | AT12-023 | Heavy chain CDR1 | ggt tac tac tgg agc |
| 106 | AT12-025 | Heavy chain CDR1 | ggt tat tac tgg agc |
| 107 | AT13-024 | Heavy chain CDR1 | agc tat ggc atg cac |
| 108 | AT12-019 | Heavy chain CDR1 | agc tat gcc atg agt |
| 109 | AT13-022 | Heavy chain CDR1 | agc tat ggc atg cac |
| 110 | AT13-023 | Heavy chain CDR1 | ggt tac ttc tgg acc |
| 111 | AT13-031 | Heavy chain CDR1 | ggt tac tac tgg agc |
| 112 | AT12-020 | Heavy chain CDR1 | acc tat agc atg aac |
| 113 | AT13-033 | Heavy chain CDR1 | aat tat ggc atg cac |
| 114 | AT13-034 | Heavy chain CDR1 | tcc cat gcc ata cac |
| 115 | AT13-035 | Heavy chain CDR1 | agc tat ggc atg cac |
| 116 | AT13-036 | Heavy chain CDR1 | agt tat agc atg aac |
| 117 | AT13-037 | Heavy chain CDR1 | acc tat ggc atg cac |
| 118 | AT12-023 | Heavy chain CDR2 | gaa atc aat cat agt gga agc acc aac tac aac ccg tcc ctc aag agt |
| 119 | AT12-025 | Heavy chain CDR2 | gaa atc aat cat agt gga agc acc aac tac aac ccg tcc ctc aag agt |
| 120 | AT13-024 | Heavy chain CDR2 | ttt ata cgg tat gat gga agt aat aaa tac ttt gca gac tcc gtg agg ggc |
| 121 | AT12-019 | Heavy chain CDR2 | act att agg gct agt ggt ggt agc aca agc tac gca gac tcc gtg aag ggc |
| 122 | AT13-022 | Heavy chain CDR2 | ata tca tat gat gga agt aat aaa tac tat gca gac tcc gtg aag ggc |
| 123 | AT13-023 | Heavy chain CDR2 | gaa acc gtt cat agt gga ggc acc aac tac aac ccg tcc ctc aag agt |
| 124 | AT13-031 | Heavy chain CDR2 | gaa atc aat cat agt gga agc acc aac tac aac ccg tcc ctc aag agt |
| 125 | AT12-020 | Heavy chain CDR2 | tcc att agt agt agt agt ggt tac ata tac tac gca gac tca gtg aag ggc |
| 126 | AT13-033 | Heavy chain CDR2 | gtc att tcg cat gat gga agt aag aca tac tat gga cac tcc gtg aag ggc |
| 127 | AT13-034 | Heavy chain CDR2 | ctt ata tgg tat gat gga agt aat aat tat tat gca gac tcc gtg aag ggc |
| 128 | AT13-035 | Heavy chain CDR2 | gtt ata tca tat gat gga agt aat aaa tac tat gca gac tcc gtg aag ggc |
| 129 | AT13-036 | Heavy chain CDR2 | tcc att agt agt agt agt act tac ata tac tac gca gac tca gtg aag ggc |
| 130 | AT13-037 | Heavy chain CDR2 | gtt ata tgg tat gat gga agt aat aca tac tat gca gac tcc gtg aag ggc |
| 131 | AT12-023 | Heavy chain CDR3 | ggc cgt agt acc agc ccg ctc gac tac tac tac tac tac atg gac gtc |
| 132 | AT12-025 | Heavy chain CDR3 | ggc tca atg gca aga ccc aag cca ttt gac tac |
| 133 | AT13-024 | Heavy chain CDR3 | gcg aaa gat ccc caa gag cgt att tat tac tct gat act agt ggt tac ctt gac tac |
| 134 | AT12-019 | Heavy chain CDR3 | tct cct gct atg att cgg gga gtt agg ggg ggt gac tac ttt gac tac |
| 135 | AT13-022 | Heavy chain CDR3 | gat ggg aag ggg att gta gtt att tac tac tac tac ggt atg gac gtc |
| 136 | AT13-023 | Heavy chain CDR3 | ggc ctt aac agc ccc ttt gac tac |
| 137 | AT13-031 | Heavy chain CDR3 | ccc cgg ggc atg tat agc agc tcg tcc ggg gac tac |
| 138 | AT12-020 | Heavy chain CDR3 | gat ggg act ttc tcc tac tac tac tac atg gac gtc |
| 139 | AT13-033 | Heavy chain CDR3 | gcc ggg ttg aac tac tat gga aac cta tta tca aac tac ttc tac tac gga atg gac gtc |
| 140 | AT13-034 | Heavy chain CDR3 | gcg aga gat ggt tgt act ggt ggt agc tgc tgc tat ttt gac aac |
| 141 | AT13-035 | Heavy chain CDR3 | gcg aaa gac tcg tat tac tat ggt tcg ggg aga cga tgg ggc tac tac ttt gac tac |
| 142 | AT13-036 | Heavy chain CDR3 | gcg aga agg agg gag gtc ggt aga gat ggc tac agt ttg tac ccc cgg ggg tac cac tac ggt atg gac gtc |
| 143 | AT13-037 | Heavy chain CDR3 | gcg aga ggc cgt gga tat agt gcc caa ggg aat cgg aat agg gct tac tac ttt gac tac |
| 144 | AT12-023 | Light chain CDR1 | caa gga gac ttc ctc aga agc tat tat gca agc |
| 145 | AT12-025 | Light chain CDR1 | cgg gca agt cag agc att agc agg tat tta aat |
| 146 | AT13-024 | Light chain CDR1 | cgg gcc agt cag agt att agt agc tgg ttg gcc |
| 147 | AT12-019 | Light chain CDR1 | cgg gcg agt cag gct ttt agc agt tat tta gtc |
| 148 | AT13-022 | Light chain CDR1 | tct gga gat aaa ttg ggg gat aaa tat gct tgc |
| 149 | AT13-023 | Light chain CDR1 | cgg gca agt cag ggc att aga aat gtt tta ggc |
| 150 | AT13-031 | Light chain CDR1 | cgg gca agt cag ggc att aga aat gat tta ggc |
| 151 | AT12-020 | Light chain CDR1 | cgg gcg agt cag gat att agc agt tct tta gcc |
| 152 | AT13-033 | Light chain CDR1 | act ggg acc agc agt gac att ggt ggt tat aac tat gtc tcc |
| 153 | AT13-034 | Light chain CDR1 | agg gcc agt cag agc att agc aac aac tta ggc |
| 154 | AT13-035 | Light chain CDR1 | caa gga gac agc ctc aga agc tat tat gca agc |
| 155 | AT13-036 | Light chain CDR1 | act gga acc agc agt gac gtt ggt ggt tat aac tat gtc tcc |
| 156 | AT13-037 | Light chain CDR1 | agg gcc agt cag agt gtt agc agc aac tta gcc |
| 157 | AT12-023 | Light chain CDR2 | ggt aaa aac aag cgg ccc tca |
| 158 | AT12-025 | Light chain CDR2 | gct gca tcc agt ttg caa agt |
| 159 | AT13-024 | Light chain CDR2 | aag gcg tct agt tta gaa agt |
| 160 | AT12-019 | Light chain CDR2 | gct aca tcc act ttg caa ggt |
| 161 | AT13-022 | Light chain CDR2 | caa gat agc aag cgg ccc tca |
| 162 | AT13-023 | Light chain CDR2 | gct gca tcc agt ttg caa agt |
| 163 | AT13-031 | Light chain CDR2 | gct gca gtc agt ttg caa agt |
| 164 | AT12-020 | Light chain CDR2 | gct gca tcc act ttg caa agt |
| 165 | AT13-033 | Light chain CDR2 | gag gtc act aag cgg ccc tca |
| 166 | AT13-034 | Light chain CDR2 | ggt gca tcc acc agg gcc act |
| 167 | AT13-035 | Light chain CDR2 | ggt aaa aac aac cgg ccc tca |
| 168 | AT13-036 | Light chain CDR2 | gat gtc aat gat cgg ccc tca |
| 169 | AT13-037 | Light chain CDR2 | ggt gca ttc acg agg gtc act |
| 170 | AT12-023 | Light chain CDR3 | aac tcc cgg gac cgc agt ggt aac cac ctg gtg |
| 171 | AT12-025 | Light chain CDR3 | caa cag agt tac agt acc cct cgc act |
| 172 | AT13-024 | Light chain CDR3 | caa cag tat aat act tac ccg tac act |
| 173 | AT12-019 | Light chain CDR3 | caa cag tat tat agt tac cct ccg act |
| 174 | AT13-022 | Light chain CDR3 | cag gcg tgg gac agc agc act gtg gta ttc |
| 175 | AT13-023 | Light chain CDR3 | cta cag cat aat agt cac ccc cgg acg |
| 176 | AT13-031 | Light chain CDR3 | cta cag cat aat agt tac cct cgg act |
| 177 | AT12-020 | Light chain CDR3 | caa cag tat tat agt tac cct ccg acg |
| 178 | AT13-033 | Light chain CDR3 | agc tca tat gca ggc agc aac gat ttg cta |
| 179 | AT13-034 | Light chain CDR3 | caa caa tat aat aac tgg cct cgg ctc act |
| 180 | AT13-035 | Light chain CDR3 | aac tcc cgg gac agc agt ggt aac cat gtg gta |
| 181 | AT13-036 | Light chain CDR3 | agc tca tat aca aga agc aac act gtg ata |
| 182 | AT13-037 | Light chain CDR3 | cag cag tac aat gac cgg ccc ccg tac act |
| 183 | AT12-023 | Heavy chain | cag gtg cag cta cag cag tgg ggc gca gga ctg ttg aag cct tcg gag acc ctg tcc ctc acc tgc gct gtc tat ggt ggg tcc ttc agt ggt tac tac tgg agc tgg atc cgc cag ccc cca ggg aag ggg ctg gag tgg att ggg gaa atc aat cat agt gga agc acc aac tac aac ccg tcc ctc aag agt cga gtc acc ata tca gta gac acg tcc aag aac cag ttc tcc ctg aag ctg agc tct gtg acc gcc gcg gac acg gct gtg tat tac tgt gcg agg ggc cgt agt acc agc ccg ctc gac tac tac tac tac tac atg gac gtc tgg gcc aaa ggg acc acg gtc acc gtc tcc tca |
| 184 | AT12-025 | Heavy chain | cag gtg cag cta cag cag tgg ggc gca gga ctg ttg aag cct tcg gag acc ctg tcc ctc acc tgc gct gtc tat ggt ggg tcc ttc agt ggt tat tac tgg agc tgg atc cgc cag ccc cca ggg aag ggg ctg gag tgg att ggg gaa atc aat cat agt gga agc acc aac tac aac ccg tcc ctc aag agt cga gtc acc ata tca gta gac acg tcc aag aac cag ttc tcc ctg aag ctg agc tct gtg acc gcc gcg gac acg gct gtg tat tac tgt gcg aga ggc tca atg gca aga ccc aag cca ttt gac tac tgg ggc cag gga acc ctg gtc acc gtc tcc tca |
| 185 | AT13-024 | Heavy chain | cag gtg cag ctg gtg gag tct ggg gga ggc gtg gtc cag cct ggg agg tcc ctg aga ctc tcc tgt gca gcg tct gga ttc acc ttc agt agc tat ggc atg cac tgg gtc cgc cag gct cca ggc aag ggg ctg gag tgg gtg gca ttt ata cgg tat gat gga agt aat aaa tac ttt gca gac tcc gtg agg ggc cga ttc acc atc tcc aga gac aat tcc aag aac acg ctg ttt ctg caa atg aac agc ctg aga gct gag gac acg gct gtg tat tac tgt gcg aaa gat ccc caa gag cgt att tat tac tct gat act agt ggt tac ctt gac tac tgg ggc cag gga acc ctg gtc acc gtc tcc tca |
| 186 | AT12-019 | Heavy chain | gag gtg cac ctg ttg gag tct ggg gga ggc ttg gta cag cct ggg ggg tcc ctg aga ctc tcc tgt gca gcc tct gga ttc acc ttt agc agc tat gcc atg agt tgg gtc cgc cag gct cca ggg aag ggg ctg gag tgg gtc tca act att agg gct agt ggt ggt agc aca agc tac gca gac tcc gtg aag ggc cgg ttc acc atc tcc aga gac aat tcc cag agc agg ttg tat ctg caa atg aac agt ctg aca gcc gag gac acg gcc gta tat tac tgt gcg aaa tct cct gct atg att cgg gga gtt agg ggg ggt gac tac ttt gac tac tgg ggc cag gga acc ctg gtc acc gtc tcc tca |
| 187 | AT13-022 | Heavy chain | cag gtg cag ctg gtg gag tct ggg gga ggc gtg gtc cag cct ggg agg tcc ctg aga ctc tcc tgt gca gcc tct gga ttc acc ttc agt agc tat ggc atg cac tgg gtc cgc cag gct cca ggc aag ggg ctg gag tgg gtg gca gtt ata tca tat gat gga agt aat aaa tac tat gca gac tcc gtg aag ggc cga ttc acc atc tcc aga gac aat tcc aag aac acg ctg tat ctg caa atg aac agc ctg aga gct gag gac acg gct gtg tat tac tgt gcg aaa gat ggg aag ggg att gta gtt att tac tac tac tac ggt atg gac gtc tgg ggc caa ggg acc acg gtc acc gtc tcc tca |
| 188 | AT13-023 | Heavy chain | cag gta cag cta cag cag tgg ggc gca gga ctg ttg aag cct tcg gag acc ctg tcc ctc acc tgc gct gtc tat ggt ggg tcc ttc agt ggt tac ttc tgg acc tgg atc cgc cag ccc cca ggg aag ggg ctg gag tgg att ggg gaa acc gtt cat agt gga ggc acc aac tac aac ccg tcc ctc aag agt cga gtc acc ata tca gtc gac acg tcc aag aac cag ttc tcc ctg agg ctg aac tct gtg acc gcc gcg gac acg gct gtg tat tac tgt gtg aga ggc ctt aac agc ccc ttt gac tac tgg ggc cag gga acc cta gtc acc gtc tcc tca |
| 189 | AT13-031 | Heavy chain | cag gtg cag cta cag cag tgg ggc gca gga ctg ttg aag cct tcg gag acc ctg tcc ctc acc tgc gct gtc tat ggt ggg tcc ttc agt ggt tac tac tgg agc tgg atc cgc cag ccc cca ggg aag ggg ctg gag tgg att ggg gaa atc aat cat agt gga agc acc aac tac aac ccg tcc ctc aag agt cga gtc acc ata tca gta gac acg tcc aag aag cag ttc tcc ctg aag ctg agc tct gtg acc gcc gcg gac acg gct gtg tat tat tgt gcg aga ggc ccc cgg ggc atg tat agc agc tcg tcc ggg gac tac tgg ggc cag gga acc ctg gtc acc gtc tcc tca |
| 190 | AT12-020 | Heavy chain | gag gtg cag ctg gtg gag tct ggg gga ggc ctg gtc aag cct ggg ggg tcc ctg aga ctc tcc tgt gca gcc tct gga ttc acc ttc agt acc tat agc atg aac tgg gtc cgc cag gct cca ggg aag ggg ctg gag tgg gtc tca tcc att agt agt agt agt ggt tac ata tac tac gca gac tca gtg aag ggc cga ttc acc atc tcc aga gac aac gcc aag aac tca ctg tat ctg caa atg aac agc ctg aga gcc gag gac acg gct gtg tat tac tgt gcg aga gat ggg act ttc tcc tac tac tac tac atg gac gtc tgg ggc aaa ggg acc acg gtc acc gtc tcc tca |
| 191 | AT13-033 | Heavy chain | cag gtg cag ctg gtg gag tct ggg gga ggc gtg gtc cag cct ggg agg tcc ctg aga ctc tcc tgt gca gtc tct gga ctc agt ttc agg aat tat ggc atg cac tgg gtc cgc cag gct ccc ggc aag ggg ctg gag tgg gtg gca gtc att tcg cat gat gga agt aag aca tac tat gga cac tcc gtg aag ggc cga ttc acc ata tcc aga gac aaa tcc aag act atg ttg ttt ctc caa atg aac agc ctg aga cct gag gac acg gct gtt tat tac tgt gcg aaa gcc ggg ttg aac tac tat gga aac cta tta tca aac tac ttc tac tac gga atg gac gtc tgg ggc caa ggg acc aca gtc acc gtc tcg tca |
| 192 | AT13-034 | Heavy chain | cag gtg cac ctg gtg gag tct ggg gga ggc gtg gtc cag cct ggg acg tcc ctg aga ctc tcc tgt gca gcg tct gaa ttc acc ttc agt tcc cat gcc ata cac tgg gtc cgc cag gct cca ggc aag ggg ctg gag tgg gtg gca ctt ata tgg tat gat gga agt aat aat tat tat gca gac tcc gtg aag ggc cga ttc acc atc tcc aga gac agt tcc aag aac acg gtg cat ctg caa atg aac agc ctg aga gtc gag gac acg gct gtg tat tac tgt gcg aga gat ggt tgt act ggt ggt agc tgc tgc tat ttt gac aac tgg ggc cag gga acc cta gtc acc gtc tcc tcg |
| 193 | AT13-035 | Heavy chain | cag gtg cag ctg gtg gag tct ggg gga ggc gtg gtc cag cct ggg agg tcc ctg aga ctc tcc tgt gca gcc tct gga ttc acc ttc agt agc tat ggc atg cac tgg gtc cgc cag gct cca ggc aag ggg ctg gag tgg gtg gca gtt ata tca tat gat gga agt aat aaa tac tat gca gac tcc gtg aag ggc cga ttc acc atc tcc aga gac aat tcc aag aac acg ctg tat ctg caa atg aac agc ctg aga gct gag gac acg gct gtg tat tac tgt gcg aaa gac tcg tat tac tat ggt tcg ggg aga cga tgg ggc tac tac ttt gac tac tgg ggc cag gga acc ctg gtc acc gtc tcc tca |
| 194 | AT13-036 | Heavy chain | gag gtg cag ctg gtg gag tct ggg gga ggc ctg gtc aag cct ggg ggg tcc ctg aga ctc tcc tgt gca gcc tct gga ttc acc ttc agt agt tat agc atg aac tgg gtc cgc cag gct cca ggg aag ggg ctg gag tgg gtc tca tcc att agt agt agt agt act tac ata tac tac gca gac tca gtg aag ggc cga ttc acc atc tcc aga gac aac gcc agg aac tca ctg tat ctg caa atg aac agc ctg aga gcc gag gac acg gct gtg tat tat tgt gcg aga agg agg gag gtc ggt aga gat ggc tac agt ttg tac ccc cgg ggg tac cac tac ggt atg gac gtc tgg ggc caa ggg acc acg gtc acc gtc tcc tca |
| 195 | AT13-037 | Heavy chain | cag gtg cag ctg gtg gag tct ggg gga ggc gtg gtc cag cct ggg agg tcc ctg aga ctc tcc tgt gca gcg tct gga ttc acc ttc agt acc tat ggc atg cac tgg gtc cgc cag gct cca ggc aag ggg ctt gag tgg gtg gca gtt ata tgg tat gat gga agt aat aca tac tat gca gac tcc gtg aag ggc cga ttc acc atc tcc aga gac aat tcc aag aac aca ctg tat ctg caa ata aag agc ctg aga gcc gag gac acg gct gtc tat tac tgt gcg aga ggc cgt gga tat agt gcc caa ggg aat cgg aat agg gct tac tac ttt gac tac tgg ggc cag gga acc ctg gtc acc gtc tcc tca |
| 196 | AT12-023 | Light chain | tct tct gag ctg act cag gac cct gct gtg tct gtg gcc ttg gga cag aca gtc agg atc aca tgc caa gga gac ttc ctc aga agc tat tat gca agc tgg tac cag cag aag cca gga cag gcc cct gta ctt gtc atc ttt ggt aaa aac aag cgg ccc tca ggg atc cca gac cga ttc tct ggc tcc agc tca gga aac aca gct tcc ttg acc atc act ggg gct cag gcg gaa gat gag gct gac tat tac tgt aac tcc cgg gac cgc agt ggt aac cac ctg gtg ttc ggc gga ggg acc aag ctg acc gtc cta |
| 197 | AT12-025 | Light chain | gac atc cag atg acc cag tct cca tcc tcc ctg tct gca tct gta gga gac aga gtc acc atc act tgc cgg gca agt cag agc att agc agg tat tta aat tgg tat cag cag aaa cca ggg aaa gcc cct aag ctc ctg atc tat gct gca tcc agt ttg caa agt ggg gtc cca tca agg ttc agt ggc agt gga tct ggg aca gat ttc act ctc acc atc agc agt ctg caa cct gaa gat ttt gca act tac tac tgt caa cag agt tac agt acc cct cgc act ttc ggc cct ggg acc aaa gtg gat atc aaa |
| 198 | AT13-024 | Light chain | gac atc cag atg acc cag tct cct tcc acc ctg tct gca tct gta gga gac aga gtc acc atc act tgc cgg gcc agt cag agt att agt agc tgg ttg gcc tgg tat cag cag aaa cca ggg aaa gcc cct aag ctc ctg atc tat aag gcg tct agt tta gaa agt ggg gtc cca tca agg ttc agc ggc act gga tct ggg aca gaa ttc act ctc acc atc agc agc ctg cag cct gat gat ttt gca act tat tac tgc caa cag tat aat act tac ccg tac act ttt ggc cag ggg acc aag ctg gag atc aaa |
| 199 | AT12-019 | Light chain | gcc atc cgg ttg acc cag tct cca tcc tca gtc tct gca tct aca gga gac aga gtc acc atc act tgt cgg gcg agt cag gct ttt agc agt tat tta gtc tgg tat cag caa aaa cca ggg aaa gcc cct aac ctc ctg atc tac gct aca tcc act ttg caa ggt ggg gtc cca tca agg ttc agc ggc agt gga tct ggg aca gat ttc act ctc acc atc agc aac ctg cag tct gaa gat ttt gca act tat tac tgt caa cag tat tat agt tac cct ccg act ttt ggc cag ggg acc aag ttg gag atc aaa |
| 200 | AT13-022 | Light chain | tcc tat gag ctg act cag cca ccc tca gtg tcc gtg tcc cca gga cag aca gcc agc atc acc tgc tct gga gat aaa ttg ggg gat aaa tat gct tgc tgg tat cag cag aag cca ggc cag tcc cct gtg ctg gtc atc tat caa gat agc aag cgg ccc tca ggg atc cct gag cga ttc tct ggc tcc aac tct ggg aac aca gcc act ctg acc atc agc ggg acc cag gct atg gat gag gct gac tat tac tgt cag gcg tgg gac agc agc act gtg gta ttc ggc gga ggg acc aag ctg acc gtc cta |
| 201 | AT13-023 | Light chain | gac atc cag atg acc cag tct cca tcc tcc ctg tct gca tct gta gga gac aga gtc acc atc act tgc cgg gca agt cag ggc att aga aat gtt tta ggc tgg tat cag cag aaa cca ggg aaa gcc cct aag tgc ctg atc tat gct gca tcc agt ttg caa agt ggg gtc cca tca agg ttc agc ggc agt gga tct ggg aca gaa ttc act ctc aca atc agc agc ctg cag cct gaa gat ttt gca act tat tac tgt cta cag cat aat agt cac ccc cgg acg ttc ggc caa ggg acc aag gtg gaa atc aaa |
| 202 | AT13-031 | Light chain | gac atc cag atg acc cag tct cca tcc tcc ctg tct gca tct gta gga gac aga gtc acc atc act tgc cgg gca agt cag ggc att aga aat gat tta ggc tgg tat cag cag aaa cca ggg aaa gcc cct aag cgc ctg atc tat gct gca gtc agt ttg caa agt ggg gtc cca tca agg ttc agc ggc agt gga tct ggg aca gaa ttc act ctc aca atc agc agc ctg cag cct gaa gat ttt gca act tat tac tgt cta cag cat aat agt tac cct cgg act ttt ggc cag ggg acc aag ctg gag atc aaa |
| 203 | AT12-020 | Light chain | gcc atc cgg atg acc cag tct cca tcc tca ttc tct gca tct aca gga gac aga gtc acc atc act tgt cgg gcg agt cag gat att agc agt tct tta gcc tgg tat cag caa aaa cca ggg aaa gcc cct aag ctc ctg atc tat gct gca tcc act ttg caa agt gga gtc cca tca agg ttc agc ggc agt gga tct ggg aca gac ttc act ctc acc atc agc tgc ctg cag tct gaa gat ttt gca act tat tac tgt caa cag tat tat agt tac cct ccg acg ttc ggc caa ggg acc agg ttg gaa atc aaa |
| 204 | AT13-033 | Light chain | cag tct gcc ctg act cag cct ccc tcc gcg tcc ggg tct cct ggt cag tca gtc acc atc tcc tgt act ggg acc agc agt gac att ggt ggt tat aac tat gtc tcc tgg tac caa cac cac cca ggc aaa gcc ccc aaa ttg atg att tat gag gtc act aag cgg ccc tca ggg gtc cct gat cgt ttc tct ggc tcc aag tct ggc aac acg gcc tcc ctg acc gtc tct gga ctc cag gct gag gat gag gct cat tat tac tgc agc tca tat gca ggc agc aac gat ttg cta ttc ggc gga ggg acc aag ctg acc gtc ctg |
| | | | |
| 205 | AT13-034 | Light chain | gaa gta gtg atg acg cag tct cca gcc acc ctg tct gtg tct cca ggg gaa aga gcc acc ctc tcc tgc agg gcc agt cag agc att agc aac aac tta ggc tgg tat cag cag aaa cct ggc cag gct ccc agg ctc ctc atc tac ggt gca tcc acc agg gcc act ggt atc cca ggc agg ttc agt ggc agt ggg tct ggg aca gag ttc act ctc acc atc tac agc ctg cag tct gag gat ttt gca gtt tat tac tgt caa caa tat aat aac tgg cct cgg ctc act ttc ggc gga ggg acc aag gtg gag atc aaa |
| 206 | AT13-035 | Light chain | tct tct gag ctg act cag gac cct gct gtg tct gtg gcc ttg gga cag aca gtc agg atc aca tgc caa gga gac agc ctc aga agc tat tat gca agc tgg tac cag cag aag cca gga cag gcc cct gta ctt gtc atc tat ggt aaa aac aac cgg ccc tca ggg atc cca gac cga ttc tct ggc tcc agc tca gga aac aca gct tcc ttg acc atc act ggg gct cag gcg gaa gat gag gct gac tat tac tgt aac tcc cgg gac agc agt ggt aac cat gtg gta ttc ggc gga ggg acc aag ctg acc gtc cta |
| 207 | AT13-036 | Light chain | cag tct gcc ctg act cag cct gcc tcc gtg tct ggg tct cct aga cag tcg atc acc atc tcc tgc act gga acc agc agt gac gtt ggt ggt tat aac tat gtc tcc tgg tac caa caa ctc cca ggc aaa gcc ccc aaa ctc atg att tat gat gtc aat gat cgg ccc tca ggg gtt tct att cgc ttc tct ggc tcc aag tct ggc aac acg gcc tcc ctg acc atc tct ggg ctc cag gct gag gac gag gct gat tat tac tgc agc tca tat aca aga agc aac act gtg ata ttc ggc gga ggg acc aaa ctg acc gtc cta |
| 208 | AT13-037 | Light chain | gaa ata gtg atg acg cag tct cca gcc acc ctg tct gtg tct cca ggg gaa agg gtc atc ctc tcc tgc agg gcc agt cag agt gtt agc agc aac tta gcc tgg tac cag cag aaa cct ggc cag cct ccc agg ctc ctc atc tat ggt gca ttc acg agg gtc act ggt gtc cca gcc agg ttc agt ggc agt ggg tct ggg aca gaa ttc act ctc acc atc agc agc ctg cag tct gaa gat ttt gca gtt tat tac tgt cag cag tac aat gac cgg ccc ccg tac act ttt ggc cag ggg acc aag ctg gag atc aaa |

**Table 1B. Preferred anti-AML antibodies according to the invention (CDR numbering according to Kabat et al. 1991)**

| **SEQ ID NO.** | **Antibody** | **Identity** | **Sequence** |
|---|---|---|---|
| 209 | AT14-013 | Heavy chain CDR1 | SPNWWT |
| 210 | AT14-014 | Heavy chain CDR1 | DAWMS |
| 211 | AT14-015 | Heavy chain CDR1 | DFAMS |
| 212 | AT14-016 | Heavy chain CDR1 | SYAMT |
| 213 | AT14-013 | Heavy chain CDR2 | EIYYGGRVSYNSALRS |
| 214 | AT14-014 | Heavy chain CDR2 | HINTKVDGGTTEYAAPVKG |
| 215 | AT14-015 | Heavy chain CDR2 | FIRTKANDGTTEYAASVKG |
| 216 | AT14-016 | Heavy chain CDR2 | SISGSGGSTYYADSVRG |
| 217 | AT14-013 | Heavy chain CDR3 | AGQKNIGCGYSSCFISWFDT |
| 218 | AT14-014 | Heavy chain CDR3 | TTEAIYDSSGYFHDY |
| 219 | AT14-015 | Heavy chain CDR3 | ASDPFMTTDYYYYYMDV |
| 220 | AT14-016 | Heavy chain CDR3 | AKGYVGCSGGNCYSGGAFDI |
| 221 | AT14-013 | Light chain CDR1 | KSSQTILQRSNHLNYLA |
| 222 | AT14-014 | Light chain CDR1 | KSSRSVLYSSNNKNYLA |
| 223 | AT14-015 | Light chain CDR1 | TGTSSDVGGYNSVS |
| 224 | AT14-016 | Light chain CDR1 | GGNNIGSESVH |
| 225 | AT14-013 | Light chain CDR2 | WASTRES |
| 226 | AT14-014 | Light chain CDR2 | WASIRES |
| 227 | AT14-015 | Light chain CDR2 | EVYKRPL |
| 228 | AT14-016 | Light chain CDR2 | YDTDRPS |
| 229 | AT14-013 | Light chain CDR3 | HQYYTTPQT |
| 230 | AT14-014 | Light chain CDR3 | QQYSRPPT |
| 231 | AT14-015 | Light chain CDR3 | SSYGGTVLF |
| 232 | AT14-016 | Light chain CDR3 | QVWDNTSDHPVVF |
| 233 | AT14-013 | Heavy chain | QGRLQESGPGLVKPSETLTLTCAVSGGSSVSSPNWWTWVRQAPGKGLEWIGEIYYGGRVSYNSALRSRVTI SSDRSKEEFSLKLRSVTAADTAIYYCAGQKNIGCGYSSCFISWFDTWGQGIAVTVSS |
| 234 | AT14-014 | Heavy chain | EVQLVESGGGLVKPGGSLRLSCAASGFTFSDAWMSWVRQAPGKGLEWVGHINTKVDGGTTEYAAPVKGR FTISRDDSKNSLYLHMDSLKTEDTAVYYCTTEAIYDSSGYFHDYWGQGSLVTVSS |
| 235 | AT14-015 | Heavy chain | EVQLVESGGGLAQPGRSLRLSCTASGFRFGDFAMSWVRQAPGKGLEWVGFIRTKANDGTTEYAASVKGRF IISRDDSKSIAYLQMNSLKTEDTAVYYCASDPFMTTDYYYYYMDVWGKGTTVTVSS |
| 236 | AT14-016 | Heavy chain | EVQVLESGGDSVQPGGSLRLSCAASGFTFSSYAMTWVRQAPGKGLKWVSSISGSGGSTYYADSVRGRFTISR DNSKNTLYVQMNSLRAEDTAVYYCAKGYVGCSGGNCYSGGAFDIWGQGTVVTVSS |
| 237 | AT14-013 | Light chain | DIVMTQSPDSLAVSLGERATIACKSSQTILQRSNHLNYLAWYQQKPGQPPKVLIYWASTRESGVPDRFSGSG SGTDFTLTINSLQAEDVAVYYCHQYYTTPQTFGQGTKVEIK |
| 238 | AT14-014 | Light chain | DIVMTQSPDSLAVSLGERATINCKSSRSVLYSSNNKNYLAWYQQKPGQPPKLLIYWASIRESGVPDRFSGSG SGTDFTLTINSLQAEDVAVYYCQQYSRPPTFGQGTKVEIK |
| 239 | AT14-015 | Light chain | QSALTQPPSASGSPGQSVTISCTGTSSDVGGYNSVSWYQHHPGKAPKLMIYEVYKRPLGVPDRFSGSKSGN TASLTVSGLQAEDEAYYYCSSYGGTVLFGGGTKLTVL |
| 240 | AT14-016 | Light chain | SYVLTQPPSVSVAPGKTARITCGGNNIGSESVHWYQQKPGQAPVVVIYYDTDRPSGIPERFSGSNSGNTATL TISRVEAGDEADYYCQVWDNTSDHPVVFGGGTKLTVL |
| 241 | AT14-013 | Heavy chain CDR1 | agt cct aac tgg tgg act |
| 242 | AT14-014 | Heavy chain CDR1 | gac gcc tgg atg agc |
| 243 | AT14-015 | Heavy chain CDR1 | gat ttt gct atg agt |
| 244 | AT14-016 | Heavy chain CDR1 | agc tat gcc atg acc |
| 245 | AT14-013 | Heavy chain CDR2 | gaa atc tat tat ggt ggg aga gtg agc tac aac tcg gcc ctc agg agt |
| 246 | AT14-014 | Heavy chain CDR2 | cat att aac acc aaa gtt gat ggt ggg aca aca gag tac gct gca ccc gtg aaa ggc |
| 247 | AT14-015 | Heavy chain CDR2 | ttc att aga acc aaa gct aat gat ggg aca aca gaa tac gcc gcg tct gtg aaa ggc |
| 248 | AT14-016 | Heavy chain CDR2 | agt att agt ggt agt ggt ggt agc aca tac tac gca gac tcc gtg agg ggc |
| 249 | AT14-013 | Heavy chain CDR3 | gcg ggt caa aaa aat att ggc tgt ggt tac agc agt tgc ttt atc agt tgg ttc gac acc |
| 250 | AT14-014 | Heavy chain CDR3 | acc aca gag gcg ata tat gat agt agt ggt tat ttc cat gac tat |
| 251 | AT14-015 | Heavy chain CDR3 | gct agc gat ccc ttc atg act aca gac tat tac tac tac tac atg gac gtc |
| 252 | AT14-016 | Heavy chain CDR3 | gcg aaa gga tat gtg ggg tgt agt ggt ggg aac tgc tac tcg ggg ggt gct ttt gat atc |
| 253 | AT14-013 | Light chain CDR1 | aag tcc agc cag act att tta caa agg tcc aac cat ttg aac tac tta gct |
| 254 | AT14-014 | Light chain CDR1 | aag tcc agc cgg agt gtt tta tac agc tcc aac aat aag aac tac tta gct |
| 255 | AT14-015 | Light chain CDR1 | act ggg acc agc agt gac gtt ggt ggt tat aac tct gtc tcc |
| 256 | AT14-016 | Light chain CDR1 | ggg ggg aac aac att gga agt gaa agt gtt cac |
| 257 | AT14-013 | Light chain CDR2 | tgg gca tct acc cgg gaa tcc |
| 258 | AT14-014 | Light chain CDR2 | tgg gca tct atc cgg gaa tcc |
| 259 | AT14-015 | Light chain CDR2 | gag gtc tat aag cgg ccc tta |
| 260 | AT14-016 | Light chain CDR2 | tat gat acc gac cgg ccc tca |
| 261 | AT14-013 | Light chain CDR3 | cac caa tat tat act act ccg cag act |
| 262 | AT14-014 | Light chain CDR3 | cag caa tat tct cgt cct ccg acg |
| 263 | AT14-015 | Light chain CDR3 | agc tca tat gga ggc acc gtg cta ttc |
| 264 | AT14-016 | Light chain CDR3 | cag gtg tgg gat aac act agt gat cat cct gtg gta ttc |
| 265 | AT14-013 | Heavy chain | cag ggg cga ctg cag gag tcg ggc cca gga ctg gtg aag cct tcg gag acc ctg acc ctc acg tgc gct gtg tcc ggt ggc tcc tcc gtc agc agt cct aac tgg tgg act tgg gtc cgc cag gcc ccc ggg aag ggg ctg gag tgg att gga gaa atc tat tat ggt ggg aga gtg agc tac aac tcg gcc ctc agg agt cga gtc acc att tca tca gac agg tcc aaa gag gag ttc tcc ctg aaa ctg agg tct gtg acc gcc gcg gac acg gcc ata tat tat tgt gcg ggt caa aaa aat att ggc tgt ggt tac agc agt tgc ttt atc agt tgg ttc gac acc tgg gga cag gga att gcg gtc acc gtc tcc tca |
| 266 | AT14-014 | Heavy chain | gag gtg cag ctg gtg gag tct ggg gga ggt ttg gta aag cct ggg ggg tcc ctt aga ctc tcc tgt gca gcc tct gga ttc act ttc agt gac gcc tgg atg agc tgg gtc cgc cag gct cca ggg aag ggg ctg gag tgg gtt ggc cat att aac acc aaa gtt gat ggt ggg aca aca gag tac gct gca ccc gtg aaa ggc aga ttc acc atc tca aga gat gat tca aaa aat tcg ctg tat ctg cac atg gac agc ctg aaa acc gag gac aca gcc gtg tat tac tgt acc aca gag gcg ata tat gat agt agt ggt tat ttc cat gac tat tgg ggc cag gga tcc ctg gtc acc gtc tcc tca |
| 267 | AT14-015 | Heavy chain | gag gtg cag ctg gtg gag tcg ggg gga ggc ttg gca cag cca ggg cgg tcc ctg aga ctc tcc tgt aca gct tct gga ttc agg ttt ggt gat ttt gct atg agt tgg gtc cgc cag gct cca ggg aag gga ctg gag tgg gta ggt ttc att aga acc aaa gct aat gat ggg aca aca gaa tac gcc gcg tct gtg aaa ggc aga ttc atc atc tca aga gat gat tcc aaa agt atc gcc tat ctg caa atg aac agc ctg aaa acc gag gac aca gcc gtt tat tac tgt gct agc gat ccc ttc atg act aca gac tat tac tac tac tac atg gac gtc tgg ggc aaa ggg acc acg gtc acc gtc tcc tca |
| 268 | AT14-016 | Heavy chain | gag gtg caa gtg ttg gag tct ggg gga gac tcg gta cag cct ggg ggg tcc ctg aga ctc tcc tgt gca gcc tct gga ttc acc ttt agc agc tat gcc atg acc tgg gtc cgc cag gct cca ggg aag ggg ctg aaa tgg gtc tca agt att agt ggt agt ggt ggt agc aca tac tac gca gac tcc gtg agg ggc cgg ttc acc atc tcc aga gac aat tcc aag aac acg ctg tat gtg cag atg aac agc ctg aga gcc gag gac acg gcc gta tat tac tgt gcg aaa gga tat gtg ggg tgt agt ggt ggg aac tgc tac tcg ggg ggt gct ttt gat atc tgg ggc caa ggg aca gtg gtc acc gtc tct tca |
| 269 | AT14-013 | Light chain | gac atc gtg atg acc cag tct cca gac tcc ctg gct gtg tct ctg ggc gag agg gcc acc atc gcc tgc aag tcc agc cag act att tta caa agg tcc aac cat ttg aac tac tta gct tgg tac cag cag aaa cca gga cag cct cct aaa gtg ctc att tat tgg gca tct acc cgg gaa tcc ggg gtc cct gac cga ttc agt ggc agc ggg tct ggg aca gat ttc act ctc acc atc aac agc ctg cag gct gag gat gtg gca gtt tat tac tgt cac caa tat tat act act ccg cag act ttt ggc cag ggg acc aag gtg gag atc aaa |
| 270 | AT14-014 | Light chain | gac atc gtg atg acc cag tct cca gac tcc ctg gct gtg tct ctg ggc gag agg gcc acc atc aac tgc aag tcc agc cgg agt gtt tta tac agc tcc aac aat aag aac tac tta gct tgg tac cag cag aaa cca gga cag cct cct aag ctg ctc att tac tgg gca tct atc cgg gaa tcc ggg gtc cct gac cga ttc agt ggc agc ggg tct ggg aca gat ttc act ctc acc atc aac agc ctg cag gct gaa gat gtg gca gtt tat tac tgt cag caa tat tct cgt cct ccg acg ttc ggc caa ggg acc aag gtg gaa atc aaa |
| 271 | AT14-015 | Light chain | cag tct gcc ctg act cag cct ccc tcc gcg tcc ggg tct cct gga cag tca gtc acc atc tcc tgc act ggg acc agc agt gac gtt ggt ggt tat aac tct gtc tcc tgg tac caa cat cac cca ggc aaa gcc ccc aaa ctc atg att tat gag gtc tat aag cgg ccc tta ggg gtc cct gat cgc ttc tct ggc tcc aag tct ggc aac acg gcc tcc ctg acc gtc tct ggg ctc cag gct gag gat gag gct tat tat tac tgc agc tca tat gga ggc acc gtg cta ttc ggc gga ggg acc aag ctg acc gtc cta |
| 272 | AT14-016 | Light chain | tcc tat gtg ctg act cag cca ccc tca gtg tca gtg gcc cca gga aag acg gcc cgg att acc tgt ggg ggg aac aac att gga agt gaa agt gtt cac tgg tac cag cag aag cca ggc cag gcc cct gtg gtg gtc atc tat tat gat acc gac cgg ccc tca ggg atc cct gag cgc ttc tct ggc tcc aac tct ggg aac acg gcc acc ctg acc atc agc agg gtc gaa gcc ggg gat gag gcc gac tat tac tgt cag gtg tgg gat aac act agt gat cat cct gtg gta ttc ggc gga ggg acc aag ctg acc gtc cta |

**Tabel 2**

| mAb are specific for AML | | | | | | | | | | | | | | |
|---|---|---|---|---|---|---|---|---|---|---|---|---|---|---|
| mAbs | | Cell lines | | | | | | | | | | | Prim cells | |
| Name | Ig class | Mel¹ | Mel² | Mel³ | BJ | FB | Col¹ | Col² | Col³ | Vero | HUVEC | HepG2 | PBMC | BM |
| AT12-019 | IgG1 κ | - | - | - | - | - | - | - | - | - | - | - | - | - |
| AT13-023 | IgG1 κ | - | - | - | - | - | - | - | - | - | - | - | - | - |
| AT13-031 | IgG1 κ | | | | | - | | | | - | - | - | - | - |
| AT12-020 | IgG3 κ | - | - | - | - | - | - | - | - | - | - | - | - | - |
| AT12-023 | IgG3 λ | - | - | - | - | - | - | - | - | - | - | - | - | - |
| AT12-025 | IgG3 κ | - | - | - | - | - | - | - | - | - | - | - | - | - |
| AT13-022 | IgG3 λ | - | - | - | - | - | - | - | - | - | - | - | - | - |
| AT13-024 | IgG3 κ | - | - | - | - | - | - | - | - | - | - | - | - | - |

| | | | | | | | | | | | | | | |
|---|---|---|---|---|---|---|---|---|---|---|---|---|---|---|
| Mel¹: Mel 126.2; Mel²: Mel BLM; Mel³: Mel WBO; BJ: Fibroblast cell line; FB: primary fibroblasts (skin); Col¹: Colo205; Col²: Caco-2; Col³ HT29; Vero: non-human kidney; PBMC: peripheral blood mononuclear cells; BM: bone marrow. | | | | | | | | | | | | | | |

**Tabel 3**

| Overview AML-specific mAbs derived from a patient with a potent GvL response (donor 59) | | | | | | | | | | |
|---|---|---|---|---|---|---|---|---|---|---|
| mAbs | | | | AML | | Primary AML* | | | | |
| Name | Ig class | CD27 | SHM V_{H}/V_{L} | THP-1 | MM6 | M0 | M1 | M1 | M1 | M4 |
| AT12-019 | IgG1 κ | + | 10/9 | ++ | + | + | + | ++ | +/- | - |
| AT13-023 | IgG1 κ | + | 8/4 | ++ | | ++ | + | | | |
| AT13-031 | IgG1 κ | + | 1/1 | ++ | + | - | + | - | - | +/- |
| AT12-020 | IgG3 κ | + | 4/2 | ++ | | | | | | |
| AT12-023 | IgG3 λ | - | Germline/6 | +++ | ++ | + | ++ | ++ | +/- | ++ |
| AT12-025 | IgG3 κ | - | 1/1 | ++ | ++ | - | +/- | + | - | - |
| AT13-022 | IgG3 λ | + | Germline | ++ | - | ++ | ++ | | | |
| AT13-024 | IgG3 κ | - | 6/3 | ++ | ++ | ++ | ++ | ++ | | |

| | | | | | | | | | | |
|---|---|---|---|---|---|---|---|---|---|---|
| MM6: MonoMac6; M0: donor 77; M1 donor 69, 79, 86 respectively; M4 donor 78 * AML according to the FAB classification; SHM: number of somatic hypermutations | | | | | | | | | | |

**Tabel 4**

| Some mAb bind other hematologic tumors | | | | | | | |
|---|---|---|---|---|---|---|---|
| mAbs | | Other hematologic tumor cell lines | | | | Primary tumors | |
| Name | Ig class | OCI-Ly1 | CC1-Lv7 | U266 | NCI-H929 | NHL pt | ALL pt |
| AT12-019 | IgG1 κ | + | - | - | - | - | - |
| AT13-023 | IgG1 κ | | | | | | |
| AT13-031 | IgG1 κ | ++ | + | - | +/- | ++ | - |
| AT12-020 | IgG3 κ | | | | | | |
| AT12-023 | IgG3 λ | - | - | +/- | - | ++ | - |
| AT12-025 | IgG3 κ | - | - | + | - | - | - |
| AT13-022 | IgG3 λ | | | | | | |
| AT13-024 | IgG3 κ | ++ | + | - | - | - | - |

| | | | | | | | |
|---|---|---|---|---|---|---|---|
| OCI-Ly1 and OCI-Ly7: diffuse large B cell lymphoma cell lines; U266 and NCI-H929: multiple myeloma cell lines; NHL pt: B-non Hodgkin lymphoma cells freshly isolated from a newly diagnosed patient ; ALL pt: B-acute lymphatic leukemia cells freshly isolated from a newly diagnosed patient. | | | | | | | |

**Tabel 5**

| Some mAb show *in vitro* activity | | | | | |
|---|---|---|---|---|---|
| mAbs | | | | Killing | |
| Name | Ig class | CD27 | SHM V_{H}/V_{L} | THP-1 | M0/5 |
| AT12-019 | IgG1 κ | + | 10/9 | no | no |
| AT13-023 | IgG1 κ | + | 8/4 | | |
| AT13-031 | IgG1 κ | + | 1/1 | | |
| AT12-020 | IgG3 K | + | 4/2 | | |
| AT12-023 | IgG3 λ | - | Germline/6 | yes | |
| AT12-025 | IgG3 K | - | 1/1 | yes | yes |
| AT13-022 | IgG3 λ | + | Germline | | |
| AT13-024 | IgG3 K | - | 6/3 | no | yes |

| | | | | | |
|---|---|---|---|---|---|
| AT13-023, AT13-031, AT12-020 and AT13-022 were not yet tested for in vitro activity | | | | | |

**Tabel 6**

| 2nd AML patient with GvL response (donor 58) | | | | | | | | | |
|---|---|---|---|---|---|---|---|---|---|
| mAbs | | | | AML | | Not binding to | | | |
| Clone | CD27 | Ig class | SHM V_{H}/V_{L} | THP-1 | MM6 | PBMC | Caco | HT-29 | HepG2 |
| AT13-033 | + | IgG3 λ | 18/5 | ++ | ++ | - | - | - | - |
| AT13-034 | + | IgG3 K | 14/6 | ++ | ++ | - | - | - | - |
| AT13-035 | - | IgG3 λ | 0/0 | ++ | ++ | - | - | - | - |
| AT13-036 | - | IgG3 λ | 4/9 | ++ | ++ | - | - | - | - |
| AT13-037 | + | IgG3 K | 4/8 | ++ | ++ | - | - | - | - |

| | | | | | | | | | |
|---|---|---|---|---|---|---|---|---|---|
| SHM: number of somatic hypermutations | | | | | | | | | |

**Table 7**

| Overview of AML-specific mAbs derived from a 3^{rd} patient with a potent GvL response (donor 101) | | | | | | | | | |
|---|---|---|---|---|---|---|---|---|---|
| mAbs | | | | AML | | Primary AML* | | | |
| Name | Ig class | CD27 | SHM V_{H}/V_{L} | THP-1 | Molm13 | M0 | M4 | M5¹ | M5² |
| AT14-013 | IgG1 κ | + | 26/11 | +++ | +++ | +++ | ++ | +/- | + |
| AT14-014 | IgG3 κ | + | 9/6 | ++ | ++ | - | - | - | - |
| AT14-015 | IgG3 λ | + | 9/9 | ++ | - | - | + | - | - |
| AT14-016 | IgG3 λ | + | 9/5 | ++ | ++ | - | + | - | - |

| | | | | | | | | | |
|---|---|---|---|---|---|---|---|---|---|
| M1: donor 77; M4: donor BL-046; M5¹: donor BL-034, M5²: donor BL-038 * AML according to the FAB classification; SHM: number of somatic hypermutations | | | | | | | | | |

**Table 8**

| mAb are specific for AML (donor 101) | | | | | | | | | | |
|---|---|---|---|---|---|---|---|---|---|---|
| mAbs | | Cell lines | | | | PBMCs | | | | Primary cells |
| Name | Ig class | FB | Col | Liv¹ | Liv² | CD3 | CD14 | CD19 | CD56 | Fetal liver cells |
| AT14-013 | IgG1 κ | +/- | - | +/- | - | - | - | - | - | - |
| AT14-014 | IgG3 κ | - | - | - | - | - | - | - | - | - |
| AT14-015 | IgG3 λ | - | - | - | - | - | - | - | - | - |
| AT14-016 | IgG3 λ | - | - | - | - | - | - | - | - | - |

| | | | | | | | | | | |
|---|---|---|---|---|---|---|---|---|---|---|
| FB: primary fibroblasts (skin); Col: Caco-2; Liv¹: Huh7 liver cell line; Liv²: HepG2 liver cell line PBMC: peripheral blood mononuclear cells; | | | | | | | | | | |

**Tabel 9A**

| Overview of AML-specific mAbs derived from a 2^{nd} patient with a potent GvL response (donor 58) | | | | | | | | | |
|---|---|---|---|---|---|---|---|---|---|
| mAbs | | | | AML | | Primary AML* | | | |
| Clone | CD27 | Ig class | SHM V_{H}/V_{L} | THP-1 | MM6 | M0 | M0 | M1 | M4 |
| AT13-033 | + | IgG3 λ | 18/5 | ++ | ++ | - | - | - | - |
| AT13-034 | + | IgG3 κ | 14/6 | ++ | ++ | +/- | - | - | - |
| AT13-035 | - | IgG3 λ | 0/0 | ++ | ++ | - | - | - | - |
| AT13-036 | - | IgG3 λ | 4/9 | ++ | ++ | - | - | - | - |
| AT13-037 | + | IgG3 κ | 4/8 | ++ | ++ | - | - | - | - |

| | | | | | | | | | |
|---|---|---|---|---|---|---|---|---|---|
| MM6: MonoMac6; M0: donor 77, BL-030; M1 donor 69, respectively; M4 donor 78 * AML according to the FAB classification; SHM: number of somatic hypermutations | | | | | | | | | |

**Tabel 9B**

| Some mAb show *in vitro* activity (donor 59) | | | | | |
|---|---|---|---|---|---|
| mAbs | | | | Killing | |
| Name | Ig class | CD27 | SHM V_{H}/V_{L} | THP-1 | M1 |
| AT12-019 | IgG1 κ | + | 10/ 9 | no | no |
| AT13-023 | IgG1 κ | + | 8/4 | no | nd |
| AT13-031 | IgG1 κ | + | 1/1 | yes* | nd |
| AT12-020 | IgG3 κ | + | 4/2 | nd | nd |
| AT12-023 | IgG3 λ | - | Germline/6 | yes | nd |
| AT12-025 | IgG3 κ | - | 1/1 | yes | yes |
| AT13-022 | IgG3 λ | + | Germline | no | nd |
| AT13-024 | IgG3 κ | - | 6/3 | no | yes |

| | | | | | |
|---|---|---|---|---|---|
| * Only at 37 C; nd = not determined | | | | | |

**Tabel 10**

| Some mAb show *in vitro* activity (donor 58) | | | | |
|---|---|---|---|---|
| mAbs | | | | Killing |
| Clone | CD27 | Ig class | SHM V_{H}/V_{L} | THP-1 |
| AT13-033 | + | IgG3 λ | 18/5 | yes |
| AT13-034 | + | IgG3 κ | 14/6 | no |
| AT13-035 | - | IgG3 λ | 0/0 | yes |
| AT13-036 | - | IgG3 λ | 4/9 | yes |
| AT13-037 | + | IgG3 κ | 4/8 | yes |

### References

Bakker AB, van den Oudenrijn S, Bakker AQ, Feller N, van Meijer M, Bia JA, Jongeneelen MA, Visser TJ, Bijl N, Geuijen CA, Marissen WE, Radosevic K, Throsby M, Schuurhuis GJ, Ossenkoppele GJ, de Kruif J, Goudsmit J, Kruisbeek AM. 2004. C-type lectin-like molecule-1: a novel myeloid cell surface marker associated with acute myeloid leukemia. Cancer Res 64:8443-50.
Bennett JM, Catovsky D, Daniel MT, Flandrin G, Galton DA, Gralnick HR, Sultan C. 1976. Proposals for the classification of the acute leukaemias. French-American-British (FAB) co-operative group. Br J Haematol 33:451-458.
Bhat NM, Bieber MM, Hsu FJ, Chapman CJ, Spellerberg M, Stevenson FK, Teng NNH. 1997. Rapid cytotoxicity of human B lymphocytes induced by VH4-34 (VH4.21) gene-encoded monoclonal antibodies. Clin Exp Immunol 108: 151-159
Biernacki MA, Marina O, Zhang W, Liu F, Bruns I, Cai A, Neuberg D, Canning CM, Alyea EP, Soiffer RJ, Brusic V, Ritz J, Wu CJ. 2010. Efficacious immune therapy in chronic myelogenous leukemia (CML) recognizes antigens that are expressed on CML progenitor cells. Cancer Research 70:906-915.
Chen Y, Wiesmann C, Fuh G, Li B, Christinger HW, McKay P, de Vos AM, Lowman HB. 1999. Selection and analysis of an optimized anti-VEGF antibody: crystal structure of an affinity-matured Fab in complex with antigen. J Mol Biol 293:865-881.
Diehl SA, Schmidlin H, Nagasawa M, van Haren SD, Kwakkenbos MJ, Yasuda E, Beaumont T, Scheeren FA, Spits H. 2008. STAT3-mediated up-regulation of BLIMP1 Is coordinated with BCL6 down-regulation to control human plasma cell differentiation. J Immunol 180:4805-4815.
Drexler HG, Minowada J. 1998. History and classification of human leukemia-lymphoma cell lines. Leuk Lymphoma 31:305-316.
Hernandez AM, Rodriguez N, Gonzalez JE, et al. Anti-NeuGcGM3 Antibodies, Actively Elicited by Idiotypic Vaccination in Nonsmall Cell Lung Cancer Patients, Induce Tumor Cell Death by an Oncosis-Like Mechanism. J Immunol 2011; 186(6):3735-3744.
Hugh J. M et al, 2004 Methods in Molecular biology Vol. 282
Kattah NH, Kattah MG, Utz PJ. The U1-snRNP complex: structural properties relating to autoimmune pathogenesis in rheumatic diseases. Immunol Reviews. 2010;233(1):126-145.
Kepp O, Galluzzi L, Lipinski M, Yuan J, Kroemer G. Cell death assays for drug discovery. Nat Rev Drug Discov 2011;1-17.
Kwakkenbos MJ, Diehl SA, Yasuda E, Bakker AQ, Van Geelen CMM, Lukens MV, Van Bleek GM, Widjojoatmodjo MN, Bogers WMJM, Mei H, Radbruch A, Scheeren FA, Spits H, Beaumont T. 2010. Generation of stable monoclonal antibody-producing B cell receptor-positive human memory B cells by genetic programming. Nat Med 16:123-128.
Majeti R, Chao MP, Alizadeh AA, Pang WW, Jaiswal S, Gibbs KD, Van Rooijen N, Weissman IL. 2009. CD47 is an adverse prognostic factor and therapeutic antibody target on human acute myeloid leukemia stem cells. Cell 138:286-299
Schlenk RF, Döhner K, Krauter J, Fröhling S, Corbacioglu A, Bullinger L, Habdank M, Späth D, Morgan M, Benner A, Schlegelberger B, Heil G, Ganser A, Döhner H, German-Austrian Acute Myeloid Leukemia Study Group. 2008. Mutations and treatment outcome in cytogenetically normal acute myeloid leukemia. N Engl J Med 358:1909-1918.
Schmid C, Labopin M, Nagler A, Bornhäuser M, Finke J, Fassas A, Volin L, Gürman G, Maertens J, Bordigoni P, Holler E, Ehninger G, Polge E, Gorin N-C, Kolb H-J, Rocha V, EBMT Acute Leukemia Working Party. Donor lymphocyte infusion in the treatment of first hematological relapse after allogeneic stem-cell transplantation in adults with acute myeloid leukemia: a retrospective risk factors analysis and comparison with other strategies by the EBMT Acute Leukemia Working Party. Journal of Clinical Oncology 25:4938-4945.
Schmiedel BJ, Werner A, Steinbacher J, et al. Generation and Preclinical Characterization of a Fc-optimized GITR-Ig Fusion Protein for Induction of NK Cell Reactivity Against Leukemia. Mol Ther. 2013;21(4):877-886.
Singh R, Cadeddu R-P, Fröbel J, et al. The non-steroidal anti-inflammatory drugs Sulindac sulfide and Diclofenac induce apoptosis and differentiation in human acute myeloid leukemia cells through an AP-1 dependent pathway. Apoptosis. 2011;16(9):889-901.
Tsuchiya S, Yamabe M, Yamaguchi Y, Kobayashi Y, Konno K, Tada K. 1988. Establishment and characterization of a human acute monocytic leukemia cell line (THP-1). Int J Cancer 26:171-176.
Walter RB, Appelbaum FR, Estey EH, Bernstein ID. Acute myeloid leukemia stem cells and CD33-targeted immunotherapy. 2012. Blood 119: 6198-208.
Willingham SB, Volkmer JP, Gentles AJ, Sahoo D, Dalerba P, Mitra SS, Wang J, Contreras-Trujillo H, Martin R, Cohen JD, Lovelace P, Scheeren FA, Chao MP, Weiskopf K, Tang C, Volkmer AK, Naik TJ, Storm TA, Mosley AR, Edris B, Schmid SM, Sun CK, Chua MS, Murillo O, Rajendran P, Cha AC, Chin RK, Kim D, Adorno M, Raveh T, Tseng D, Jaiswal S, Enger PØ, Steinberg GK, Li G, So SK, Majeti R, Harsh GR, van de Rijn M, Teng NN, Sunwoo JB, Alizadeh AA, Clarke MF, Weissman IL. 2012. The CD47-signal regulatory protein alpha (SIRPa) interaction is a therapeutic target for human solid tumors. Proc Natl Acad Sci USA 109:6662-7.
Wu CJ, Yang XF, McLaughlin S, Neuberg D, Canning C, Stein B, Alyea EP, Soiffer RJ, Dranoff G, Ritz J. 2000. Detection of a potent humoral response associated with immune-induced remission of chronic myelogenous leukemia. J Clin Invest 106:705-714.

## Claims

1. An isolated, synthetic or recombinant antibody, or a functional part thereof capable of binding to AML cells, comprising:
- a heavy chain CDR1, CDR2 and CDR3 sequence and a light chain CDR1, CDR2 and CDR3 sequence as defined by SEQ ID NO: 209, 213, 217, 221, 225 and 229 respectively and which is able to bind a cell surface component of different AML cells of at least three FAB classifications.

2. An antibody or functional part thereof according to claim 1, comprising a variable heavy chain sequence having at least 90% sequence identity with a sequence comprising SEQ ID NO: 233, and/or comprising a variable light chain sequence having at least 90% sequence identity with a sequence comprising SEQ ID NO: 237.

3. An antibody or functional part thereof according to claim 1 or 2, wherein said antibody is of the IgG isotype, preferably IgG1 or IgG3.

4. An antibody or functional part thereof according to any one of claims 1-3, which is coupled to another compound, preferably wherein said other compound is a detectable label, a chemotherapeutic drug, a toxic moiety, an immunomodulatory molecule, another AML-specific binding compound, or a radioactive compound.

5. An isolated, synthetic or recombinant nucleic acid molecule, which encodes at least the variable heavy chain sequence and/or the variable light chain sequence of an antibody or functional part thereof according to any one of claims 1-3, preferably wherein said nucleic acid molecule comprises a sequence which has at least 80% sequence identity with a sequence comprising SEQ ID NO: 265, and/or a sequence which has at least 80% sequence identity with a sequence comprising SEQ ID NO: 269.

6. A nucleic acid molecule or functional equivalent thereof according to claim 5, wherein said nucleic acid molecule or functional equivalent comprises DNA, RNA, cDNA, peptide nucleic acid (PNA), locked nucleic acid (LNA) or a DNA/RNA helix.

7. A nucleic acid molecule or functional equivalent thereof, encoding an antibody or functional part thereof according to any one of claims 1-4.

8. A vector comprising a nucleic acid molecule according to any one of claims 5-7.

9. An isolated or recombinant cell, comprising a nucleic acid molecule according to any one of claims 5-7, or a vector according to claim 8.

10. A composition comprising an antibody or functional part thereof according to any one of claims 1-4, or a nucleic acid molecule according to any one of claims 5-7, or a vector according to claim 8, or a cell according to claim 9, preferably wherein said composition is a pharmaceutical composition which comprises a pharmaceutically acceptable carrier, diluent or excipient, more preferably wherein said pharmaceutical composition comprises at least two antibodies or functional parts thereof according to any one of claims 1-4.

11. An antibody or functional part according to any one of claims 1-4, or a nucleic acid molecule according to any one of claims 5-7, or a vector according to claim 8, or a cell according to claim 9, for use as a medicament or prophylactic agent.

12. An antibody or functional part according to any one of claims 1-4, or a nucleic acid molecule according to any one of claims 5-7, or a vector according to claim 8, or a cell according to claim 9, for use in a method for at least in part treating or preventing myeloid leukemia (AML) or B- non-Hodgkin lymphoma (B-NHL) or for use in diagnosis of AML or B-NHL.

13. Use of an antibody or functional part according to any one of claims 1-4, or a nucleic acid molecule according to any one of claims 5-7, or a vector according to claim 8, or a cell according to claim 9, for determining whether a sample comprises AML cells or B- non-Hodgkin lymphoma cells.

14. A method for producing an antibody or functional part according to any one of claims 1-4, the method comprising providing a cell with a nucleic acid molecule or a vector according to any one of claims 5-8, and allowing said cell to translate said nucleic acid molecule or vector, thereby producing said antibody or functional part according to any one of claims 1-4, the method preferably further comprising harvesting, purifying and/or isolating said antibody or functional part.

15. A method for determining whether AML or B-NHL cells are present in a sample comprising:
- contacting said sample with an antibody or functional part according to any one of claims 1-4, and
- allowing said antibody or functional part to bind myeloproliferative cells or lymphoproliferative cells, if present, and
- determining whether or not AML or B-NHL cells are bound to said antibody or functional part, thereby determining whether or not AML or B-NHL cells are present in said sample.

16. A method for determining whether an individual is suffering from AML or B-NHL, comprising:
- contacting a sample obtained from said individual with an antibody or functional part thereof according to any one of claims 1-4, and
- allowing said antibody or functional part to bind AML or B-NHL cells, if present, and
- determining whether or not AML or B-NHL cells are bound to said antibody or functional part, thereby determining whether or nor said individual is suffering from AML or B-NHL.

17. A bispecific or multispecific binding compound comprising an antibody or functional part thereof according to any one of claims 1-4 and an immunomodulatory molecule, preferably a CD3-specific binding compound.

18. A synthetic or recombinant antibody, or a functional part thereof, which comprises one Fab fragment of an antibody according to any one of claims 1-4, and one Fab fragment of another antibody.

19. A chimeric antigen receptor (CAR) T cell comprising an antibody or functional part thereof according to any one of claims 1-4.

20. Use of an antibody or functional part according to any one of claims 1-4 for determining whether an AML patient has a Graft versus Leukemia response.

## Patentansprüche

1. Isolierter, synthetischer oder rekombinanter Antikörper oder funktioneller Teil davon, der in der Lage ist an AML-Zellen zu binden, umfassend:
- eine CDR1-, -CDR2- und -CDR3-Sequenz einer schweren Kette und eine CDR1-, -CDR2- und -CDR3-Sequenz einer leichten Kette wie durch SEQ ID NO: 209, 213, 217, 221, 225 bzw. 229 definiert, und der in der Lage ist an eine Zelloberflächenkomponente verschiedener AML-Zellen von mindestens drei FAB-Klassifikationen zu binden.

2. Antikörper oder funktioneller Teil davon nach Anspruch 1, umfassend eine variable Sequenz einer schweren Kette mit mindestens 90 % Sequenzidentität mit einer Sequenz umfassend SEQ ID NO: 233 und/oder umfassend eine variable Sequenz einer leichten Kette mit mindestens 90 % Sequenzidentität mit einer Sequenz umfassend SEQ ID NO: 237.

3. Antikörper oder funktioneller Teil davon nach Anspruch 1 oder 2, wobei der Antikörper vom IgG-Isotyp, vorzugsweise IgG1 oder IgG3, ist.

4. Antikörper oder funktioneller Teil davon nach einem der Ansprüche 1 bis 3, der an eine andere Verbindung gekoppelt ist, wobei vorzugsweise die andere Verbindung eine detektierbare Markierung, ein chemotherapeutisches Arzneimittel, ein toxischer Rest, ein immunmodulatorisches Molekül, eine andere AML-spezifisch bindende Verbindung oder eine radioaktive Verbindung ist.

5. Isoliertes, synthetisches oder rekombinantes Nukleinsäuremolekül, das mindestens die variable Sequenz der schweren Kette und/oder die variable Sequenz der leichten Kette eines Antikörpers oder eines funktionellen Teils davon nach einem der Ansprüche 1 bis 3 kodiert, wobei vorzugsweise das Nukleinsäuremolekül eine Sequenz umfasst, die mindestens 80 % Sequenzidentität hat mit einer Sequenz umfassend SEQ ID NO: 265 und/oder eine Sequenz, die mindestens 80 % Sequenzidentität hat mit einer Sequenz umfassend SEQ ID NO: 269.

6. Nukleinsäuremolekül oder funktionelles Äquivalent davon nach Anspruch 5, wobei das Nukleinsäuremolekül oder funktionelle Äquivalent DNA, RNA, cDNA, Peptidnukleinsäure (PNA), Locked-Nukleinsäure (LNA) oder eine DNA/RNA-Helix umfasst.

7. Nukleinsäuremolekül oder funktionelles Äquivalent davon, das einen Antikörper oder einen funktionellen Teil davon nach einem der Ansprüche 1 bis 4 kodiert.

8. Vektor, umfassend ein Nukleinsäuremolekül nach einem der Ansprüche 5 bis 7.

9. Isolierte oder rekombinante Zelle, umfassend ein Nukleinsäuremolekül nach einem der Ansprüche 5 bis 7 oder einen Vektor nach Anspruch 8.

10. Zusammensetzung, umfassend einen Antikörper oder einen funktionellen Teil davon nach einem der Ansprüche 1 bis 4, oder ein Nukleinsäuremolekül nach einem der Ansprüche 5 bis 7, oder einen Vektor nach Anspruch 8, oder eine Zelle nach Anspruch 9, wobei vorzugsweise die Zusammensetzung eine pharmazeutische Zusammensetzung ist, die einen pharmazeutisch akzeptabelen Träger, ein Verdünnungsmittel oder einen Hilfsstoff umfasst, wobei bevorzugter die pharmazeutische Zusammensetzung mindestens zwei Antikörper oder funktionelle Teile davon nach einem der Ansprüche 1 bis 4 umfasst.

11. Antikörper oder funktioneller Teil nach einem der Ansprüche 1-4, oder ein Nukleinsäuremolekül nach einem der Ansprüche 5 bis 7, oder ein Vektor nach Anspruch 8, oder eine Zelle nach Anspruch 9 zur Verwendung als Medikament oder Prophylaktikum.

12. Antikörper oder funktioneller Teil nach einem der Ansprüche 1-4, oder ein Nukleinsäuremolekül nach einem der Ansprüche 5 bis 7, oder ein Vektor nach Anspruch 8, oder eine Zelle nach Anspruch 9 zur Verwendung in einem Verfahren zur mindestens teilweisen Behandlung oder Verbeugung von myeloischer Leukämie (AML) oder B-Non-Hodgkin-Lymphom (B-NHL) oder zur Verwendung bei der Diagnose von AML oder B-NHL.

13. Verwendung eines Antikörpers oder eines funktionellen Teils nach einem der Ansprüche 1 bis 4, oder eines Nukleinsäuremoleküls nach einem der Ansprüche 5 bis 7, oder eines Vektors nach Anspruch 8 ,oder einer Zelle nach Anspruch 9 zum Bestimmen, ob eine Probe AML-Zellen oder B-Non-Hodgkin-Lymphomzellen umfasst.

14. Verfahren zur Herstellung eines Antikörpers oder eines funktionellen Teils nach einem der Ansprüche 1 bis 4, wobei das Verfahren Bereitstellen einer Zelle mit einem Nukleinsäuremolekül oder eines Vektors nach einem der Ansprüche 5 bis 8 umfasst, und der Zelle erlaubt das Nukleinsäuremolekül oder den Vektor zu translatieren, wodurch der Antikörper oder der funktionelle Teil nach einem der Ansprüche 1 bis 4 hergestellt wird, wobei das Verfahren vorzugsweise ferner Ernten, Reinigen und/oder Isolieren des Antikörpers oder des funktionellen Teils umfasst.

15. Verfahren zum Bestimmen, ob AML- oder B-NHL-Zellen in einer Probe vorhanden sind, umfassend:
- Kontaktieren der Probe mit einem Antikörper oder funktionellen Teil davon nach einem der Ansprüche 1 bis 4, und
- Erlauben dem Antikörper oder dem funktionellen Teil myeloproliferative Zellen oder lymphoproliferative Zellen zu binden, falls vorhanden, und
- Bestimmen, ob oder nicht AML- oder B-NHL-Zellen an den Antikörper oder den funktionellen Teil gebunden sind, um dadurch zu bestimmen, ob oder nicht AML- oder B-NHL-Zellen in der Probe vorhanden sind.

16. Verfahren zum Bestimmen, ob ein Individuum an AML oder B-NHL leidet, umfassend:
- Kontaktieren einer Probe erhalten von dem Individuum mit einem Antikörper oder funktionellen Teil davon nach einem der Ansprüche 1 bis 4, und
- Erlauben dem Antikörper oder dem funktionellen Teil AML- oder B-NHL-Zellen, falls vorhanden, bindet, und
- Bestimmen, ob oder nicht AML- oder B-NHL-Zellen an den Antikörper oder den funktionellen Teil gebunden sind, um dadurch zu bestimmen, ob oder nicht das Individuum an AML oder B-NHL leidet.

17. Bi- oder multispezifische bindende Verbindung, umfassend einen Antikörper oder einen funktionellen Teil davon nach einem der Ansprüche 1 bis 4 und ein immunmodulatorisches Molekül, vorzugsweise eine CD3-spezifisch bindende Verbindung.

18. Ein synthetischer oder rekombinanter Antikörper oder ein funktioneller Teil davon, der ein Fab-Fragment eines Antikörpers nach einem der Ansprüche 1 bis 4 und ein Fab-Fragment eines anderen Antikörpers umfasst.

19. Eine chimäre Antigenrezeptor (CAR) T-Zelle, umfassend einen Antikörper oder einen funktionellen Teil davon nach einem der Ansprüche 1 bis 4.

20. Verwendung eines Antikörpers oder funktionellen Teils nach einem der Ansprüche 1 bis 4 zum Bestimmen, ob ein AML-Patient eine Graft-versus-Leukämie-Reaktion hat.

## Revendications

1. Anticorps isolé, synthétique ou recombiné, ou une partie fonctionnelle de celui-ci capable de se lier à des cellules de LMA, comprenant :
- une séquence CDR1, CDR2 et CDR3 de la chaîne lourde et une séquence CDR1, CDR2 et CDR3 de la chaîne légère telles que définies par les SEQ ID NO : 209, 213, 217, 221, 225 et 229 respectivement et qui est capable de se lier à un composant de surface cellulaire de différentes cellules de LMA d'au moins trois classifications FAB.

2. Anticorps ou partie fonctionnelle de celui-ci selon la revendication 1, comprenant une séquence variable de chaîne lourde ayant au moins 90 % d'identité de séquence avec une séquence comprenant la SEQ ID NO : 233, et/ou comprenant une séquence variable de chaîne légère ayant au moins 90 % d'identité de séquence avec une séquence comprenant la SEQ ID NO : 237.

3. Anticorps ou partie fonctionnelle de celui-ci selon la revendication 1 ou 2, dans lequel ledit anticorps est de l'isotype IgG, de préférence IgG1 ou IgG3.

4. Anticorps ou partie fonctionnelle de celui-ci selon l'une quelconque des revendications 1 à 3, qui est couplé à un autre composé, de préférence dans lequel ledit autre composé est un marqueur détectable, un médicament chimiothérapeutique, un fragment toxique, une molécule immunomodulatrice, un autre composé de liaison spécifique de la LMA, ou un composé radioactif.

5. Molécule d'acide nucléique isolée, synthétique ou recombinée, qui code au moins la séquence variable de la chaîne lourde et/ou la séquence variable de la chaîne légère d'un anticorps ou d'une partie fonctionnelle de celui-ci selon l'une quelconque des revendications 1 à 3, de préférence dans laquelle ladite molécule d'acide nucléique comprend une séquence ayant au moins 80 % d'identité de séquence avec une séquence comprenant la SEQ ID NO : 265, et/ou une séquence ayant au moins 80 % d'identité de séquence avec une séquence comprenant la SEQ ID NO : 269.

6. Molécule d'acide nucléique ou équivalent fonctionnel de celle-ci selon la revendication 5, dans laquelle ladite molécule d'acide nucléique ou ledit équivalent fonctionnel comprend de l'ADN, de l'ARN, de l'ADNc, un acide nucléique peptidique (PNA), un acide nucléique verrouillé (LNA) ou une hélice d'ADN/ARN.

7. Molécule d'acide nucléique ou équivalent fonctionnel de celle-ci, codant un anticorps ou une partie fonctionnelle de celui-ci selon l'une quelconque des revendications 1 à 4.

8. Vecteur comprenant une molécule d'acide nucléique selon l'une quelconque des revendications 5 à 7.

9. Cellule isolée ou recombinée, comprenant une molécule d'acide nucléique selon l'une quelconque des revendications 5 à 7, ou un vecteur selon la revendication 8.

10. Composition comprenant un anticorps ou une partie fonctionnelle de celui-ci selon l'une quelconque des revendications 1 à 4, ou une molécule d'acide nucléique selon l'une quelconque des revendications 5 à 7, ou un vecteur selon la revendication 8, ou une cellule selon la revendication 9, de préférence dans laquelle ladite composition est une composition pharmaceutique qui comprend un véhicule, un diluant ou un excipient pharmaceutiquement acceptable, plus préférentiellement dans laquelle ladite composition pharmaceutique comprend au moins deux anticorps ou parties fonctionnelles de ceux-ci selon l'une quelconque des revendications 1 à 4.

11. Anticorps ou partie fonctionnelle selon l'une quelconque des revendications 1 à 4, ou molécule d'acide nucléique selon l'une quelconque des revendications 5 à 7, ou vecteur selon la revendication 8, ou cellule selon la revendication 9, pour une utilisation en tant que médicament ou agent prophylactique.

12. Anticorps ou partie fonctionnelle selon l'une quelconque des revendications 1 à 4, ou molécule d'acide nucléique selon l'une quelconque des revendications 5 à 7, ou vecteur selon la revendication 8, ou cellule selon la revendication 9, pour une utilisation dans un procédé de traitement ou de prévention au moins en partie de la leucémie myéloïde aiguë (LMA) ou du lymphome non hodgkinien B (B-NHL) ou pour une utilisation dans le diagnostic de la LMA ou du B-NHL.

13. Utilisation d'un anticorps ou d'une partie fonctionnelle selon l'une quelconque des revendications 1 à 4, ou d'une molécule d'acide nucléique selon l'une quelconque des revendications 5 à 7, ou d'un vecteur selon la revendication 8, ou d'une cellule selon la revendication 9, pour déterminer si un échantillon comprend des cellules de LMA ou des cellules de lymphome non hodgkinien B.

14. Procédé de production d'un anticorps ou d'une partie fonctionnelle selon l'une quelconque des revendications 1 à 4, le procédé comprenant la fourniture d'une cellule avec une molécule d'acide nucléique ou un vecteur selon l'une quelconque des revendications 5 à 8, et le fait de permettre la traduction par ladite cellule de ladite molécule d'acide nucléique ou dudit vecteur, produisant ainsi ledit anticorps ou ladite partie fonctionnelle selon l'une quelconque des revendications 1 à 4, le procédé comprenant en outre la récolte, la purification et/ou l'isolement dudit anticorps ou de ladite partie fonctionnelle.

15. Procédé de détermination de la présence de cellules de LMA ou de B-NHL dans un échantillon comprenant :
- la mise en contact dudit échantillon avec un anticorps ou une partie fonctionnelle selon l'une quelconque des revendications 1 à 4, et
- le fait de permettre la liaison dudit anticorps ou de ladite partie fonctionnelle à des cellules myéloprolifératives ou lymphoprolifératives, si présentes, et
- la détermination que des cellules de LMA ou de B-NHL sont ou non liées audit anticorps ou à ladite partie fonctionnelle, déterminant ainsi si des cellules de LMA ou de B-NHL sont ou non présentes dans ledit échantillon.

16. Procédé de détermination si un individu est atteint de LMA ou de B-NHL, comprenant :
- la mise en contact d'un échantillon prélevé chez ledit individu avec un anticorps ou une partie fonctionnelle de celui-ci selon l'une quelconque des revendications 1 à 4, et
- le fait de permettre la liaison dudit anticorps ou de ladite partie fonctionnelle à des cellules de LMA ou de B-NHL, si présentes, et
- la détermination que des cellules de LMA ou de B-NHL sont ou non liées audit anticorps ou à ladite partie fonctionnelle, déterminant ainsi si ledit individu est ou non atteint de LMA ou de B-NHL.

17. Composé de liaison bispécifique ou multispécifique comprenant un anticorps ou une partie fonctionnelle de celui-ci selon l'une quelconque des revendications 1 à 4 et une molécule immunomodulatrice, de préférence un composé de liaison spécifique du CD3.

18. Anticorps synthétique ou recombiné, ou une partie fonctionnelle de celui-ci, qui comprend un fragment Fab d'un anticorps selon l'une quelconque des revendications 1 à 4, et un fragment Fab d'un autre anticorps.

19. Cellule T à récepteur antigénique chimérique (CAR) comprenant un anticorps ou une partie fonctionnelle de celui-ci selon l'une quelconque des revendications 1 à 4.

20. Utilisation d'un anticorps ou d'une partie fonctionnelle selon l'une quelconque des revendications 1 à 4 pour déterminer si un patient atteint de LMA présente une réponse d'effet greffon contre leucémie.
